# EUROPEAN PATENT SPECIFICATION

(11) **EP 1 185 665 B1**
(45) Date of publication and mention of the grant of the patent: **10.09.2008**
(21) Application number: 00939534.4
(22) Date of filing: 02.06.2000
(51) Int. Cl.: C12N 15/51, C07K 14/18, C12Q 1/68, C12N 7/00

(54) **INFECTIOUS cDNA CLONE OF GB VIRUS B AND USES THEREOF**
INFEKTIÖSES CDNA KLON DES GB-VIRUS B UND DESSEN VERWENDUNGEN
CLONE INFECTIEUX D'ADNC DU GB VIRUS-B ET SES UTILISATIONS

(30) Priority: 04.06.1999 US 137694 P
(43) Date of publication of application: 13.03.2002
(73) Proprietor: The Government of the United States of America as represented by the Secretary of the Department of Health and Human Services, Rockville, MD 20852-3804 (US)
(72) Inventor: BUKH, Jens, Rockville, MD 20851 (US); YANAGI, Masayuki, Rockville, MD 20852 (US); EMERSON, Suzanne, U., Kensington, MD 20895 (US); PURCELL, Robert, H., Boyds, MD 20841 (US)
(74) Representative: Gervasi, Gemma
(86) International application number: PCT/US2000/015293
(87) International publication number: WO 2000/075337

(56) References cited:
- WO-A-95/21922
- SCARSELLI ELISA ET AL: "GB virus B and hepatitis C virus NS3 serine proteases share substrate specificity." JOURNAL OF VIROLOGY, vol. 71, no. 7, July 1997 (1997-07), pages 4985-4989, XP002150190 ISSN: 0022-538X cited in the application
- HONDA MASAO ET AL: "A phylogenetically conserved stem-loop structure at the 5' border of the internal ribosome entry site of hepatitis C virus is required for cap-independent viral translation." JOURNAL OF VIROLOGY, vol. 73, no. 2, February 1999 (1999-02), pages 1165-1174, XP002150191 ISSN: 0022-538X cited in the application
- YANAGI MASAYUKI ET AL: "In vivo analysis of the 3' untranslated region of the hepatitis C virus after in vitro mutagenesis of an infectious cDNA clone." PROCEEDINGS OF THE NATIONAL ACADEMY OF SCIENCES OF THE UNITED STATES, vol. 96, no. 5, 2 March 1999 (1999-03-02), pages 2291-2295, XP002150192 ISSN: 0027-8424 cited in the application
- YANAGI M ET AL: "Transcripts of a chimeric cDNA clone of hepatitis C virus genotype 1b are infectious in vivo" VIROLOGY, vol. 244, no. 1, 1998, pages 161-172, XP002089701 ISSN: 0042-6822 cited in the application
- BUKH JENS ET AL: "Toward a surrogate model for hepatitis C virus: An infectious molecular clone of the GB virus-B hepatitis agent." VIROLOGY, vol. 262, no. 2, 30 September 1999 (1999-09-30), pages 470-478, XP002150193 ISSN: 0042-6822
- SBARDELLATI ANDREA ET AL: "Identification of a novel sequence at the 3' end of the GB virus B genome." JOURNAL OF VIROLOGY, vol. 73, no. 12, December 1999 (1999-12), pages 10546-10550, XP002150194 ISSN: 0022-538X
- BUTKIEWICZ N. ET AL.: "Virus-specific cofactor requirement and chimeric hepatitis C virus/GB virus B nonstructural protein 3." J VIROL 2000 MAY;74(9):4291-301, XP002150195

## Description

### Field of Invention

The present invention relates to nucleic acid sequence which comprises the genome of an infectious GB virus B (GBV-B) clone. The invention also relates to the use of the nucleic acid sequence of the infectious GB virus B clone to study indirectly the molecular properties of hepatitis C virus (HCV), and in the production of HCV/GBV-B chimeras. The invention further relates to the use of the infectious nucleic acid sequence of the GB virus B clone and the HCV/GBV-B chimeras in the development of vaccines and therapeutics for HCV.

### Background of Invention

Transmission studies of potential human hepatitis agents were first reported in 1967 (Deinhardt 1967). Four tamarins inoculated with acute phase sera from a surgeon with acute hepatitis (patient GB) developed hepatitis, as did most tamarins inoculated in serial passage studies. Subsequent studies indicated that the etiological agent responsible for the development of hepatitis in these animals was not any of the known human hepatitis viruses (Purcell 1994). In 1995, two related RNA viruses named GB virus-B (GBV-B) and GB virus A (GBV-A) were identified in acute phase sera of a tamarin which developed hepatitis following inoculation with serum of the eleventh tamarin passage of the putative GB agent (Simons 1995a).

GBV-B infection of tamarins resulted in acute resolving hepatitis (Schlauder 1995, Buhk 1997). The natural host of GBV-B is still unknown as the virus has not been detected in uninoculated animals or in humans.

GBV-A, on the other hand, is an indigenous tamarin virus rather than a component of the original GB inoculum (Bukh 1997, Erker 1998). Experimental infection of tamarins with GBV-A did not produce hepatitis (Schlauder 1995). A human agent, GBV-C or hepatitis G virus, most closely related to GBV-A, was later identified (Simons 1995b, Linnen 1996). However, it is still not clear whether this virus actually causes hepatitis (Alter 1998, Bukh 1998a). Thus, of the known GB viruses, GBV-B may be the only true hepatitis virus.

Based on analysis of their genomic sequences, GBV-A, GBV-B and GBV-C were classified as members of the Flaviviridae family of viruses, and among the known viruses, GBV-B is the virus most closely related to hepatitis C virus (HCV) (Muerhoff 1995, Robertson 1998).

The GBV-B virus contains a positive-sense, single-stranded RNA genome of 9143 nucleotides (nts) (Simons 1995a, Muerhoff 1995). The viral genome of GBV-B consists of a 5' untranslated region (UTR), a single long open reading frame (ORF) and a 3' UTR. Based on known motifs, structural proteins were predicted to be encoded in the 5' portion of the ORF and nonstructural (NS) proteins in the 3' portion of the ORF (Muerhoff 1995). The hydropathy plots of the polyproteins of GBV-B and HCV are very similar even though the overall homology of the predicted polyproteins between GBV-B and HCV is only about 25-30% (Muerhoff 1995). The putative envelope proteins (E1 and E2) of GBV-B and HCV share common structural features, and significant homology was observed between the NS3 serine protease, the NS3 RNA helicase, and the NS5 RNA-dependent RNA polymerase regions of GBV-B and HCV (Muerhoff 1995). Furthermore, the function and substrate specificity of the GBV-B and HCV NS3 serine proteases are also similar (Scarselli 1997). The genomic structure and organization of GBV-B and HCV share additional features of interest. First, colinear regions with significant sequence homology were identified in the 5' UTRs (Muerhoff 1995) and the predicted IRES structure of GBV-B is similar to that of HCV (Lemon 1997). Second, both viruses begin the 3' UTR with a short sequence followed by a poly (U) stretch followed by additional nucleotides (50 nucleotides for GBV-B and 98 nucleotides for HCV). However, the 3' terminal sequence of HCV forms a stable stem-loop structure (Kolykhalov 1996) whereas the published 3' terminal sequence of GBV-B does not.

To date, molecular studies of HCV are severely limited by the lack of an efficient cell culture system for the virus and by expense and limited availability of chimpanzees, the sole animal model for HCV. Accordingly, a less expensive and more readily available animal than chimpanzees is necessary as an animal model for the study of HCV.

### Summary of Invention

The present invention relates to an isolated nucleic acid molecule which encodes a GB virus-B and which is capable of producing infectious virus when transfected into cells characterized in that the 3' UTR sequence is the 3'UTR sequence of SEQ ID NO:1. It is therefore an object of the invention to provide nucleic acid sequence which encodes an infectious GBV-B. Such nucleic acid sequence is referred to throughout the application as "infectious nucleic acid sequence".

As significant structural homology exists between the genomes of GBV-B and HCV, the invention also relates to the use of infection of tamarins with the infectious nucleic acid sequence of GBV-B or with mutants of the infectious sequence to study indirectly the molecular properties of hepatitis C virus (HCV) or as a preliminary screen to identify agents which have antiviral activity against HCV.

The invention further relates to "chimeric nucleic acid sequences" consisting of portions of the infectious nucleic acid sequence of GBV-B and portions of the nucleic acid sequences of other viruses closely related to GBV-B such as HCV, GBV-C or other members of the *Flaviviridae* family which do not replicate in tamarins. In a preferred embodiment, the chimeric nucleic acid sequences consist of portions of the infectious nucleic acid sequence of GBV-B and portions of the nucleic acid sequence of HCV. The nucleic acid sequences taken from GBV-B and HCV can be open-reading frame sequences, and/or sequences from the 5'UTR and/or 3'UTR.

In one embodiment, GBV-B/HCV chimeras may be made in which 5' or 3' UTR sequences of the GBV-B infectious clone are replaced with the corresponding sequence from an HCV clone.

In another embodiment, GBV-B/HCV chimeras may be constructed in which the structural or non-structural regions of GBV-B are replaced by corresponding regions of HCV. Thus, such a chimera would contain, for example, the HCV structual region in a GBV-B "genomic backbone". Of course, it is understood by one of skill in the art that the construction of the above-described chimeric nucleic acid sequences may be reversed such that, for example, the GBV structural region may replace the structual region of an HCV genome to produce a chimera in which the GBV structural region is contained in an HCV backbone.

The invention further relates to the use of the chimeric nucleic acid sequences of the invention to study the functions of HCV genes, and for the development of vaccine and antiviral agents against HCV.

The invention also relates to the use of the infectious GBV-B nucleic acid sequence, or the chimeric sequences of the invention to identify cell lines capable of supporting the replication of GBV-B or the chimeras of the invention.

The present invention further relates to the in vitro production of GBV-B, or chimeric GBV-B/HCV viruses from the nucleic acid sequences of the invention.

The invention also provides that the nucleic acid sequences and viruses of the invention be supplied, alone or in the form of a pharmaceutical composition.

### Brief-Description Of Figures

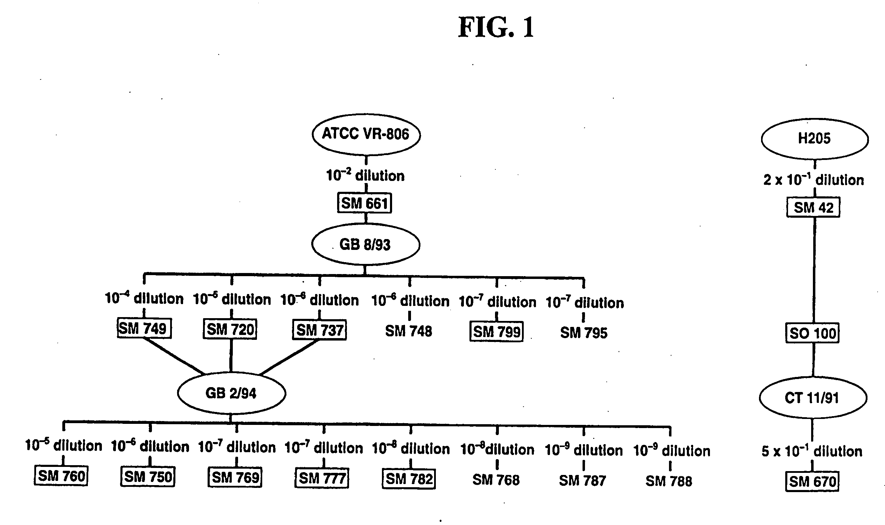

Figure 1 shows a flow diagram of GB virus transmission studies in two species of tamarins, *Saguinus mystax* (SM) and *Saguinus oedipus* (SO). The animals infected with GBV-B (Simons 1995a) are boxed. Two serum pools (GB 8/93 and GB 2/94) were made from acutely infected animals. Both pools contained GBV-B, as well as GBV-A (Simons 1995) at a titer of 10⁸ genome equivalent (GE)/ml. A 10% liver homogenate (CT 11/91) was made from a sacrificed tamarin. A number of S. *mystax* tamarins (SM 737, 749, 750, 760, 782, 795 and 799) and *S*. *oedipus* tamarins (SO 100) were naturally infected with GBV-A_{SM} and GBV-A_{SO}, respectively, prior to inoculation (Bukh 1997). Only two tamarins (SM 720 and 748), both GBV-A_{SM} negative, became infected with GBV-A (Simons 1995) following inoculation. Tamarins SM42 and SM670 were not tested for GBV-A or GBV-A_{SM}.

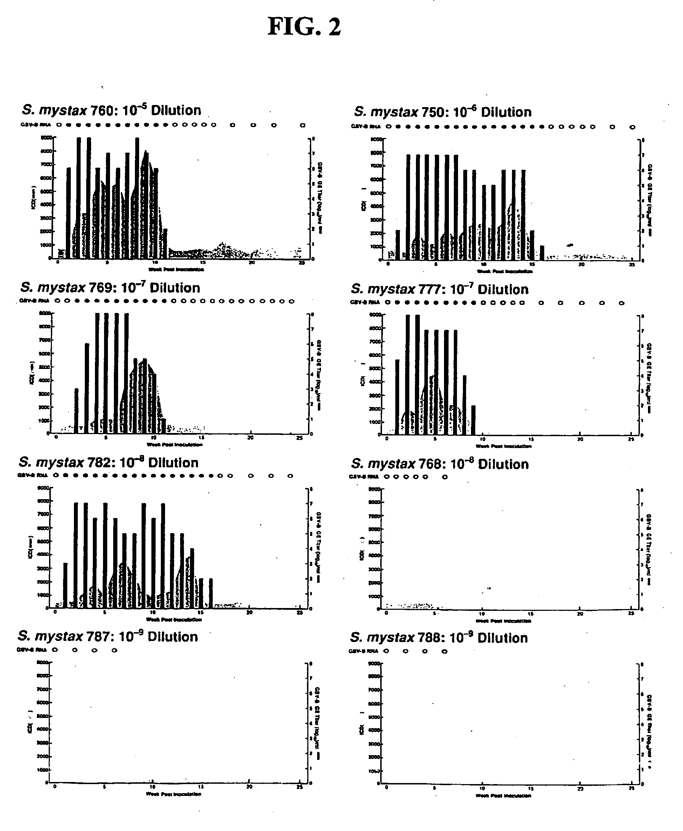

Figure 2 shows the course of GBV-B infection in tamarins (*S*. *mystax*) inoculated with a dilution series of the GB 2/94 pool. All animals were inoculated intravenously at week 0 with 1 ml of the indicated dilution. Results of qualitative RT-nested PCR for GBV-B in serum are shown at the top (filled circles, positive; empty circles, negative). Serum levels of isocitrate dehydrogenase (ICD in units/ml); shaded area) and the estimated log₁₀ GBV-B GE titer (vertical columns) were plotted against time.

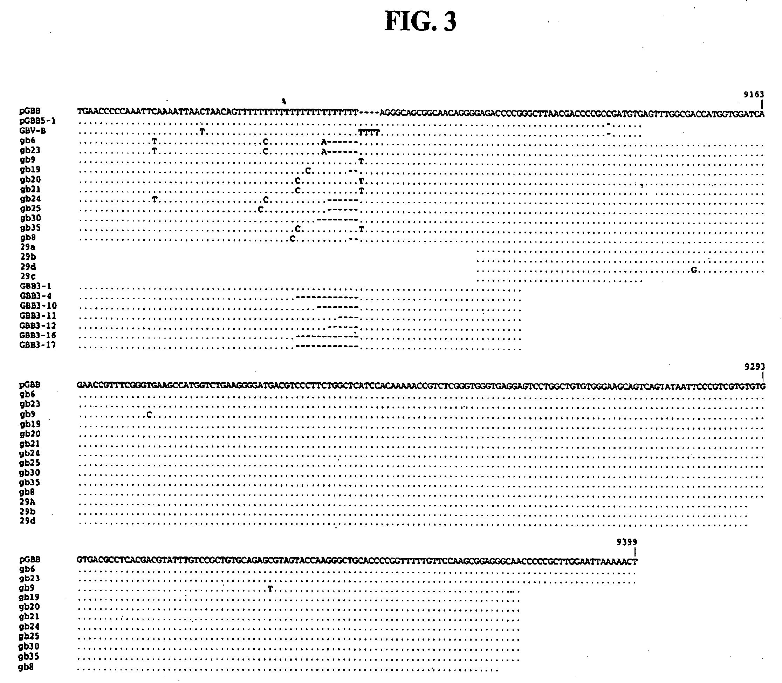

Figure 3 shows alignment of the 3' UTR sequences of GBV-B. The sequence of the infectious clone of GBV-B (pGBB) is shown at the top (nts. 9038-9399). The other sequences shown are: pGBB5-1, a non-infectious clone of GBV-B; GBV-B, a prototype of GBV-B (Simons 1995); eleven "gb" clones obtained from CT 11/91 liver homogenate by 5' RACE on the minus-strand GBV-B RNA; four "29" clones obtained from GB 2/94 pool by RT-PCR across 5'-to-3'-end-ligated viral GBV-B RNA; and seven "GBB3" clones obtained from GB 2/94 pool by standard RT-PCR. With pGBB as the reference, nucleotide substitutions or insertions are shown as uppercase letters, identical nucleotides are shown as dots and nucleotide deletions are shown as dashes.

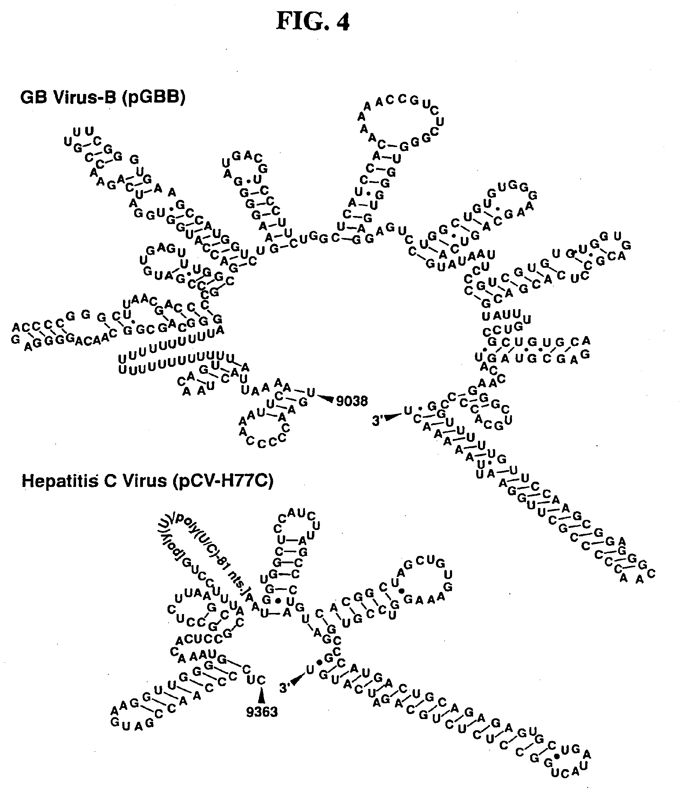

Figure 4 shows the predicted secondary structure of the 3' UTRs of GBV-B and HCV as determined by the program "mfold" (Genetics Computer Group).

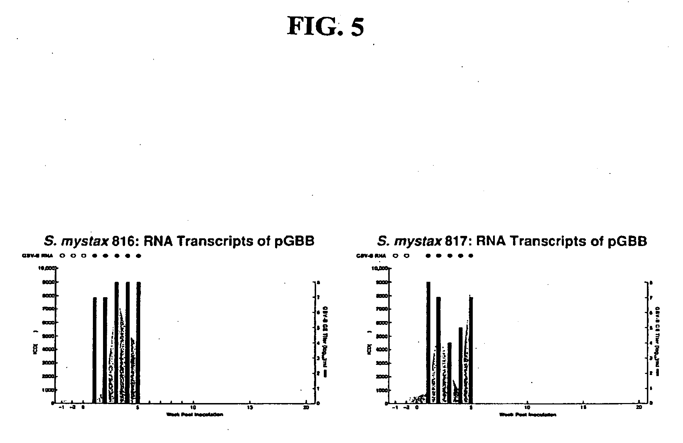

Figure 5 shows the course of GBV-B infection in *S*. *mystax* tamarins transfected with RNA transcripts of pGBB. Both animals were negative for GBV-A_{SM}. At week 0 transcription mixtures were injected into tamarins by percutaneous intrahepatic injection guided by ultrasound. Results of qualitative RT-nested PCR for GBV-B in serum is shown at the top (filled circles, positive; empty circles, negative). Serum levels of isocitrate dehydrogenase (ICD in units/ml; shaded area) and the estimated log₁₀ GBV-B GE titer (vertical columns) were plotted against time.

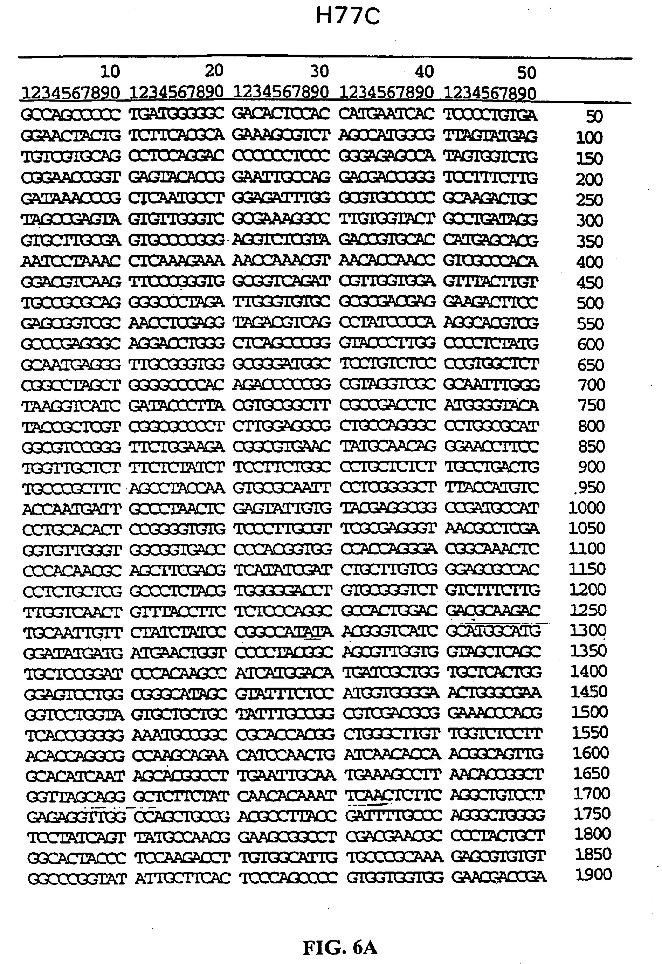

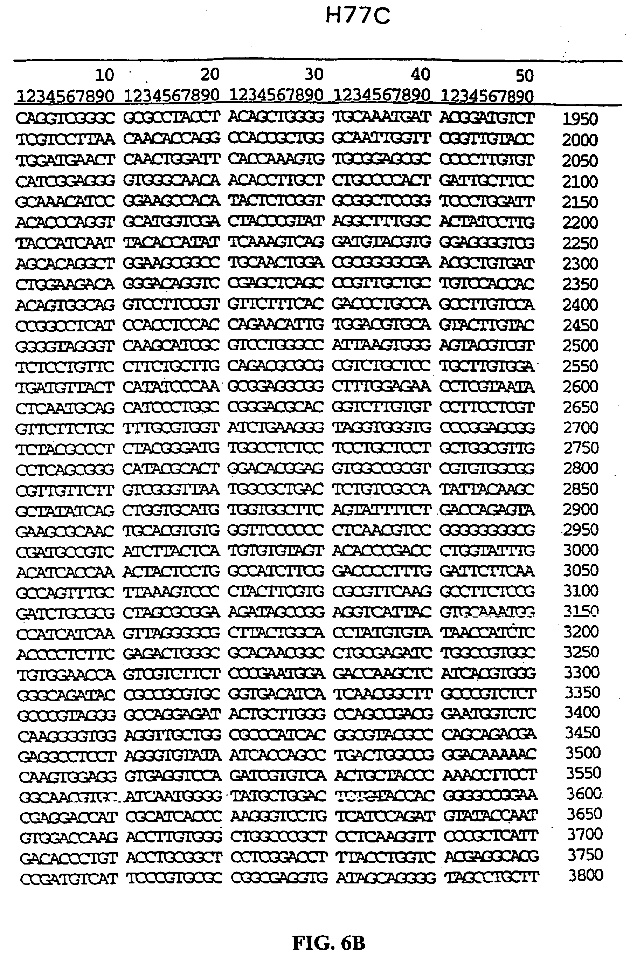

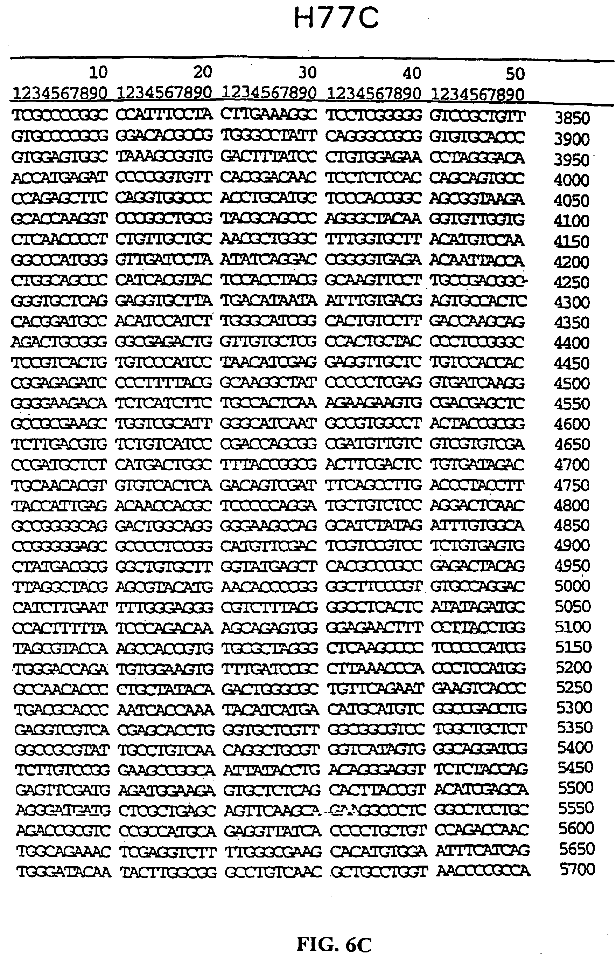

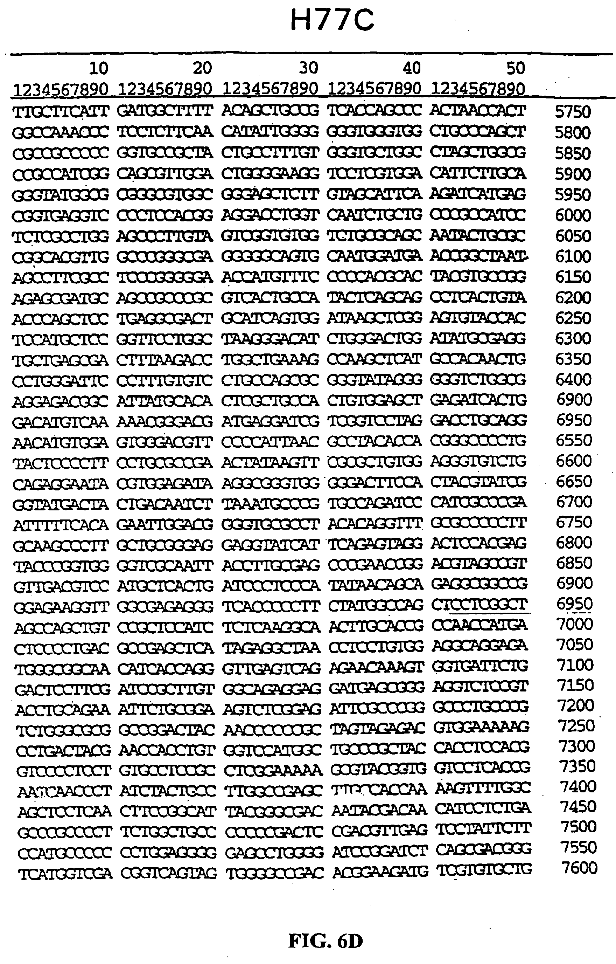

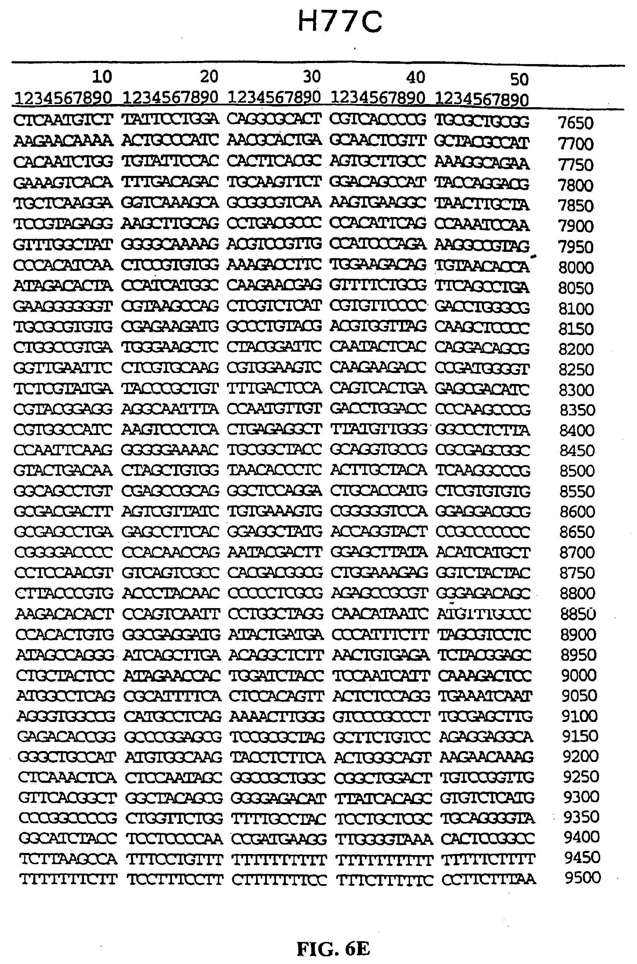

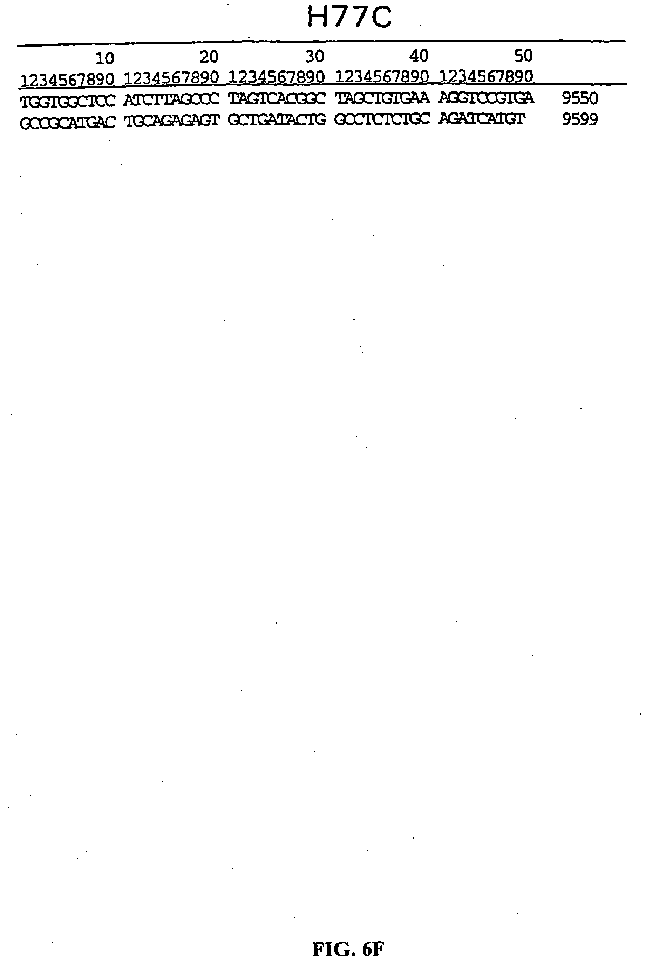

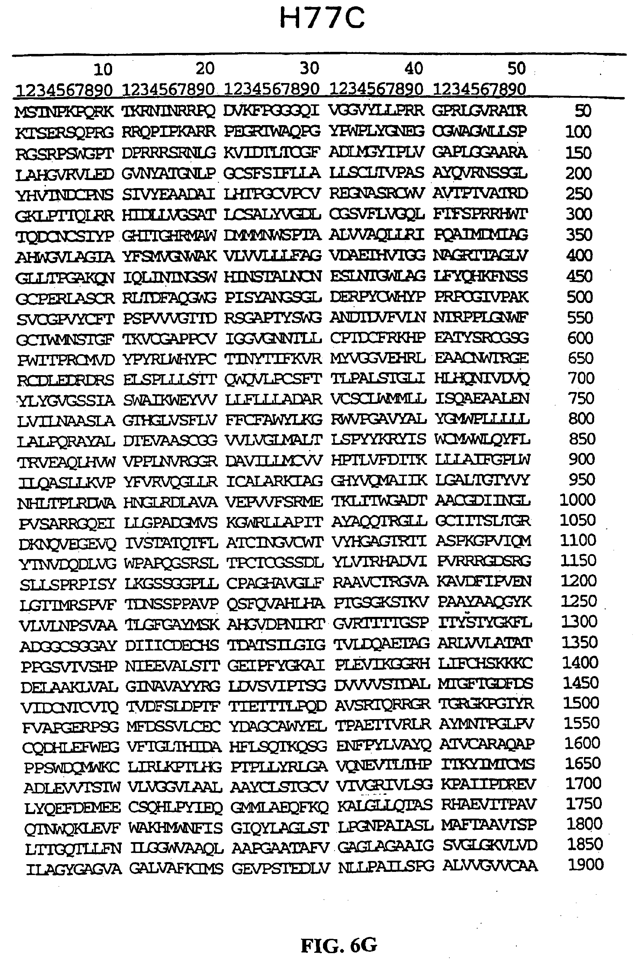

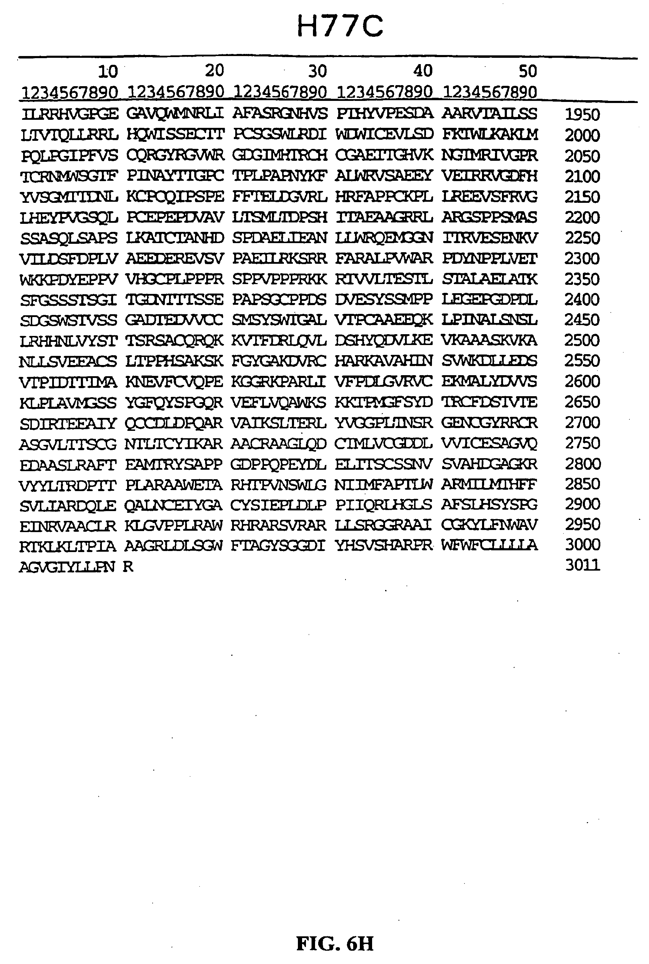

Figures 6A-6F show the nucleotide sequence of the infectious hepatitis C virus clone of genotype 1a strain H77C and Figures 6G-6H show the amino acid sequence encoded by the clone.

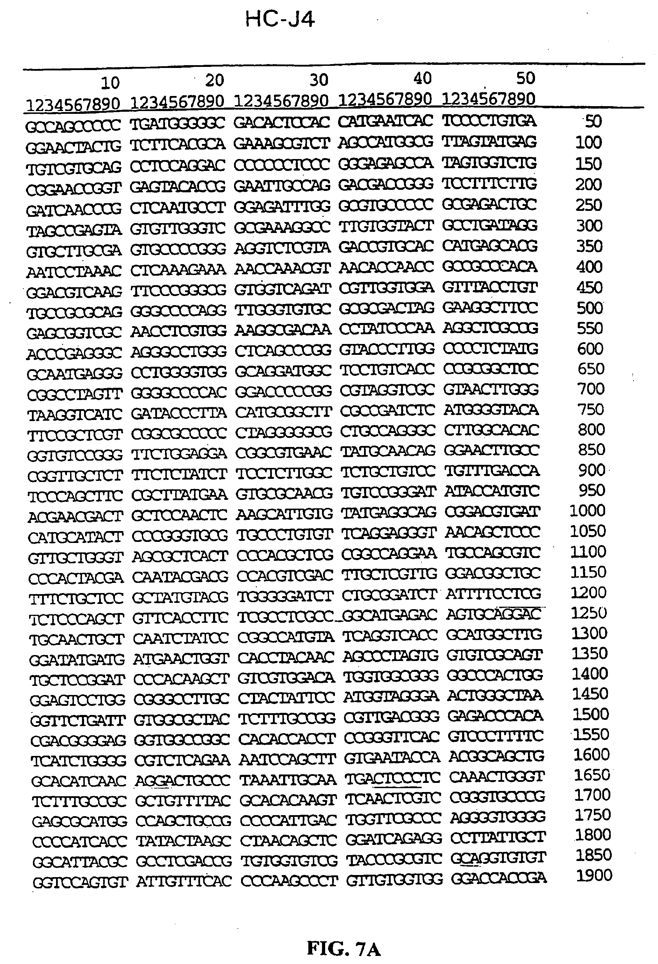

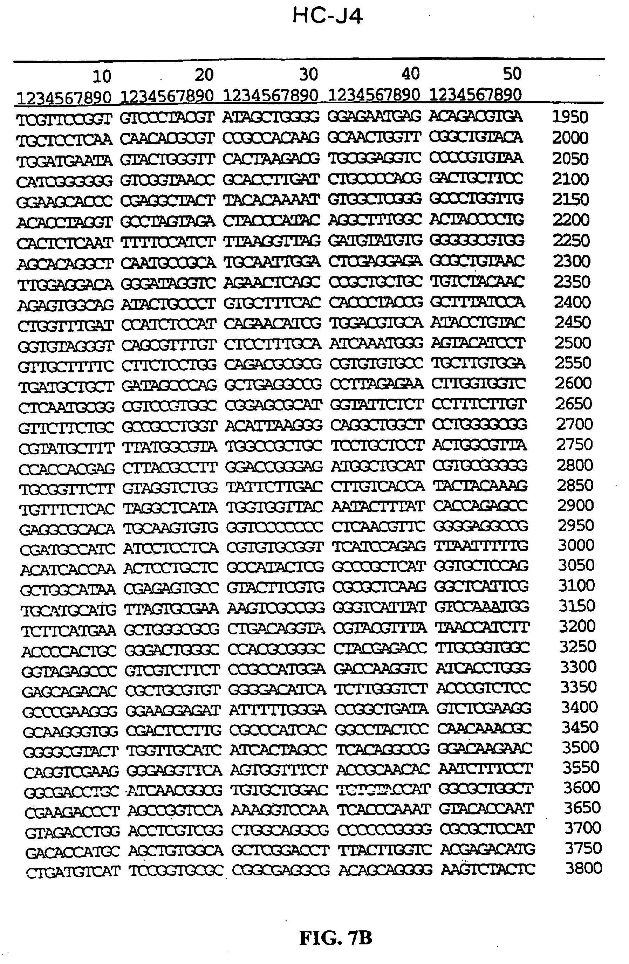

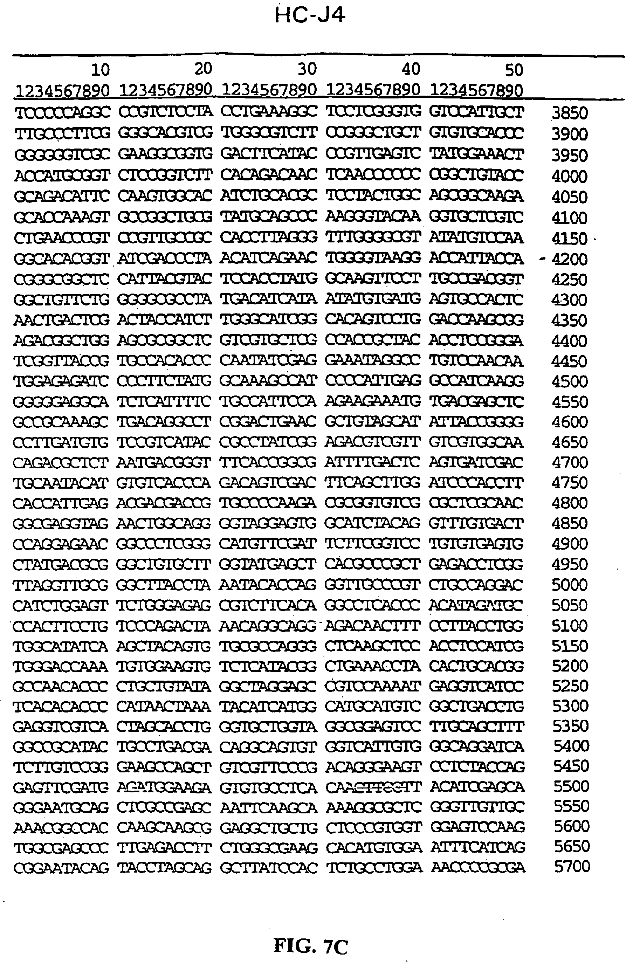

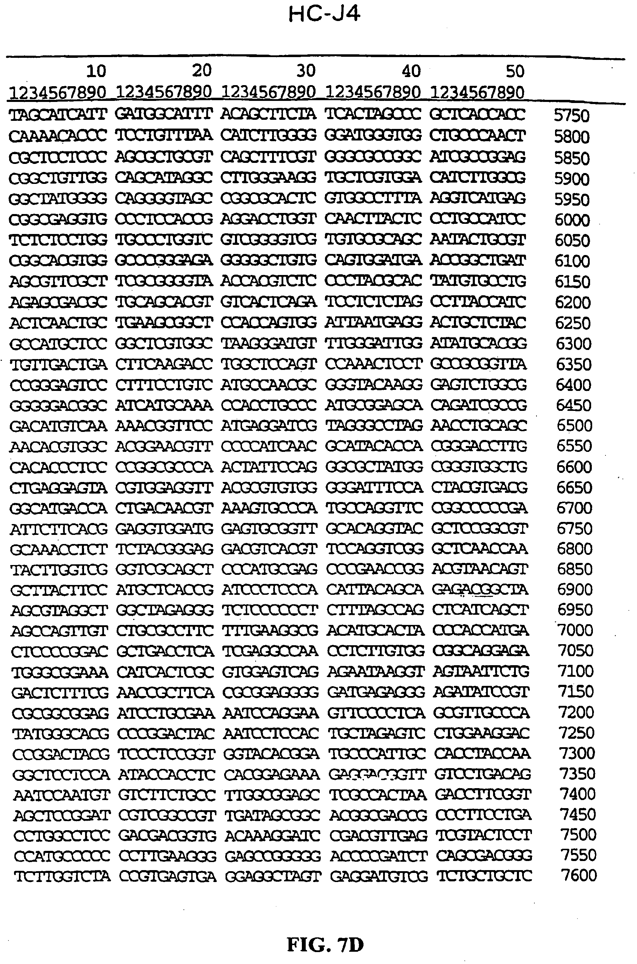

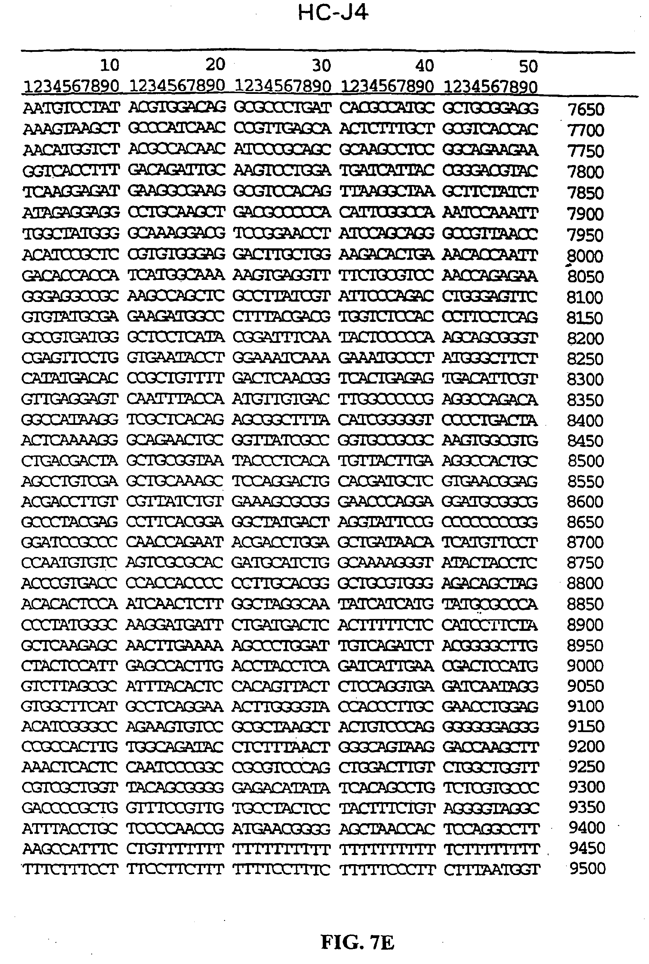

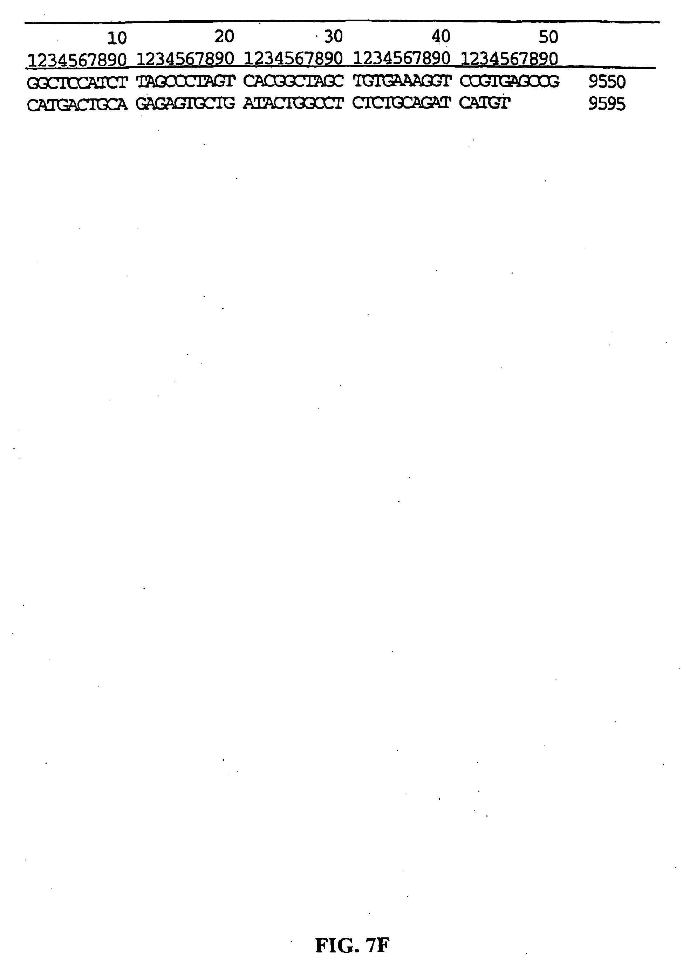

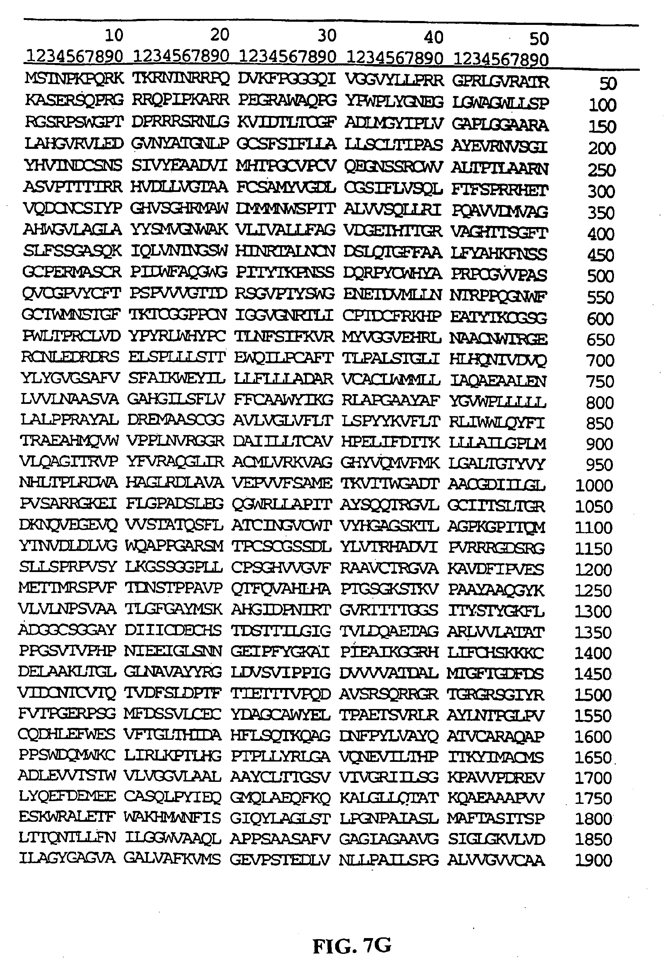

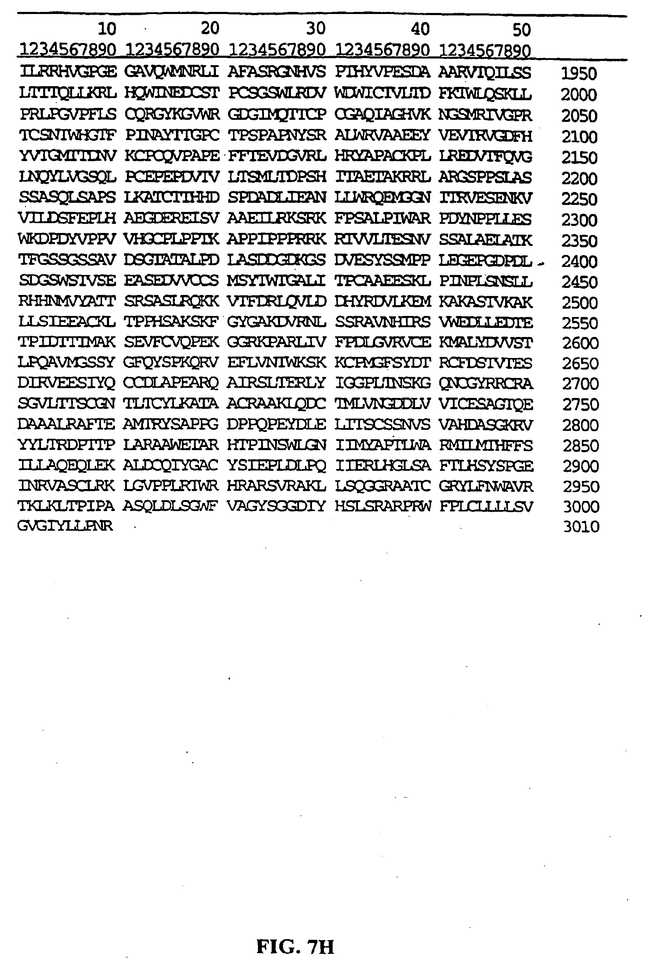

Figures 7A-7F show the nucleotide sequence of the infectious hepatitis C virus clone of genotype 1b strain HC-J4 and Figures 7G-H show the amino acid sequence encoded by the clone.

### Description of The Invention

The present invention relates to an isolated nucleic acid molecule which encodes a GB virus- B and which is capable of producing infectious virus when transfected into cells characterized in that the 3' UTR sequence is the 3' UTR sequence of SEQ ID NO:1. The nucleic acid sequence which comprises the genome of an infectious GBV-B virus is shown in SEQ ID NO:1 and is contained in the plasmid construct pGBB deposited with the American Type Culture Collection (ATCC) on May 28, 1999 and having ATCC accession number PTA-152. The present invention relates to the identification of a 260 nucleotide sequence at the 3' end of the infectious GBV-B clone which is shown in Example 3 to be necessary for the development of the infectious clone.

Since GBV-B is the virus most closely related to HCV, the present invention also relates to experimental infection of tamarins with the infectious GBV-B clone of the invention or with mutants of the infectious GBV clone to study indirectly the molecular properties of HCV or as a preliminary screen to identify agents which have antiviral activity against HCV. For example, since the predicted internal ribosome entry site (IRES) structure in the 5'UTR of GBV-B is similar to that of HCV (Lemon 1997), the NS3 serine proteases of GBV-B and HCV have been shown to share substrate specificity in vitro (Scarselli 1997), and the 3'UTRs of HCV (Yanagi 1999) and GBV-B (see Examples) have been shown to be critical for viral infectivity, mutagenesis of these regions in the GBV-B infectious clone may be undertaken to examine IRES function, NS3 serine protease activity or the role of the 3'UTR in viral infectivity in vivo. Where such "mutations" are introduced into the GBV-B clone of the invention to create a "mutated" GBV-B sequence, the mutations include, but are not limited to, point mutations, deletions and insertions. Of course, one of ordinary skill in the art would recognize that the size of the insertions would be limited by the ability of the resultant nucleic acid sequence to be properly packaged within the virion. Such mutations could be produced by techniques known to those of skill in the art such as site-directed mutagenesis, fusion PCR, and restriction digestion followed by religation.

Alternatively, given the significant structural homology that exists between the genomes of GBV and HCV, the infectious GBV-B clone may be used to screen for inhibitors of IRES function or viral enzyme activity (for example, NS3 helicase, NS3 protease, NS2-NS3 protease or NS5B RNA polymerase activity). Such inhibitors may be useful as antiviral agents to HCV since viral enzyme activity and IRES function are known to be critical for HCV replication.

The effect of such inhibitors on the IRES function or viral activity of the GBV-B encoded by the infectious sequence of the invention may be measured by assays known to those of skill in the art to measure directly or indirectly viral replication or viral pathogenicity. Such assays include, but are not limited to, the measurement of virus titer in serum or liver of an infected tamarin by PCR or the measurement of GBV-B viral protein expression in liver cells of an infected tamarin by immunoflourescence or Western blot. Of course, it is understood that a comparison of results obtained for control tamarins (treated only with infectious nucleic acid sequence) with those obtained for treated tamarins (nucleic acid sequence and antiviral agent) would indicate, the degree, if any, of antiviral activity of the candidate antiviral agent. Of course, one of ordinary skill in the art would readily understand that the tamarins can be treated with the candidate antiviral agent either before or after exposure to the infectious nucleic acid sequence of the present invention.

In yet another embodiment, the invention relates to "chimeric nucleic acid sequences" which consist of portions of the infectious nucleic acid sequence of GBV-B and portions of nucleic acid sequences of viruses which are related to GBV-B such as HCV, GBV-C and other members of the Flaviviridae family which do not infect tamarins. In a preferred embodiment, chimeric nucleic acid sequences consist of portions of the infectious nucleic acid sequence of GBV-B and portions of nucleic acid sequences of hepatitis C viruses (HCV) of various genotypes or subtypes; preferably portions of nucleic acid sequence of infectious HCV clones of genotypes 1a (ATCC accession number PTA-157; Figures 6A-6F), 1b (ATCC accession number 209596; Figures 7A-7F) or 2a (ATCC accession number PTA-153; SEQ ID NO: 4). The nucleic acid sequences taken from GBV-B and HCV can be open-reading frame sequences, and/or sequences from the 5'UTR and/or 3'UTR. The gene borders of the HCV genome, including nucleotide and amino acid locations, have been determined, for example, as depicted in Houghton, M. (1996), and the putative gene borders of the GBV-B are shown in Table 1.

Of course, it is understood that the production of GBV-B/HCV chimeras could include insertion of specific genes or regions of the infectious GBV-B clone into an HCV "genomic backbone" (where the HCV genomic backbone is preferably an infectious nucleic acid sequence of HCV genotypes 1a, 1b or 2a described above) or alternatively, could include insertion of specific genes (or portions thereof) or regions of an HCV genome into the GBV-B infectious clone of the invention. Of course, where HCV genes or regions are to be inserted into the GBV-B infectious clone, it is to be understood that the inserted HCV sequences may be unmodified or may be mutated in order to examine the effect of the mutation(s) on the function of the inserted HCV gene or region in the chimeric GBV-B-HCV virus.

Such chimeras can readily be produced by methods known to those of ordinary skill in the art.

In one embodiment, GBV-B/HCV chimeras may be made in which 5' or 3' UTR sequences of the GBV-B infectious clone are replaced with the corresponding sequence from an HCV clone. For example, chimeras may be constructed in which the IRES sequence of the infectious GBV-B clone is replaced by the IRES sequence of HCV. Such chimeras can be used in identifying inhibitors of IRES activity which would be useful as antiviral agents, or could be used to examine HCV IRES function in vivo. Alternatively, mutations could be introduced into the HCV IRES contained in the GBV-B clone in order to examine the effect of the mutation(s) on IRES function in vivo.

Alternatively, GBV-B/HCV chimeras may be made in which the 3'UTR sequence of GBV-B is replaced by the 3'UTR sequence of HCV. As the 3' terminal stem-loop structure is believed to be important for initiation of RNA replication and has been shown to be critical for infectivity of HCV in vivo, such chimeras may be used for more detailed analysis of the function of the 3' UTR sequence of HCV in vivo and for the testing of candidate antiviral agents.

In another embodiment, GBV-B/HCV chimeras may be constructed in which the structural or non-structural regions of GBV-B are replaced by corresponding regions of HCV. Such chimeras would be useful in identifying whether the inability of HCV to infect tamarins is due to the inability of HCV's structural region to bind the receptor necessary for infection of tamarins or to the absence of sequences in HCV's nonstructural regions which are necessary for replication in tamarins. For example, the ability to infect tamarins with GBV-B/HCV chimeras in which the non-structural region of GBV-B is replaced by the non-structural region of HCV would indicate that the structural genes of GBV-B are necessary for viral infection in tamarins, and that the inability of HCV to infect tamarins is likely due to its lack of receptors for HCV.

Alternatively, the ability to infect tamarins with GBV-B/HCV chimeras in which the structural region of GBV-B is replaced by the structural region of HCV would indicate that the non-structural genes of GBV-B are critical for viral infection in tamarins, and that the inability of HCV to infect tamarins is likely due to HCV's lack of nonstructural sequences which are necessary for replication in tamarins.

Of course, GBV-B-HCV chimeras may be constructed in which only a portion of the non-structural or structural regions of GBV-B are replaced by the corresponding portions of HCV sequences. For example, a chimera in which only one or two of the three structural genes (C, E1 and E2) of GBV-B are replaced by the corresponding HCV structural genes may be made. In one embodiment, nucleic acid sequences comprising the E1 and E2 genes of GBV-B may be replaced by the sequences comprising the HCV E1 and E2 genes. In another embodiment, nucleic acid sequence comprising either the E1 or E2 gene of GBV-B is replaced by sequence encoding either the HCV E1 or E2 gene.

Alternatively, only a fragment of a GBV-B structural gene in the infectious GBV clone may be replaced with the corresponding HCV gene fragments. For example, the amino terminal of the GBV-B E1 gene may be replaced by the corresponding portion of an HCV E1 gene or an amino terminal portion of the GBV-B E2 gene may be replaced by an amino terminal portion of HCV E2 gene tht containing the HVR1 region. As the structural genes of HCV are believed to be important for neutralization, chimeras containing an HCV structural gene(s) or fragment(s) thereof can be used to develop vaccines against HCV.

In yet another embodiment, chimeras in which individual non-structural genes of GBV-B, such as NS3 RNA helicase, NS3 protease, or the NS5B RNA-dependent RNA polymerase are replaced by the corresponding non-structural genes of HCV may be constructed. Such chimeras would, for example, be useful in identifying inhibitors of viral enzyme activity which would be useful as antiviral agents. Of course, it is understood that in order to construct chimeras in which the polyprotein cleavage sites of the GBV-B remain intact, it may be desirable to replace only a fragment of a nonstructural gene of GBV-B with the corresponding HCV gene fragment.

Polypeptides encoded by the nucleic acid sequences of the invention or fragments thereof may be fully or partially purified from viruses produced by cells transfected with the nucleic acid sequences of the invention. In another embodiment, the polypeptide or polypeptides may be produced recombinantly from a fragment of the nucleic acid sequences of the invention. In yet another embodiment, the polypeptides may be chemically synthesized.

The present invention further relates to the in vitro production of GBV-B, mutated GBV-B or chimeric GBV-B/HCV viruses from the nucleic acid sequences of the invention.

In one embodiment, the sequences of the invention can be inserted into an expression vector that functions in eukaryotic cells. Eukaryotic expression vectors suitable for producing high efficiency gene transfer in vivo are well known to those of ordinary skill in the art and include, but are not limited to, plasmids, vaccinia viruses, retroviruses, adenoviruses and adeno-associated viruses.

In another embodiment, the sequences contained in the recombinant expression vector can be transcribed in vitro by methods known to those of ordinary skill in the art in order to produce RNA transcripts which encode the GBV-B of the invention. The GBV-B of the invention may then be produced by transfecting cells by methods known to those of ordinary skill in the art with either the in vitro transcription mixture containing the RNA transcripts or with the recombinant expression vectors containing the nucleic acid sequences described herein.

In assaying the ability of the mutated GBV-B sequences or of the chimeric sequences of the invention to infect tamarins, the virulence phenotype of the virus produced by transfection of tamarins with the sequences of the invention can be monitored by methods known in the art such as measurement of liver enzyme levels (alanine aminotransferase (ALT) or isocitrate dehydrogenase (ICD)) or by histopathology of liver biopsies.

The present invention also relates to the use of the infectious GBV-B sequence, the mutated GBV-B nucleic acid sequences or the chimeric sequences of the invention to identify cell lines capable of supporting the replication of GBV-B or the chimeras of the invention.

Transfection of tissue culture cells with the nucleic acid sequences of the invention may be done by methods of transfection known in the art such as electroporation, precipitation with DEAE-Dextran or calcium phosphate, or incorporation into liposomes.

In one such embodiment, the method comprises the growing of animal cells in vitro and transfecting the cells with the nucleic acid of the invention, then determining if the cells show indicia of GBV-B or HCV infection. Such indicia include the detection of viral antigens in the cell, for example, by immunofluorescence procedures well known in the art; the detection of viral polypeptides by Western blotting using antibodies specific therefor; and the detection of newly transcribed viral RNA within the cells via methods such as RT-PCR. The presence of live, infectious virus particles following such tests may also be shown by injection of cell culture medium or cell lysates into healthy, susceptible animals, with subsequent exhibition of the signs and symptoms of GBV-B infection.

Suitable cells or cell lines for culturing GBV-B or the chimeric GBV-B-HCV include, but are not limited to, lymphocyte and hepatocyte cell lines known in the art.

Alternatively, primary hepatocytes can be cultured, and then infected; or, the hepatocyte cultures could be derived from the livers of infected tamarins. In addition, various immortalization methods known to those of ordinary skill in the art can be used to obtain cell-lines derived from hepatocyte cultures. For example, primary hepatocyte cultures may be fused to a variety of cells to maintain stability.

The invention also provides that the nucleic acid sequences and viruses of the invention be supplied in the form of a kit, alone or in the form of a pharmaceutical composition.

All scientific publication and/or patents cited herein are specifically incorporated by reference. The following examples illustrate various aspects of the invention but are in no way intended to limit the scope thereof.

### EXAMPLES

### Materials and Methods

### Source of GB virus B

Two tamarin pools VR-806, (American Type Culture Collection) and H205, were used for experimental transmission of the GB virus agents to tamarins species *Saguinus mystax* and *Saguinus oedipus*.

### Amplification, cloning and sequence analysis of GBV-B

Viral RNA was extracted from aliquots of the GB 2/94 serum pool or CT 11/91 liver homogenate with the TRIzol system (GIBCO/BRL). Primers used in cDNA synthesis and PCR amplification were based on the genomic sequence of GBV-B published by Simons et al (Simons 1995) shown in SEQ ID NO:3. Long RT-PCR was performed using Superscript II reverse transcriptase (GIBCO/BRL) and the Advantage cDNA polymerase mix (Clontech) as described previously (Tellier 1996). Four subgenomic regions of GBV-B covering the entire published sequence (Simons 1995) were amplified from serum and the PCR products were purified and cloned into pGEM-9Zf(-) (Promega) or pCR2.1 vector (Invitrogen) using standard procedures.

The 5' terminus of GBV-B was amplified from serum by using the rapid amplification of cDNA ends (RACE) with dC or dA tailing (GIBCO/BRL) and GBV-B specific antisense primers. Two different approaches were used to determine the 3' terminal sequence of GBV-B. In one approach, GBV-B RNA extracted from serum was circularized with T4 RNA ligase (Promega) and the 5'-to-3'-end-ligated viral RNA was amplified in RT-PCR using specific GBV-B primers. In the second approach, the 5' end of the negative strand GBV-B RNA extracted from the liver homogenate was amplified using the 5' RACE with dC tailing and GBV-B specific sense primers. The PCR products were cloned directly into pCR2.1-TOPO by using the TOPO TA Cloning Kit (Invitrogen).

The consensus sequence of GBV-B was determined by direct sequencing of PCR products (nucleotides 1-9078 and nucleotides 9130-9359) and by sequence analysis of the clones (nucleotides 1-7135 and nucleotides 7151-9399). Nucleotide positions correspond to those of the infectious clone (pGBB). Analyses of genomic sequences were performed with GeneWorks (Oxford Molecular Group) (Bukh 1995). To determine whether the GenBank data base contained sequences with homology to the GBV-B 3' UTR sequence identified in the present invention, a "Blast" search was performed. The predicted secondary structure of the GBV-B and HCV 3' UTR sequences were determined by the program "mfold" (Genetics Computer Group). Construction of consensus cDNA clones of GBV-B

First, clone pGBB5-1, a consensus clone of GBV-B 2/94 containing the 3' terminus of GBV-B as published by Simons *et al* was constructed (Simons 1995a). The core sequence of the T7 promoter, a 5' guanosine residue and the sequence of GBV-B (9139 nucleotides) were cloned into pGEM-9Zf(-) vector using *Not*I/*Sac*I sites. A *BamH*I site was included at the GBV-B 3' terminus. Digested fragments containing the consensus sequence were purified from subclones and ligated using convenient sites. Next, a second consensus clone of GBV-B, clone pGBB, was constructed by inserting the additional 3' terminal sequence, amplified by PCR from one of the clones obtained by the RACE procedure described above, into pGBB5-1 using *Xma*I (at position 9114) and *BamH*I sites. A *Xho*I site was inserted following the GBV-B 3' terminus. DH5-alpha competent cells (GIBCO BRL) were transformed and selected on LB agar plates containing 100 µg/ml ampicillin (SIGMA) and amplified in LB liquid cultures at 30°C for 18-20 hrs (Yanagi 1997). Each cDNA clone was re-transformed to select a single clone, and large-scale preparation of plasmid DNA was performed with a QIAGEN plasmid Maxi kit as described previously (Yanagi 1997). Each clone was genetically stable since the digestion pattern was as expected following retransformation and the complete sequence was the expected one.

### Intrahepatic transfection of tamarins with transcribed GBV-B RNA

In 100 µl reactions, RNA was transcribed *in vitro* with T7 RNA polymerase (Promega) from 10 µg of linearized template plasmid. The plasmid pGBB5-1 was linearized with *BamH*I (Promega) and the plasmid pGBB was linearized with *Xho*I (Promega). The integrity of the RNA was checked by electrophoresis through agarose gel stained with ethidium bromide. Each transcription mixture was diluted with 400 µl of ice-cold phosphate-buffered saline without calcium or magnesium (SIGMA) and then immediately frozen on dry ice and stored at -80°C. Within 24 hours of synthesis, two transcription mixtures were injected into each tamarin by percutaneous intrahepatic injection guided by ultrasound (Emerson, 1992; Yanagi 1998, 1999). If the tamarin did not become infected, the same transfection was repeated once. All transfected animals were negative for GBV-A_{SM} as determined by the protocol described previously (Bukh 1997a).

### Monitoring of experimental course in tamarins

Serum samples were collected weekly from the tamarins and monitored for liver enzyme levels [alanine aminotransferase (ALT), gamma-glutamyltranspeptidase (GGT), and isocitrate dehydrogenase (ICD)] by standard methods and for GBV-B RNA by a specific reverse transcriptase-polymerase chain reaction (RT-PCR) assay. Total RNA was extracted from 100 µl of serum using the TRIzol reagent. The RNA pellet was resuspended in 10 mM dithiothreitol (DTT) containing 5% (vol/vol) of RNasin (20-40 u/µl) (Promega). The RT-nested PCR was performed with primers from the 5' UTR of GBV-B (external primer pair: 5'-CCT AGC AGG GCG TGG GGG ATT TCC-3' and 5'-AGG TCT GCG TCC TTG GTA GTG ACC-3'; internal primer pair: 5'-GGA TTT CCC CTG CCC GTC TG-3' and 5'-CCC CGG TCT TCC CTA CAG TG-3'). The reverse transcription was performed with avian myeloblastosis virus reverse transcriptase (Promega) and the external anti-sense primer and nested PCR was performed with AmpliTaq DNA polymerase or AmpliTaq Gold DNA polymerase (Perkin Elmer) as described previously (Bukh 1998a). Specificity was confirmed by sequence analysis of selected DNA products. Each set of experiments included a positive control sample (a 10⁻⁶ dilution of GB 8/93, estimated titer 100 genome equivalent (GE)) and appropriate negative control samples. The genome equivalent (GE) titer of GBV-B in positive samples was determined by RT-nested PCR on 10-fold serial dilutions of the extracted RNA (Bukh 1998a). One GE was defined as the number of GBV-B genomes present in the highest dilution positive in RT-nested PCR. The sensitivity of this RT-nested PCR assay for GBV-B was equivalent to that of our RT-nested PCR assay for HCV (Bukh 1998b), for example, conserved NS3 primers which had the same sensitivity for GBV-B as the 5' UTR primers could detect HCV at optimal sensitivity in samples with known HCV genome titer. Testing for GBV-A and GBV-A variants was performed by RT-nested PCR assays as described previously (Bukh 1997a).

The consensus sequence of the complete ORF was determined by direct sequencing of overlapping PCR products obtained by long RT-nested PCR on serum from one of the tamarins infected with RNA transcripts as previously described (Yanagi 1997).

### Example 1

### Transmission of GB Agent in Tamarins

To generate virus pools of the GB agent, tamarins were inoculated intravenously with pooled sera of the eleventh tamarin passage of this agent (Fig. 1). Acute phase sera from a *S*. *mystax* tamarin which developed hepatitis were pooled (GB 8/93) and inoculated into additional *S*. *mystax* tamarins to generate a second pool of acute phase serum (GB 2/94). Both serum pools contained approximately 10⁸ GE/ml of GBV-B and GBV-A. A 10% liver homogenate (CT 11/91) was prepared from a S. oedipus tamarin which developed hepatitis following inoculation with the twelfth passage of the GB agent. The titer of GBV-B in the liver homogenate was approximately 10⁷ GE/ml. The GB 2/94 serum and CT 11/91 liver samples were used as GBV-B cloning sources in the present study.

Inoculation of eight *S*. *mystax* tamarins with ten-fold serial dilutions of the GB 2/94 pool demonstrated that its infectivity titer of GBV-B was 10⁸ tamarin 50% infectious doses (TID₅₀) (Fig. 2). The five GBV-B infected tamarins all developed acute resolving hepatitis characterized by early appearance of viremia (weeks 1 or 2 p.i.), peak viral titers of 10⁷-10⁸ GE/ml and clearance of viremia after 9-16 weeks (Fig. 2). Two of these tamarins (*S*. *mystax* 769 and 777) were infected only with GBV-B and were negative for GBV-A and GBV-A_{SM}, whereas the other three tamarins were infected with both GBV-B and GBV-A_{SM}. A *S*. *mystax* tamarin inoculated with the liver homogenate also developed acute resolving hepatitis with peak GBV-B titers of 10⁷ GE/ml and clearance of viremia after 11 weeks. Likewise, four *S. mystax* tamarins inoculated with dilutions of the GB 8/93 pool developed acute resolving hepatitis with clearance of the GBV-B virus after 11-26 weeks. Thus, GBV-B infection in *S*. *mystax* tamarins is characterized by acute hepatitis, early appearance of viremia, high peak viral titers and viral clearance.

### Example 2

### Novel 3' Terminal Sequence of GBV-B

The consensus sequence of the complete 5' UTR of GBV-B (nucleotides 1-445) was deduced from 13 clones containing nucleotides 1-283 and 3 clones containing nucleotides 31-445. In addition, the entire 5' UTR sequence was determined by direct sequencing of the amplicons. The sequences of the various clones were highly conserved and the consensus 5' UTR sequence of GBV-B from this pool was identical to that of the previously published sequence for GBV-B (Simons 1995a). It is noteworthy that 13 of 15 clones analyzed from the rapid amplification of cDNA ends (RACE) procedure contained the published GBV-B 5' terminus (A residue) and that the same 5' terminus was obtained whether the 5' RACE was performed with dC or dA tailing.

The consensus sequence of the ORF (nucleotides 446-9037) was determined by direct sequencing of PCR products obtained using long RT-PCR (Yanagi 1997). In addition, 3 clones containing nts. 446-7135 (one of these clones had a deletion of nts. 3036-3636), 2 clones containing nts. 2019-3373, 5 clones containing nts. 7151-8261 and 7 clones containing nts. 7521-9037 were analyzed. The sequences of GBV-B clones in this pool were very homogeneous. Evidence of micro-heterogeneity was found at only 70 (0.8%) nucleotide and 36 (1.3%) amino acid positions, scattered throughout the ORF. The proportion of amino acid positions with heterogeneity ranged from 0.5-3.2% in different putative gene regions (lowest in NS3 and NS5B; highest in E2 and NS2). The GBV-B ORF sequence differed from the published sequence of GBV-B (Simons 1995) at 34 (0.4%) nucleotide and 12 (0.4%) deduced amino acid positions, respectively (Table 1).

**Table 1**

| Nucleotide and amino acid differences among GBV-B (Simons 1995a), the consensus sequence of GBV-B recovered from a virus pool used as the cloning source (GBV-B, 2/94) and the infectious clone of GBV-B (pGBB). | | | | | | | |
|---|---|---|---|---|---|---|---|
| Genomic Region* | Position nt [aa] | Nucleotide | | | Amino Acid | | |
| | | | GBV-B | | | GBV-B | |
| | | GBV-B | 2/94 | pGBB | GBV-B | 2/94 | PGBB |
| 5' UTR (1-445) | | | | | | | |
| C (446-913) | | | | | | | |
| E1 (914-1489) | 1030 | C | T | T | | | |
| E2 (1490-2641) | 1498 | T | C (t) | C | | | |
| | 1628 [395] | G | A (g) | A | V | I (V) | I |
| | 2552 [703] | G | A (g) | A | D | N (D) | N |
| | 2562,2563 | C,A | A,C | A.C | P | H | H |
| | [706] | | | | | | |
| | 2566 | T | C | C | | | |
| | 2625 [727] | C | T | T | A | V | V |
| NS2 (2642-3385) | 2647 | C | T (c) | T | | | |
| | 2816 [791] | C | T | T | L | F | F |
| | 2855 [804] | A | G | G | T | A | A |
| | 3235 | A | G | G | | | |
| NS3 (3386-5125) | 3475** | C | C (t) | T | | | |
| | 3760 | C | T (c) | T | | | |
| | 4114 | C | T | T | | | |
| | 4117 | C | A | A | | | |
| | 4177 | T | C | C | | | |
| | 4615 | C | T | T | | | |
| NS4A (5126-5290) | | | | | | | |
| NS4B (5291-6034) | 5329 | C | T | T | | | |
| | 5332 | T | C | C | | | |
| | 5350 | A | C | C | | | |
| | 5455 | C | T (c) | T | | | |
| NS5A (6035-7267) | 6413 | T | A (t) | A | L | M (L) | M |
| | [1990] | | | | | | |
| | 6577 | G | T | T | | | |
| | 6690 | T | C (t) | C | I | T(I) | T |
| | [2082] | | | | | | |
| | 6965 | T | C (t) | C | S | P (S) | P |
| | [2174] | | | | | | |
| | 7015 | A | G (a) | G | | | |
| | 7128 | G | A | A | G | E | E |
| | [2228] | | | | | | |
| | 7138** | A | A | G | | | |
| | 7142 | A | G | G | T | A | A |
| | [2233] | | | | | | |
| NS5B (7268-9037) | 7282 | T | C (t) | C | | | |
| | 7849 | C | A | A | | | |
| | 7852 | C | T | T | | | |
| | 8942 | G | A (g) | A | V | I (V) | I |
| | [2981] | | | | | | |
| | 8971 | T | C | C | | | |
| | 9026 | C | T (c) | T | | | |
| 3' UTR (9038- | 9061 | T | C | C | | | |
| 9399) | | | | | | | |
| | Poly(U) | 27 nts | 11-23 nts | 23 nts | | | |
| | 9134 | Deletion | C | C | | | |
| | 9141-9399 | ND | 259 nts | 259 nts | | | |

| | | | | | | | |
|---|---|---|---|---|---|---|---|
| -Nucleotide positions corresponding to pGBB. Putative gene borders defined as suggested by homology with HCV (Muerhoff 1995). No homology was observed at the NS2-NS3 junction. **Positions that differ between the cloning source (GBV-B 2/94) and the infectious clone of GBV-B (pGBB). The change introduced into pGBB at position 7138 introduced an artificial *Sal*I site. nd: Not determined. Nucleotides and amino acids shown in parenthesis were found as a minor species in the cloning source (GBV-B, 2/94 | | | | | | | |

The sequence for the 3' UTR is shown in Figure 3. Additional 3' UTR sequence was initially identified by performing RT-PCR across 5'-to-3'-end-ligated viral RNA extracted from serum. In all 4 clones with GBV-B sequences, the 5' UTR was truncated compared to the published sequence (simon 1995a). However, whereas one clone (29c) had the exact 3' terminus previously published by Simons *et al*. (Simons 1995a), the three other clones (29a, 29b, 29d) had 150 additional terminal nucleotides. Compared with the published sequence, all four clones had a single nucleotide insertion (C residue) at position 9134. Next, RACE using dC-tailing only was performed on the 5' end of the negative-strand RNA extracted from the liver homogenate. All 11 clones analyzed had additional sequences at the 3' terminus. Compared with the published GBV-B sequence, two clones (gb6, gb23) had 259 additional nucleotides, 8 clones (gb9, gb19, gb20, gb21, gb24, gb25, gb30, gb35) had 236 additional nucleotides and 1 clone (gb8) had 232 additional nucleotides. Moreover, all of these clones had the insertion at position 9134. The 3' UTR sequences among the various clones were highly conserved (Fig. 3). To demonstrate that the terminal 22 nucleotides found only in clones gb6 and gb23 existed in circulating viruses, RT-nested PCR was performed on 10-fold serially diluted RNA extracted from the serum pool GB 2/94 using an RT and external antisense primer deduced from this sequence. GBV-B RNA was detected at a dilution of 10⁻⁷ and the sequence of the amplicon was identical to the sequence recovered from the liver homogenate. Thus, the 3' UTR of GBV-B consists of a short sequence of 30 nucleotides followed by a 11-24 nucleotide-long poly (U) tract (single C residues were observed in GBV-B from the liver homogenate) and a 3' terminal sequence of at least 309 nucleotides. The new GBV-B 3' UTR sequence did not have significant homology to any of the sequences deposited in the GenBank database. A prediction of the secondary structure of the 3' UTR sequence is shown in Figure 4. The most notable feature of the secondary structure is a highly stable stem-loop structure at the very 3' end consisting of 47 nucleotides.

### Example 3

### The pGBB Clone of GBV-B is Infectious in vivo

The infectivity of RNA transcripts from the consensus clone pGBB5-1 which encompassed only the published GBV-B sequence (Simons 1995) was first tested. Within the GBV-B sequence there were no deduced amino acid differences and only 2 nucleotide differences (at nucleotide positions 3475 and 7138) between the consensus sequence of the cloning source (GBV-B 2/94) and the sequence of pGBB5-1 clone. In addition, the 3' UTR of pGBB5-1 had a deletion at nucleotide position 9134 and was missing the 3' terminal 259 nucleotides (Fig. 3). Prior to transcription, the pGBB5-1 clone was linearized at the *BamH*I site with digestion at the exact GBV-B 3' terminus. The RNA transcripts from pGGB5-1 were injected into the liver of two tamarins (*S*. *mystax* 797 and 815). GBV-B RNA was not detected in weekly serum samples collected during 17 weeks of follow-up. As the susceptibility of these two tamarins to GBV-B was subsequently demonstrated by experimental infection using a GBV-B virus pool, the consensus clone pGBB5-1 which lacks the 3' terminal sequence of GBV-B is thus not infectious *in vivo*.

Next, the infectivity of RNA transcripts from the full-length consensus GBV-B cDNA clone pGBB was tested. The pGBB clone was identical to the pGBB5-1 clone except in the 3' UTR. Thus, in addition to a 5' UTR of 445 nucleotides, an ORF of 8592 nucleotides encoding 2864 amino acids and a 3' UTR of 103 nucleotides, the pGBB clone also contains an additional 259 nucleotides in its 3' UTR. pGBB was linearized at the *Xho*I site which added an additional C residue at the 3' end of the transcribed GBV-B RNA. When RNA transcripts from the pGBB clone were injected into the liver of two tamarins (*S*. *mystax* 816 and 817), both tamarins became infected with GBV-B with viremia at week 1 p.i. and peak viral titers of 10⁸ GE/ml (Fig. 5). The consensus sequence of PCR products of the complete ORF, amplified from serum obtained during week 2 p.i. from one tamarin (*S*. *mystax* 817), was identical to the sequence of pGBB, including at the two positions which differed from the consensus sequence of the cloning source and from the published sequence of GBV-B (Table 1). By performing RT-PCR as desired above, it was demonstrated that the very 3' terminal GBV-B sequence of pGBB existed in the circulating viruses in this tamarin. Within two weeks of the transfection both tamarins developed hepatitis with dramatically elevated liver enzyme levels (Fig. 5). Thus, the pGBB clone is infectious *in vivo* whereas the clone pGBB5-1 which lacks the last 259 nucleotides was not.

### References

1. Alter, H. J., Nakatsuji, Y., Melpolder, J., Wages, J., Wesley, R., Shih, J. W.-K. & Kim, J. P. (1997) The incidence of transfusion-associated hepatitis G virus infection and its relation to liver disease. N. Engl. J. Med. 336, 747-754.
2. Alter, M. J., Gallagher, M., Morris, T. T., Moyer, L. A., Meeks, E. L., Krawczynski, K., Kim, J. P. & Margolis, H. S. (1997) Acute non-A-E hepatitis in the United States and the role of hepatitis G virus infection. N. Engl. J. Med. 336, 741-746.
3. Bukh, J. & Apgar, C. L. (1997a) Five new or recently discovered (GBV-A) virus species are indigenous to New World monkeys and may constitute a separate genus of the Flaviviridae. Virology 229, 429-436.
4. Bukh, J., Apgar, C. L., Engle, R., Govindarajan, S., Hegerich, P. A., Tellier, R., Wong, D. C., Elkins, R. & Kew, M. C. (1998b) Experimental infection of chimpanzees with hepatitis C virus of genotype 5a: genetic analysis of the virus and generation of a standardized challenge pool. J. Infect. Dis. 178, 1193-1197.
5. Bukh, J., Apgar, C. L. and Purcell, R. H. (1997b) Natural history of GBV-A and GBV-B in animal models: discovery of indigenous Flaviviridae-like viruses in several species of New World monkeys. In Viral Hepatitis and Liver Disease (Proceedings of the IX Triennial International Symposium on Viral Hepatitis and Liver Disease, Rome, Italy, 1996) (M. Rizzetto, R. H. Purcell, J. L. Gerin, G. Verme, Eds.), pp. 392-395. Edizione Minerva Medica, Turin, Italy.
6. Bukh, J., Kim, J. P., Govindarajan, S., Apgar, C. L., Foung, S. K. H., Wages, J., Yun, A. J., Shapiro, M., Emerson, S. U. & Purcell, R. H. (1998a) Experimental infection of chimpanzees with hepatitis G virus and genetic analysis of the virus. J. Infect. Dis. 177, 855-862.
7. Bukh, J., Miller, R. H. & Purcell, R. H. (1995) Genetic heterogeneity of hepatitis C virus: quasispecies and genotypes. Semin. Liver Dis. 15, 41-63.
8. Deinhardt, F., Holmes, A. W., Capps, R. B. & Popper, H. (1967) Studies on the transmission of human viral hepatitis to marmoset monkeys: Transmission of disease, serial passages, and description of liver lesions. J. Exp. Med. 125, 673-687.
9. Emerson, S. U., , Lewis, M., Govindarajan, S., Shapiro, M., Moskal, T. & Purcell, R. H. (1992) cDNA clone of hepatitis A virus encoding a virulent virus: induction of viral hepatitis by direct nucleic acid transfection of marmosets. J. Virol. 66, 6649-6654.
10. Erker, J. C., Desai, S. M., Leary, T. P., Chalmers, M. L., Montes, C. C. & Mushahwar, I. K. (1998) Genomic analysis of two GB virus A variants isolated from captive monkeys. J. Gen. Virol. 79, 41-45.
11. Frolov, I., McBride, M. S. & Rice, C. M. (1998) Cis-acting RNA elements required for replication of bovine viral diarrhea virus-hepatits C virus 5' nontranslated region chimeras. RNA 4, 1418-1435.
12. Houghton, M. (1996) Hepatitis C viruses. In "Fields Virology" (B. N. Fields, D. M. Knipe, P. M. Howley, et al., Eds.), Third ed., pp. 1035-1058. Lippincott-Raven Publishers, Philadelphia.
13. Kolykhalov, A. A., Feinstone, S. M. & Rice, C. M. (1996) Identification of a highly conserved sequence element at the 3' terminus of hepatitis C virus genome RNA. J. Virol. 70, 3363-3371.
14. Kolykhalov, A. A., Agapov, E. V., Blight, K. J., Mihalik, K., Feinstone, S. M. & Rice, C. M. (1997) Transmission of hepatitis C by intrahepatic inoculation with transcribed RNA. Science 277, 570-574.
15. Lemon, S. M. & Honda, M. (1997) Internal ribosome entry sites within the RNA genomes of hepatits C virus and other flaviviruses. Semin. Virol. 8, 274-288.
16. Linnen, J., Wages, J., Jr., Zhang-Keck, Z. Y., Fry, K. E., Krawczynski, K. Z., Alter, H., Koonin, E., Gallagher, M., Alter, M., Hadziyannis, S., Karayiannis, P., Fung, K., Nakatsuji, Y., Shih, J. W.-K., Young, L., Piatak, M., Jr., Hoover, C., Fernandez, J., Chen, S., Zou, J.-C., Morris, T., Hyams, K. C., Ismay, S., Lifson, J. D., Hess, G., Foung, S. K. H., Thomas, H., Bradley, D., Margolis, H. & Kim, J. P. (1996) Molecular cloning and disease association of hepatitis G virus: A transfusion-transmissible agent. Science 271, 505-508.
17. Lu, H.-H. & Wimmer, E. (1996) Poliovirus chimeras replicating under the translational control of genetic elements of hepatitis C virus reveal unusual properties of the internal ribosomal entry site of hepatitis C virus. Proc. Natl. Acad. Sci. USA 93, 1412-1417.
18. Muerhoff, A. S., Leary, T. P., Simons, J. N., Pilot-Matias, T. J., Dawson, G. J., Erker, J. C., Chalmers, M. L., Schlauder, G. G., Desai, S. M. & Mushahwar I. K. (1995) Genomic organization of GB viruses A and B: Two new members of the Flaviviridae associated with GB agent hepatitis. J. Virol. 69, 5621-5630.
19. Purcell RH. (1993) The discovery of the hepatitis viruses. Gastroenterology 104, 955-963.
20. Rice, C. M. (1996) Flaviviridae: The viruses and their replication, In "Fields Virology". (B. N. Fields, D. M. Knipe, P. M. Howley, et al., Eds.), Third ed., pp. 931-959. Lippincott-Raven Publishers, Philadelphia.
21. Robertson, B., Myers, G., Howard, C., Brettin, T., Bukh, J., Gaschen, B., Gojobori, T., Maertens, G., Mizokami, M., Nainan, O., Netesov, S., Nishioka, K., Shin-i, T., Simmonds, P., Smith, D., Stuyver, L. & Weiner, A. (1998). Classification, nomenclature, and database development for hepatitis C virus (HCV) and related viruses: proposals for standardization. Arch. Virol. 143, 2493-2503.
22. Scarcelli, E., Urbani, A., Sbardellati, A., Tomei, L., De Francesco, R. & Traboni, C. (1997) GB virus B and hepatitis C virus NS3 serine proteases share substrate specificity. J. Virol. 71, 4985-4989.
23. Schlauder, G. G., Dawson, G. J., Simons, J. N., Pilot-Matias, T. J., Gutierrez, R. A., Heynen, C. A., Knigge, M. F., Kurpiewski, G. S., Buijk, S. L., Leary, T. P., Muerhoff, A. S., Desai, S. M. & Mushahwar I. K. (1995) Molecular and serologic analysis in the transmission of the GB hepatitis agents. J. Med. Virol. 46, 81-90.
24. Simons, J. N., Pilot-Matias, T. J., Leary, T. P., Dawson, G. J., Desai, S. M., Schlauder, G. G., Muerhoff, A. S., Erker, J. C., Buijk, S. L., Chalmers, M. L., Van Sant, C. L. & Mushahwar, I. K. (1995a) Identification of two flavivirus-like genomes in the GB hepatitis agent. Proc. Natl. Acad. Sci. USA 92, 3401-3405.
25. Simons, J. N., Leary, T. P., Dawson, G. J., Pilot-Matias, T. J., Muerhoff, A. S., Schlauder, G. G., Desai, S. M. & Mushahwar, I. K. (1995b) Isolation of novel virus-like sequences associated with human hepatitis. Nature Med. 1, 564-569.
26. Tanaka, T., Kato, N., Cho, M.-J. & Shimotohno, K. (1995) A novel sequence found at the 3' terminus of hepatitis C virus genome. Biochem. Biophys. Res. Commun. 215, 744-749.
27. Tellier, R., Bukh, J., Emerson, S. U., Miller, R. H. & Purcell, R. H. (1996) Long PCR and its application to hepatitis viruses: amplification of hepatitis A, hepatitis B, and hepatitis C virus genomes. J. Clin. Microbiol. 34, 3085-3091.
28. Yanagi, M., Purcell, R. H., Emerson, S. U. & Bukh, J. (1997) Transcripts from a single full-length cDNA clone of hepatitis C virus are infectious when directly transfected into the liver of a chimpanzee. Proc. Natl. Acad. Sci. USA 94, 8738-8743.
29. Yanagi, M., St. Claire, M., Shapiro, M., Emerson, S. U., Purcell, R. H. & Bukh, J. (1998) Transcripts of a chimeric cDNA clone of hepatitis C virus genotype 1b are infectious in vivo. Virology 244, 161-172.
30. Yanagi, M., St. Claire, M., Emerson, S. U., Purcell, R. H. & Bukh, J. (1999) In vivo analysis of the 3' untranslated region of the hepatitis C virus after in vitro mutagenesis of an infectious cDNA clone. Proc. Natl. Acad. Sci. USA 96, 2291-2295.

## Claims

1. An isolated nucleic acid molecule which encodes a GB virus-B and which is capable of producing infectious virus when transfected into cells **characterized in that** the 3' UTR sequence is the 3' UTR sequence of SEQ ID NO: 1.

2. The nucleic acid molecule of claim 1, wherein said molecule encodes the amino acid sequence of SEQ ID NO:2.

3. The nucleic acid molecule of claim 2, wherein said molecule comprises the nucleic acid sequence of SEQ ID NO:1.

4. A DNA construct comprising a DNA molecule according to claim 1.

5. A DNA construct comprising a DNA molecule according to claim 3.

6. An isolated RNA transcript of the DNA construct of claims 4 or 5

7. An isolated cell transfected with the DNA construct of claims 4 or 5.

8. An isolated cell transfected with the RNA transcript of claim 6.

9. An isolated GB virus B produced by the cell of claim 7.

10. An isolated GB virus B produced by the cell of claim 8.

11. An isolated GB virus whose genome comprises a nucleic acid molecule according to claim 1.

12. An isolated GB virus whose genome comprises a nucleic acid molecule according to claim 3.

13. A method for producing a GB virus-B comprising transfecting a host cell with the DNA construct of claims 4 or 5.

14. A method for producing a GB virus-B comprising transfecting a host cell with the RNA transcript of claim 6.

15. A composition comprising a nucleic acid molecule of claim 1 suspended in a suitable amount of a pharmaceutically acceptable diluent or excipient.

16. A composition comprising a nucleic acid molecule of claim 3 suspended in a suitable amount of a pharmaceutically acceptable diluent or excipient.

17. A nucleic acid molecule encoding a chimeric virus genome, said genome being a GB virus-B genome encoded by the nucleic acid molecule according to claim 1 in which a 5' UTR sequence of the genome of a GB virus-B is replaced by a corresponding 5' UTR sequence of a hepatitis C virus genome.

18. The nucleic acid molecule of claim 17 wherein the 5' UTR sequence is the IRES sequence.

19. A nucleic acid molecule encoding a chimeric virus genome, said genome being a GB virus -B genome encoded by the nucleic acid molecule according to claim 1 in which the non-structural region of the genome of a GB virus-B has been replaced by the non-structural region of a hepatitis C virus genome.

20. The nucleic acid molecule of claim 19, wherein at least one gene from the non-structural region of the genome of a GB virus-B has been replaced by the corresponding gene from the non-structural region of a hepatitis C virus genome.

21. The nucleic acid molecule of claim 20, wherein the gene from the non-structural region is selected from the group consisting of NS3 protease, NS3 RNA helicase, or NS5B RNA polymerase.

22. A nucleic acid encoding a chimeric virus genome, said genome being a GB virus -B genome encoded by the nucleic acid molecule according to claim 1 in which the structural region of the genome of a GB virus-B has been replaced by the structural region of a hepatitis C virus genome.

23. The nucleic acid molecule of claim 22, wherein at least one gene from the structural region of the genome of a GB virus-B has been replaced by the corresponding gene from the structural region of a hepatitis C virus genome.

24. The nucleic acid molecule of claim 23, wherein the gene from the structural region is selected from the group consisting of E1, E2 or C.

25. The nucleic acid molecule of claim 23, wherein the E1 and E2 genes from the structural region of the genome of a GB virus-B have been replaced by the E1 and E2 genes of a hepatitis C virus genome.

26. The nucleic acid molecule of claim 23, wherein the E1 gene from the structural region of the genome of a GB virus-B has been replaced by the E1 gene of a hepatitis C virus genome.

27. The nucleic acid molecule of claim 23, wherein the E2 gene from the structural region of the genome of a GB virus-B has been replaced by the E2 gene of a hepatitis C virus genome.

28. A DNA construct comprising the DNA molecule of claims 17, 19 or 22.

29. A RNA transcript of the DNA construct of claim 28.

30. A virus whose genome comprises a DNA molecule according to claims 17, 19 or 22.

## Patentansprüche

1. Isoliertes Nukleinsäuremolekül, das ein GB-Virus-B codiert und das, wenn in Zellen transfiziert, ein infektiöses Virus erzeugen kann, **dadurch gekennzeichnet, daß** die 3'-UTR-Sequenz die 3'-UTR-Sequenz der SEQ ID Nr. 1 ist.

2. Nukleinsäuremolekül gemäß Anspruch 1, worin das Molekül die Aminosäuresequenz der SEQ ID Nr. 2 codiert.

3. Nukleinsäuremolekül gemäß Anspruch 2, worin das Molekül die Nukleinsäuresequenz der SEQ ID Nr. 1 umfaßt.

4. DNA-Konstrukt, das ein DNA-Molekül gemäß Anspruch 1 umfaßt.

5. DNA-Konstrukt, das ein DNA-Molekül gemäß Anspruch 3 umfaßt.

6. Isoliertes RNA-Transkript des DNA-Konstrukts gemäß Anspruch 4 oder 5.

7. Isolierte Zelle, die mit dem DNA-Konstrukt gemäß Anspruch 4 oder 5 transfiziert ist.

8. Isolierte Zelle, die mit dem RNA-Transkript gemäß Anspruch 6 transfiziert ist.

9. Isoliertes GB-Virus-B, das durch die Zelle gemäß Anspruch 7 erzeugt wird.

10. Isoliertes GB-Virus-B, das durch die Zelle gemäß Anspruch 8 erzeugt wird.

11. Isoliertes GB-Virus, dessen Genom ein Nukleinsäuremolekül gemäß Anspruch 1 umfaßt.

12. Isoliertes GB-Virus, dessen Genom ein Nukleinsäuremolekül gemäß Anspruch 3 umfaßt.

13. Verfahren zum Erzeugen eines GB-Virus-B, das das Transfizieren einer Wirtszelle mit dem DNA-Konstrukt gemäß Anspruch 4 oder 5 umfaßt.

14. Verfahren zum Erzeugen eines GB-Virus-B, das das Transfizieren einer Wirtszelle mit dem RNA-Transkript gemäß Anspruch 6 umfaßt.

15. Zusammensetzung, die ein Nukleinsäuremolekül gemäß Anspruch 1 suspendiert in einer geeigneten Menge eines pharmazeutisch annehmbaren Verdünnungsstoffs oder Exzipienten umfaßt.

16. Zusammensetzung, die ein Nukleinsäuremolekül gemäß Anspruch 3 suspendiert in einer geeigneten Menge eines pharmazeutisch annehmbaren Verdünnungsstoffs oder Exzipienten umfaßt.

17. Nukleinsäuremolekül, das ein chimäres Virusgenom codiert, wobei das Genom ein GB-Virus-B-Genom ist, das durch das Nukleinsäuremolekül gemäß Anspruch 1 codiert wird, worin eine 5'-UTR-Sequenz des Genoms eines GB-Virus-B durch eine entsprechende 5'-UTR-Sequenz eines Hepatitis-C-Virus-Genoms ersetzt ist.

18. Nukleinsäuremolekül gemäß Anspruch 17, worin die 5'-UTR-Sequenz die IRES-Sequenz ist.

19. Nukleinsäuremolekül, das ein chimäres Virusgenom codiert, wobei das Genom ein GB-Virus-B-Genom ist, das durch das Nukleinsäuremolekül gemäß Anspruch 1 codiert wird, worin die Nichtstrukturregion des Genoms eines GB-Virus-B durch die Nichtstrukturregion eines Hepatitis-C-Virus-Genoms ersetzt worden ist.

20. Nukleinsäuremolekül gemäß Anspruch 19, worin wenigstens ein Gen der Nichtstrukturregion des Genoms eines GB-Virus-B durch das entsprechende Gen der Nichtstrukturregion eines Hepatitis-C-Virus-Genoms ersetzt worden ist.

21. Nukleinsäuremolekül gemäß Anspruch 20, worin das Gen der Nichtstrukturregion ausgewählt ist aus der Gruppe bestehend aus NS3-Protease, NS3-RNA-Helicase oder NS5B-RNA-Polymerase.

22. Nukleinsäure, die ein chimäres Virusgenom codiert, wobei das Genom ein GB-Virus-B-Genom ist, das durch das Nukleinsäuremolekül gemäß Anspruch 1 codiert wird, worin die Strukturregion des Genoms eines GB-Virus-B durch die Strukturregion eines Hepatitis-C-Virus-Genoms ersetzt worden ist.

23. Nukleinsäuremolekül gemäß Anspruch 22, worin wenigstens ein Gen der Strukturregion des Genoms eines GB-Virus-B durch das entsprechende Gen der Strukturregion eines Hepatitis-C-Virus-Genoms ersetzt worden ist.

24. Nukleinsäuremolekül gemäß Anspruch 23, worin das Gen der Strukturregion ausgewählt ist aus der Gruppe bestehend aus E1, E2 oder C.

25. Nukleinsäuremolekül gemäß Anspruch 23, worin die E1- und E2-Gene der Strukturregion des Genoms eines GB-Virus-B durch die E1- und E2-Gene eines Hepatitis-C-Virus-Genoms ersetzt worden sind.

26. Nukleinsäuremolekül gemäß Anspruch 23, worin das E1-Gen der Strukturregion des Genoms eines GB-Virus-B durch das E1-Gen eines Hepatitis-C-Virus-Genoms ersetzt worden ist.

27. Nukleinsäuremolekül gemäß Anspruch 23, worin das E2-Gen der Strukturregion des Genoms eines GB-Virus-B durch das E2-Gen eines Hepatitis-C-Virus-Genoms ersetzt worden ist.

28. DNA-Konstrukt, das das DNA-Molekül gemäß Anspruch 17, 19 oder 22 umfaßt.

29. RNA-Transkript des DNA-Konstrukts gemäß Anspruch 28.

30. Virus, dessen Genom ein DNA-Molekül gemäß Anspruch 17, 19 oder 22 umfaßt.

## Revendications

1. Molécule d'acide nucléique isolée qui code pour un virus GB-B et qui est capable de produire un virus infectieux lorsqu'elle est transfectée dans des cellules, **caractérisée en ce que** la séquence 3' UTR est la séquence 3' UTR de SEQ ID N° : 1.

2. Molécule d'acide nucléique selon la revendication 1, ladite molécule codant pour la séquence d'acides aminés de SEQ ID N° : 2.

3. Molécule d'acide nucléique selon la revendication 2, ladite molécule comprenant la séquence d'acide nucléique de SEQ ID N° : 1.

4. Construction d'ADN comprenant une molécule d'ADN selon la revendication 1.

5. Construction d'ADN comprenant une molécule d'ADN selon la revendication 3.

6. Produit de transcription d'ARN isolé de la construction d'ADN selon les revendications 4 ou 5.

7. Cellule isolée transfectée avec la construction d'ADN selon les revendications 4 ou 5.

8. Cellule isolée transfectée avec le produit de transcription d'ARN selon la revendication 6.

9. Virus GB-B isolé produit par la cellule de la revendication 7.

10. Virus GB-B isolé produit par la cellule de la revendication 8.

11. Virus GB isolé dont le génome comprend une molécule d'acide nucléique selon la revendication 1.

12. Virus GB isolé dont le génome comprend une molécule d'acide nucléique selon la revendication 3.

13. Procédé de production d'un virus GB-B comprenant la transfection d'une cellule hôte avec la construction d'ADN selon les revendications 4 ou 5.

14. Procédé de production d'un virus GB-B comprenant la transfection d'une cellule hôte avec le produit de transcription d'ARN selon la revendication 6.

15. Composition comprenant une molécule d'acide nucléique selon la revendication 1, en suspension dans une quantité appropriée d'un diluant ou d'un excipient pharmaceutiquement acceptable.

16. Composition comprenant une molécule d'acide nucléique selon la revendication 3, en suspension dans une quantité appropriée d'un diluant ou d'un excipient pharmaceutiquement acceptable.

17. Molécule d'acide nucléique codant pour le génome d'un virus chimérique, ledit génome étant le génome du virus GB-B codé par la molécule d'acide nucléique selon la revendication 1, dans laquelle une séquence 5' UTR du génome d'un virus GB-B est remplacée par une séquence 5' UTR correspondante du génome d'un virus de l'hépatite C.

18. Molécule d'acide nucléique selon la revendication 17, dans laquelle ladite séquence 5' UTR est la séquence IRES.

19. Molécule d'acide nucléique codant pour un génome de virus chimérique, ledit génome étant un génome de virus GB-B codé par la molécule d'acide nucléique selon la revendication 1, dans laquelle la région non structurelle du génome d'un virus GB-B a été remplacée par la région non structurelle du génome d'un virus de l'hépatite C.

20. Molécule d'acide nucléique selon la revendication 19, dans laquelle au moins un gène de la région non structurelle du génome d'un virus GB-B a été remplacé par le gène correspondant de la région non structurelle du génome d'un virus de l'hépatite C.

21. Molécule d'acide nucléique selon la revendication 20, dans laquelle le gène de la région non structurelle est choisi dans le groupe comprenant la NS3 protéase, la NS3 ARN hélicase ou la NS5B ARN polymérase.

22. Acide nucléique codant pour le génome d'un virus chimérique, ledit génome étant le génome d'un virus GB-B codé par la molécule d'acide nucléique selon la revendication 1 dans laquelle la région structurelle du génome d'un virus GB-B a été remplacée par la région structurelle du génome d'un virus de l'hépatite C.

23. Molécule d'acide nucléique selon la revendication 22, dans laquelle au moins un gène de la région structurelle du génome d'un virus GB-B a été remplacé par le gène correspondant de la région structurelle du génome d'un virus de l'hépatite C.

24. Molécule d'acide nucléique selon la revendication 23, dans laquelle le gène de la région structurelle est choisi dans le groupe comprenant E1, E2 ou C.

25. Molécule d'acide nucléique selon la revendication 23, dans laquelle les gènes E1 et E2 de la région structurelle du génome d'un virus GB-B ont été remplacés par les gènes E1 et E2 du génome du virus de l'hépatite C.

26. Molécule d'acide nucléique selon la revendication 23, dans laquelle le gène E1 de la région structurelle du génome d'un virus GB-B a été remplacé par le gène E1 du génome d'un virus de l'hépatite C.

27. Molécule d'acide nucléique selon la revendication 23, dans laquelle le gène E2 de la région structurelle du génome d'un virus GB-B a été remplacé par le gène E2 du génome d'un virus de l'hépatite C.

28. Construction d'ADN comprenant la molécule d'ADN selon les revendications 17, 19 ou 22.

29. Produit de transcription d'ARN de la construction d'ADN selon la revendication 28.

30. Virus dont le génome comprend une molécule d'ADN selon les revendications 17, 19 ou 22.


### SEQUENCE LISTING

<110> Bukh, Jens
   Purcell, Robert
   Yanagi, Masayuki
   Emerson, Suzanne
<120> Infectious cDNA Clone of GB virus B and Uses Thereof
<130> 2026-4308PC
<140> TBA
   <141> 2000-06-02
<150> 60/137,694
   <151> 1999-06-04
<160> 5
<170> PatentIn Ver. 2.1
<210> 1
   <211> 9399
   <212> DNA
   <213> GBV-B virus
<400> 1
   accacaaaca ctccagtttg ttacactccg ctaggaatgc tcctggagca ccccccctag 60
   cagggcgtgg gggatttccc ctgcccgtct gcagaagggt ggagccaacc accttagtat 120
   gtaggcggcg ggactcatga cgctcgcgtg atgacaagcg ccaagcttga cttggatggc 180
   cctgatgggc gttcatgggt tcggtggtgg tggcgcttta ggcagcctcc acgcccacca 240
   cctcccagat agagcggcgg cactgtaggg aagaccgggg accggtcact accaaggacg 300
   cagacctctt tttgagtatc acgcctccgg aagtagttgg gcaagcccac ctatatgtgt 360
   tgggatggtt ggggttagcc atccataccg tactgcctga tagggtcctt gcgaggggat 420
   ctgggagtct cgtagaccgt agcacatgcc tgttatttct actcaaacaa gtcctgtacc 480
   tgcgcccaga acgcgcaaga acaagcagac gcaggcttca tatcctgtgt ccattaaaac 540
   atctgttgaa aggggacaac gagcaaagcg caaagtccag cgcgatgctc ggcctcgtaa 600
   ttacaaaatt gctggtatcc atgatggctt gcagacattg gctcaggctg ctttgccagc 660
   tcatggttgg ggacgccaag accctcgcca taagtctcgc aatcttggaa tccttctgga 720
   ttaccctttg gggtggattg gtgatgttac aactcacaca cctctagtag gcccgctggt 780
   ggcaggagcg gtcgttcgac cagtctgcca gatagtacgc ttgctggagg atggagtcaa 840
   ctgggctact ggttggttcg gtgtccacct ttttgtggta tgtctgctat ctttggcctg 900
   tccctgtagt ggggcgcggg tcactgaccc agacacaaat accacaatcc tgaccaattg 960
   ctgccagcgt aatcaggtta tctattgttc tccttccact tgcctacacg agcctggttg 1020
   tgtgatctgt gcggacgagt gctgggttcc cgccaatccg tacatctcac acccttccaa 1080
   ttggactggc acggactcct tcttggctga ccacattgat tttgttatgg gcgctcttgt 1140
   gacctgtgac gcccttgaca ttggtgagtt gtgtggtgcg tgtgtattag tcggtgactg 1200
   gcttgtcagg cactggctta ttcacataga cctcaatgaa actggtactt gttacctgga 1260
   agtgcccact ggaatagatc ctgggttcct agggtttatc gggtggatgg ccggcaaggt 1320
   cgaggctgtc atcttcttga ccaaactggc ttcacaagta ccatacgcta ttgcgactat 1380
   gtttagcagt gtacactacc tggcggttgg cgctctgatc tactatgcct ctcggggcaa 1440
   gtggtatcag ttgctcctag cgcttatgct ttacatagaa gcgacctctg gaaaccccat 1500
   cagggtgccc actggatgct caatagctga gttttgctcg cctttgatga taccatgtcc 1560
   ttgccactct tatttgagtg agaatgtgtc agaagtcatt tgttacagtc caaagtggac 1620
   caggcctatc actctagagt ataacaactc catatcttgg tacccctata caatccctgg 1680
   tgcgagggga tgtatggtta aattcaaaaa taacacatgg ggttgctgcc gtattcgcaa 1740
   tgtgccatcg tactgcacta tgggcactga tgcagtgtgg aacgacactc gcaacactta 1800
   cgaagcatgc ggtgtaacac catggctaac aaccgcatgg cacaacggct cagccctgaa 1860
   attggctata ttacaatacc ctgggtctaa agaaatgttt aaacctcata attggatgtc 1920
   aggccatttg tattttgagg gatcagatac ccctatagtt tacttttatg accctgtgaa 1980
   ttccactctc ctaccaccgg agaggtgggc taggttgccc ggtaccccac ctgtggtacg 2040
   tggttcttgg ttacaggttc cgcaagggtt ttacagtgat gtgaaagacc tagccacagg 2100
   attgatcacc aaagacaaag cctggaaaaa ttatcaggtc ttatattccg ccacgggtgc 2160
   tttgtctctt acgggagtta ccaccaaggc cgtggtgcta attctgttgg ggttgtgtgg 2220
   cagcaagtat cttattttag cctacctctg ttacttgtcc ctttgttttg ggcgcgcttc 2280
   tggttaccct ttgcgtcctg tgctcccatc ccagtcgtat ctccaagctg gctgggatgt 2340
   tttgtctaaa gctcaagtag ctccttttgc tttgattttc ttcatctgtt gctatctccg 2400
   ctgcaggcta cgttatgctg cccttttagg gtttgtgccc atggctgcgg gcttgcccct 2460
   aactttcttt gttgcagcag ctgctgccca accagattat gactggtggg tgcgactgct 2520
   agtggcaggg ttagttttgt gggccggccg taaccgtggt caccgcatag ctctgcttgt 2580
   aggtccttgg cctctggtag cgcttttaac cctcttgcat ttggttacgc ctgcttcagc 2640
   ttttgatacc gagataattg gagggctgac aataccacct gtagtagcat tagttgtcat 2700
   gtctcgtttt ggcttctttg ctcacttgtt acctcgctgt gctttagtta actcctatct 2760
   ttggcaacgt tgggagaatt ggttttggaa cgttacacta agaccggaga ggtttttcct 2820
   tgtgctggtt tgtttccccg gtgcgacata tgacgcgctg gtgactttct gtgtgtgtca 2880
   cgtagctctt ctatgtttaa catccagtgc agcatcgttc tttgggactg actctagggt 2940
   tagggcccat agaatgttgg tgcgtctcgg aaagtgtcat gcttggtatt ctcattatgt 3000
   tcttaagttt ttcctcttag tgtttggtga gaatggtgtg tttttctata agcacttgca 3060
   tggtgatgtc ttgcctaatg attttgcctc gaaactacca ttgcaagagc catttttccc 3120
   ttttgaaggc aaggcaaggg tctataggaa tgaaggaaga cgcttggcgt gtggggacac 3180
   ggttgatggt ttgcccgttg ttgcgcgtct cggcgacctt gttttcgcag ggttggctat 3240
   gccgccagat gggtgggcca ttaccgcacc ttttacgctg cagtgtctct ctgaacgtgg 3300
   cacgctgtca gcgatggcag tggtcatgac tggtatagac ccccgaactt ggactggaac 3360
   tatcttcaga ttaggatctc tggccactag ctacatggga tttgtttgtg acaacgtgtt 3420
   gtatactgct caccatggca gcaaggggcg ccggttggct catcccacag gctctataca 3480
   cccaataacc gttgacgcgg ctaatgacca ggacatctat caaccaccat gtggagctgg 3540
   gtcccttact cggtgctctt gcggggagac caaggggtat ctggtaacac gactggggtc 3600
   attggttgag gtcaacaaat ccgatgaccc ttattggtgt gtgtgcgggg cccttcccat 3660
   ggctgttgcc aagggttctt caggtgcccc gattctgtgc tcctccgggc atgttattgg 3720
   gatgttcacc gctgctagaa attctggcgg ttcagtcagt cagattaggg ttaggccgtt 3780
   ggtgtgtgct ggataccatc cccagtacac agcacatgcc actcttgata caaaacctac 3840
   tgtgcctaac gagtattcag tgcaaatttt aattgccccc actggcagcg gcaagtcaac 3900
   caaattacca ctttcttaca tgcaggagaa gtatgaggtc ttggtcctaa atcccagtgt 3960
   ggctacaaca gcatcaatgc caaagtacat gcacgcgacg tacggcgtga atccaaattg 4020
   ctattttaat ggcaaatgta ccaacacagg ggcttcactt acgtacagca catatggcat 4080
   gtacctgacc ggagcatgtt cccggaacta tgatgtaatc atttgtgacg aatgccatgc 4140
   taccgatgca accaccgtgt tgggcattgg aaaggtccta accgaagctc catccaaaaa 4200
   tgttaggcta gtggttcttg ccacggctac cccccctgga gtaatcccta caccacatgc 4260
   caacataact gagattcaat taaccgatga aggcactatc ccctttcatg gaaaaaagat 4320
   taaggaggaa aatctgaaga aagggagaca ccttatcttt gaggctacca aaaaacactg 4380
   tgatgagctt gctaacgagt tagctcgaaa gggaataaca gctgtctctt actatagggg 4440
   atgtgacatc tcaaaaatcc ctgagggcga ctgtgtagta gttgccactg atgccttgtg 4500
   tacagggtac actggtgact ttgattccgt gtatgactgc agcctcatgg tagaaggcac 4560
   atgccatgtt gaccttgacc ctactttcac catgggtgtt cgtgtgtgcg gggtttcagc 4620
   aatagttaaa ggccagcgta ggggccgcac aggccgtggg agagctggca tatactacta 4680
   tgtagacggg agttgtaccc cttcgggtat ggttcctgaa tgcaacattg ttgaagcctt 4740
   cgacgcagcc aaggcatggt atggtttgtc atcaacagaa gctcaaacta ttctggacac 4800
   ctatcgcacc caacctgggt tacctgcgat aggagcaaat ttggacgagt gggctgatct 4860
   cttttctatg gtcaaccccg aaccttcatt tgtcaatact gcaaaaagaa ctgctgacaa 4920
   ttatgttttg ttgactgcag cccaactaca actgtgtcat cagtatggct atgctgctcc 4980
   caatgacgca ccacggtggc agggagcccg gcttgggaaa aaaccttgtg gggttctgtg 5040
   gcgcttggac ggcgctgacg cctgtcctgg cccagagccc agcgaggtga ccagatacca 5100
   aatgtgcttc actgaagtca atacttctgg gacagccgca ctcgctgttg gcgttggagt 5160
   ggctatggct tatctagcca ttgacacttt tggcgccact tgtgtgcggc gttgctggtc 5220
   tattacatca gtccctaccg gtgctactgt cgccccagtg gttgacgaag aagaaatcgt 5280
   ggaggagtgt gcatcattca ttcccttgga ggccatggtt gctgcaattg acaagctgaa 5340
   gagtacaatc accacaacta gtcctttcac attggaaacc gcccttgaaa aacttaacac 5400
   ctttcttggg cctcatgcag ctacaatcct tgctatcata gagtattgct gtggtttagt 5460
   cactttacct gacaatccct ttgcatcatg cgtgtttgct ttcattgcgg gtattactac 5520
   cccactacct cacaagatca aaatgttcct gtcattattt ggaggcgcaa ttgcgtccaa 5580
   gcttacagac gctagaggcg cactggcgtt catgatggcc ggggctgcgg gaacagctct 5640
   tggtacatgg acatcggtgg gttttgtctt tgacatgcta ggcggctatg ctgccgcctc 5700
   atccactgct tgcttgacat ttaaatgctt gatgggtgag tggcccacta tggatcagct 5760
   tgctggttta gtctactccg cgttcaatcc ggccgcagga gttgtgggcg tcttgtcagc 5820
   ttgtgcaatg tttgctttga caacagcagg gccagatcac tggcccaaca gacttcttac 5880
   tatgcttgct aggagcaaca ctgtatgtaa tgagtacttt attgccactc gtgacatccg 5940
   caggaagata ctgggcattc tggaggcatc taccccctgg agtgtcatat cagcttgcat 6000
   ccgttggctc cacaccccga cggaggatga ttgcggcctc attgcttggg gtctagagat 6060
   ttggcagtat gtgtgcaatt tctttgtgat ttgctttaat gtccttaaag ctggagttca 6120
   gagcatggtt aacattcctg gttgtccttt ctacagctgc cagaaggggt acaagggccc 6180
   ctggattgga tcaggtatgc tccaagcacg ctgtccatgc ggtgctgaac tcatcttttc 6240
   tgttgagaat ggttttgcaa aactttacaa aggacccaga acttgttcaa attactggag 6300
   aggggctgtt ccagtcaacg ctaggctgtg tgggtcggct agaccggacc caactgattg 6360
   gactagtctt gtcgtcaatt atggcgttag ggactactgt aaatatgaga aaatgggaga 6420
   tcacattttt gttacagcag tatcctctcc aaatgtctgt ttcacccagg tgcccccaac 6480
   cttgagagct gcagtggccg tggacggcgt acaggttcag tgttatctag gtgagcccaa 6540
   aactccttgg acgacatctg cttgctgtta cggtcctgac ggtaagggta aaactgttaa 6600
   gcttcccttc cgcgttgacg gtcacacacc tggtgtgcgc atgcaactta atttgcgtga 6660
   tgcacttgag acaaatgact gtaattccac aaacaacact cctagtgatg aagccgcagt 6720
   gtccgctctt gttttcaaac aggagttgcg gcgtacaaac caattgcttg aggcaatttc 6780
   agctggcgtt gacaccacca aactgccagc cccctccatc gaagaggtag tggtaagaaa 6840
   gcgccagttc cgggcaagaa ctggttcgct taccttgcct ccccctccga gatccgtccc 6900
   aggagtgtca tgtcctgaaa gcctgcaacg aagtgacccg ttagaaggtc cttcaaacct 6960
   ccctccttca ccacctgttc tacagttggc catgccgatg cccctgttgg gagcgggtga 7020
   gtgtaaccct ttcactgcaa ttggatgtgc aatgaccgaa acaggcggag gccctgatga 7080
   tttacccagt taccctccca aaaaggaggt ctctgaatgg tcagacgaaa gttggtcgac 7140
   ggctacaacc gcttccagct acgttactgg ccccccgtac cctaagatac ggggaaagga 7200
   ttccactcag tcagcccccg ccaaacggcc tacaaaaaag aagttgggaa agagtgagtt 7260
   ttcgtgcagc atgagctaca cctggaccga cgtgattagc ttcaaaactg cttctaaagt 7320
   tctgtctgca actcgggcca tcactagtgg tttcctcaaa caaagatcat tggtgtatgt 7380
   gactgagccg cgggatgcgg agcttagaaa acaaaaagtc actattaata gacaacctct 7440
   gttcccccca tcataccaca agcaagtgag attggctaag gaaaaagctt caaaagttgt 7500
   cggtgtcatg tgggactatg atgaagtagc agctcacacg ccctctaagt ctgctaagtc 7560
   ccacatcact ggccttcggg gcactgatgt tcgttctgga gcagcccgca aggctgttct 7620
   ggacttgcag aagtgtgtcg aggcaggtga gataccgagt cattatcggc aaactgtgat 7680
   agttccaaag gaggaggtct tcgtgaagac cccccagaaa ccaacaaaga aacccccaag 7740
   gcttatctcg tacccccacc ttgaaatgag atgtgttgag aagatgtact acggtcaggt 7800
   tgctcctgac gtagttaaag ctgtcatggg agatgcgtac gggtttgtag atccacgtac 7860
   ccgtgtcaag cgtctgttgt cgatgtggtc acccgatgca gtcggagcca catgcgatac 7920
   agtgtgtttt gacagtacca tcacacccga ggatatcatg gtggagacag acatctactc 7980
   agcagctaaa ctcagtgacc aacaccgagc tggcattcac accattgcga ggcagttata 8040
   cgctggagga ccgatgatcg cttatgatgg ccgagagatc ggatatcgta ggtgtaggtc 8100
   ttccggcgtc tatactacct caagttccaa cagtttgacc tgctggctga aggtaaatgc 8160
   tgcagccgaa caggctggca tgaagaaccc tcgcttcctt atttgcggcg atgattgcac 8220
   cgtaatttgg aagagcgccg gagcagatgc agacaaacaa gcaatgcgtg tctttgctag 8280
   ctggatgaag gtgatgggtg caccacaaga ttgtgtgcct caacccaaat acagtttgga 8340
   agaattaaca tcatgctcat caaatgttac ctctggaatt accaaaagtg gcaagcctta 8400
   ctactttctt acaagagatc ctcgtatccc ccttggcagg tgctctgccg agggtctggg 8460
   atacaacccc agtgctgcgt ggattgggta tctaatacat cactacccat gtttgtgggt 8520
   tagccgtgtg ttggctgtcc atttcatgga gcagatgctc tttgaggaca aacttcccga 8580
   gactgtgacc tttgactggt atgggaaaaa ttatacggtg cctgtagaag atctgcccag 8640
   catcattgct ggtgtgcacg gtattgaggc tttctcggtg gtgcgctaca ccaacgctga 8700
   gatcctcaga gtttcccaat cactaacaga catgaccatg ccccccctgc gagcctggcg 8760
   aaagaaagcc agggcggtcc tcgccagcgc caagaggcgt ggcggagcac acgcaaaatt 8820
   ggctcgcttc cttctctggc atgctacatc tagacctcta ccagatttgg ataagacgag 8880
   cgtggctcgg tacaccactt tcaattattg tgatgtttac tccccggagg gggatgtgtt 8940
   tattacacca cagagaagat tgcagaagtt ccttgtgaag tatttggctg tcattgtttt 9000
   tgccctaggg ctcattgctg ttggattagc catcagctga acccccaaat tcaaaattaa 9060
   ctaacagttt tttttttttt tttttttttt agggcagcgg caacagggga gaccccgggc 9120
   ttaacgaccc cgccgatgtg agtttggcga ccatggtgga tcagaaccgt ttcgggtgaa 9180
   gccatggtct gaaggggatg acgtcccttc tggctcatcc acaaaaaccg tctcgggtgg 9240
   gtgaggagtc ctggctgtgt gggaagcagt cagtataatt cccgtcgtgt gtggtgacgc 9300
   ctcacgacgt atttgtccgc tgtgcagagc gtagtaccaa gggctgcacc ccggtttttg 9360
   ttccaagcgg agggcaaccc ccgcttggaa ttaaaaact                        9399
<210> 2
   <211> 2864
   <212> PRT
   <213> GBV-B virus
<400> 2
   Met Pro Val Ile Ser Thr Gln Thr Ser Pro Val Pro Ala Pro Arg Thr
   1 5 10 15
   Arg Lys Asn Lys Gln Thr Gln Ala Ser Tyr Pro Val Ser Ile Lys Thr
   20 25 30
   Ser Val Glu Arg Gly Gln Arg Ala Lys Arg Lys Val Gln Arg Asp Ala
   35 40 45
   Arg Pro Arg Asn Tyr Lys Ile Ala Gly Ile His Asp Gly Leu Gln Thr
   50 55 60
   Leu Ala Gln Ala Ala Leu Pro Ala His Gly Trp Gly Arg Gln Asp Pro
   65 70 75 80
   Arg His Lys Ser Arg Asn Leu Gly Ile Leu Leu Asp Tyr Pro Leu Gly
   85 90 95
   Trp Ile Gly Asp Val Thr Thr His Thr Pro Leu Val Gly Pro Leu Val
   100 105 110
   Ala Gly Ala Val Val Arg Pro Val Cys Gln Ile Val Arg Leu Leu Glu
   115 120 125
   Asp Gly Val Asn Trp Ala Thr Gly Trp Phe Gly Val His Leu Phe Val
   130 135 140
   Val Cys Leu Leu Ser Leu Ala Cys Pro Cys Ser Gly Ala Arg Val Thr
   145 150 155 160
   Asp Pro Asp Thr Asn Thr Thr Ile Leu Thr Asn Cys Cys Gln Arg Asn
   165 170 175
   Gln Val Ile Tyr Cys Ser Pro Ser Thr Cys Leu His Glu Pro Gly Cys
   180 185 190
   Val Ile Cys Ala Asp Glu Cys Trp Val Pro Ala Asn Pro Tyr Ile Ser
   195 200 205
   His Pro Ser Asn Trp Thr Gly Thr Asp Ser Phe Leu Ala Asp His Ile
   210 215 220
   Asp Phe Val Met Gly Ala Leu Val Thr Cys Asp Ala Leu Asp Ile Gly
   225 230 235 240
   Glu Leu Cys Gly Ala Cys Val Leu Val Gly Asp Trp Leu Val Arg His
   245 250 255
   Trp Leu Ile His Ile Asp Leu Asn Glu Thr Gly Thr Cys Tyr Leu Glu
               260                 265                 270
   Val Pro Thr Gly Ile Asp Pro Gly Phe Leu Gly Phe Ile Gly Trp Met
           275                 280                 285
   Ala Gly Lys Val Glu Ala Val Ile Phe Leu Thr Lys Leu Ala Ser Gln
       290                 295                 300
   Val Pro Tyr Ala Ile Ala Thr Met Phe Ser Ser Val His Tyr Leu Ala
   305                 310                 315                 320
   Val Gly Ala Leu Ile Tyr Tyr Ala Ser Arg Gly Lys Trp Tyr Gln Leu
                   325                 330                 335
   Leu Leu Ala Leu Met Leu Tyr Ile Glu Ala Thr Ser Gly Asn Pro Ile
               340                 345                 350
   Arg Val Pro Thr Gly Cys Ser Ile Ala Glu Phe Cys Ser Pro Leu Met
           355                 360                 365
   Ile Pro Cys Pro Cys His Ser Tyr Leu Ser Glu Asn Val Ser Glu Val
       370                 375                 380
   Ile Cys Tyr Ser Pro Lys Trp Thr Arg Pro Ile Thr Leu Glu Tyr Asn
   385                 390                 395                 400
   Asn Ser Ile Ser Trp Tyr Pro Tyr Thr Ile Pro Gly Ala Arg Gly Cys
                   405                 410                 415
   Met Val Lys Phe Lys Asn Asn Thr Trp Gly Cys Cys Arg Ile Arg Asn
               420                 425                 430
   Val Pro Ser Tyr Cys Thr Met Gly Thr Asp Ala Val Trp Asn Asp Thr
           435                 440                 445
   Arg Asn Thr Tyr Glu Ala Cys Gly Val Thr Pro Trp Leu Thr Thr Ala
       450                 455                 460
   Trp His Asn Gly Ser Ala Leu Lys Leu Ala Ile Leu Gln Tyr Pro Gly
   465                 470                 475                 480
   Ser Lys Glu Met Phe Lys Pro His Asn Trp Met Ser Gly His Leu Tyr
                   485                 490                 495
   Phe Glu Gly Ser Asp Thr Pro Ile Val Tyr Phe Tyr Asp Pro Val Asn
               500                 505                 510
   Ser Thr Leu Leu Pro Pro Glu Arg Trp Ala Arg Leu Pro Gly Thr Pro
   515 520 525
   Pro Val Val Arg Gly Ser Trp Leu Gln Val Pro Gln Gly Phe Tyr Ser
   530 535 540
   Asp Val Lys Asp Leu Ala Thr Gly Leu Ile Thr Lys Asp Lys Ala Trp
   545 550 555 560
   Lys Asn Tyr Gln Val Leu Tyr Ser Ala Thr Gly Ala Leu Ser Leu Thr
   565 570 575
   Gly Val Thr Thr Lys Ala Val Val Leu Ile Leu Leu Gly Leu Cys Gly
   580 585 590
   Ser Lys Tyr Leu Ile Leu Ala Tyr Leu Cys Tyr Leu Ser Leu Cys Phe
   595 600 605
   Gly Arg Ala Ser Gly Tyr Pro Leu Arg Pro Val Leu Pro Ser Gln Ser
   610 615 620
   Tyr Leu Gln Ala Gly Trp Asp Val Leu Ser Lys Ala Gln Val Ala Pro
   625 630 635 640
   Phe Ala Leu Ile Phe Phe Ile Cys Cys Tyr Leu Arg Cys Arg Leu Arg
   645 650 655
   Tyr Ala Ala Leu Leu Gly Phe Val Pro Met Ala Ala Gly Leu Pro Leu
   660 665 670
   Thr Phe Phe Val Ala Ala Ala Ala Ala Gln Pro Asp Tyr Asp Trp Trp
   675 680 685
   Val Arg Leu Leu Val Ala Gly Leu Val Leu Trp Ala Gly Arg Asn Arg
   690 695 700
   Gly His Arg Ile Ala Leu Leu Val Gly Pro Trp Pro Leu Val Ala Leu
   705 710 715 720
   Leu Thr Leu Leu His Leu Val Thr Pro Ala Ser Ala Phe Asp Thr Glu
   725 730 735
   Ile Ile Gly Gly Leu Thr Ile Pro Pro Val Val Ala Leu Val Val Met
   740 745 750
   Ser Arg Phe Gly Phe Phe Ala His Leu Leu Pro Arg Cys Ala Leu Val
   755 760 765
   Asn Ser Tyr Leu Trp Gln Arg Trp Glu Asn Trp Phe Trp Asn Val Thr
   770 775 780
   Leu Arg Pro Glu Arg Phe Phe Leu Val Leu Val Cys Phe Pro Gly Ala
   785 790 795 800
   Thr Tyr Asp Ala Leu Val Thr Phe Cys Val Cys His Val Ala Leu Leu
   805 810 815
   Cys Leu Thr Ser Ser Ala Ala Ser Phe Phe Gly Thr Asp Ser Arg Val
   820 825 830
   Arg Ala His Arg Met Leu Val Arg Leu Gly Lys Cys His Ala Trp Tyr
   835 840 845
   Ser His Tyr Val Leu Lys Phe Phe Leu Leu Val Phe Gly Glu Asn Gly
   850 855 860
   Val Phe Phe Tyr Lys His Leu His Gly Asp Val Leu Pro Asn Asp Phe
   865 870 875 880
   Ala Ser Lys Leu Pro Leu Gln Glu Pro Phe Phe Pro Phe Glu Gly Lys
   885 890 895
   Ala Arg Val Tyr Arg Asn Glu Gly Arg Arg Leu Ala Cys Gly Asp Thr
   900 905 910
   Val Asp Gly Leu Pro Val Val Ala Arg Leu Gly Asp Leu Val Phe Ala
   915 920 925
   Gly Leu Ala Met Pro Pro Asp Gly Trp Ala Ile Thr Ala Pro Phe Thr
   930 935 940
   Leu Gln Cys Leu Ser Glu Arg Gly Thr Leu Ser Ala Met Ala Val Val
   945 950 955 960
   Met Thr Gly Ile Asp Pro Arg Thr Trp Thr Gly Thr Ile Phe Arg Leu
   965 970 975
   Gly Ser Leu Ala Thr Ser Tyr Met Gly Phe Val Cys Asp Asn Val Leu
   980 985 990
   Tyr Thr Ala His His Gly Ser Lys Gly Arg Arg Leu Ala His Pro Thr
   995 1000 1005
   Gly Ser Ile His Pro Ile Thr Val Asp Ala Ala Asn Asp Gln Asp Ile
   1010 1015 1020
   Tyr Gln Pro Pro Cys Gly Ala Gly Ser Leu Thr Arg Cys Ser Cys Gly
   1025 1030 1035 1040
   Glu Thr Lys Gly Tyr Leu Val Thr Arg Leu Gly Ser Leu Val Glu Val
   1045 1050 1055
   Asn Lys Ser Asp Asp Pro Tyr Trp Cys Val Cys Gly Ala Leu Pro Met
   1060 1065 1070
   Ala Val Ala Lys Gly Ser Ser Gly Ala Pro Ile Leu Cys Ser Ser Gly
   1075 1080 1085
   His Val Ile Gly Met Phe Thr Ala Ala Arg Asn Ser Gly Gly Ser Val
   1090 1095 1100
   Ser Gln Ile Arg Val Arg Pro Leu Val Cys Ala Gly Tyr His Pro Gln
   1105 1110 1115 1120
   Tyr Thr Ala His Ala Thr Leu Asp Thr Lys Pro Thr Val Pro Asn Glu
   1125 1130 1135
   Tyr Ser Val Gln Ile Leu Ile Ala Pro Thr Gly Ser Gly Lys Ser Thr
   1140 1145 1150
   Lys Leu Pro Leu Ser Tyr Met Gln Glu Lys Tyr Glu Val Leu Val Leu
   1155 1160 1165
   Asn Pro Ser Val Ala Thr Thr Ala Ser Met Pro Lys Tyr Met His Ala
   1170 1175 1180
   Thr Tyr Gly Val Asn Pro Asn Cys Tyr Phe Asn Gly Lys Cys Thr Asn
   1185 1190 1195 1200
   Thr Gly Ala Ser Leu Thr Tyr Ser Thr Tyr Gly Met Tyr Leu Thr Gly
   1205 1210 1215
   Ala Cys Ser Arg Asn Tyr Asp Val Ile Ile Cys Asp Glu Cys His Ala
   1220 1225 1230
   Thr Asp Ala Thr Thr Val Leu Gly Ile Gly Lys Val Leu Thr Glu Ala
   1235 1240 1245
   Pro Ser Lys Asn Val Arg Leu Val Val Leu Ala Thr Ala Thr Pro Pro
   1250 1255 1260
   Gly Val Ile Pro Thr Pro His Ala Asn Ile Thr Glu Ile Gln Leu Thr
   1265 1270 1275 1280
   Asp Glu Gly Thr Ile Pro Phe His Gly Lys Lys Ile Lys Glu Glu Asn
   1285 1290 1295
   Leu Lys Lys Gly Arg His Leu Ile Phe Glu Ala Thr Lys Lys His Cys
   1300 1305 1310
   Asp Glu Leu Ala Asn Glu Leu Ala Arg Lys Gly Ile Thr Ala Val Ser
   1315 1320 1325
   Tyr Tyr Arg Gly Cys Asp Ile Ser Lys Ile Pro Glu Gly Asp Cys Val
   1330 1335 1340
   Val Val Ala Thr Asp Ala Leu Cys Thr Gly Tyr Thr Gly Asp Phe Asp
   1345 1350 1355 1360
   Ser Val Tyr Asp Cys Ser Leu Met Val Glu Gly Thr Cys His Val Asp
   1365 1370 1375
   Leu Asp Pro Thr Phe Thr Met Gly Val Arg Val Cys Gly Val Ser Ala
   1380 1385 1390
   Ile Val Lys Gly Gln Arg Arg Gly Arg Thr Gly Arg Gly Arg Ala Gly
   1395 1400 1405
   Ile Tyr Tyr Tyr Val Asp Gly Ser Cys Thr Pro Ser Gly Met Val Pro
   1410 1415 1420
   Glu Cys Asn Ile Val Glu Ala Phe Asp Ala Ala Lys Ala Trp Tyr Gly
   1425 1430 1435 1440
   Leu Ser Ser Thr Glu Ala Gln Thr Ile Leu Asp Thr Tyr Arg Thr Gln
   1445 1450 1455
   Pro Gly Leu Pro Ala Ile Gly Ala Asn Leu Asp Glu Trp Ala Asp Leu
   1460 1465 1470
   Phe Ser Met Val Asn Pro Glu Pro Ser Phe Val Asn Thr Ala Lys Arg
   1475 1480 1485
   Thr Ala Asp Asn Tyr Val Leu Leu Thr Ala Ala Gln Leu Gln Leu Cys
   1490 1495 1500
   His Gln Tyr Gly Tyr Ala Ala Pro Asn Asp Ala Pro Arg Trp Gln Gly
   1505 1510 1515 1520
   Ala Arg Leu Gly Lys Lys Pro Cys Gly Val Leu Trp Arg Leu Asp Gly
   1525 1530 1535
   Ala Asp Ala Cys Pro Gly Pro Glu Pro Ser Glu Val Thr Arg Tyr Gln
   1540 1545 1550
   Met Cys Phe Thr Glu Val Asn Thr Ser Gly Thr Ala Ala Leu Ala Val
   1555 1560 1565
   Gly Val Gly Val Ala Met Ala Tyr Leu Ala Ile Asp Thr Phe Gly Ala
   1570 1575 1580
   Thr Cys Val Arg Arg Cys Trp Ser Ile Thr Ser Val Pro Thr Gly Ala
   1585 1590 1595 1600
   Thr Val Ala Pro Val Val Asp Glu Glu Glu Ile Val Glu Glu Cys Ala
   1605 1610 1615
   Ser Phe Ile Pro Leu Glu Ala Met Val Ala Ala Ile Asp Lys Leu Lys
   1620 1625 1630
   Ser Thr Ile Thr Thr Thr Ser Pro Phe Thr Leu Glu Thr Ala Leu Glu
   1635 1640 1645
   Lys Leu Asn Thr Phe Leu Gly Pro His Ala Ala Thr Ile Leu Ala Ile
   1650 1655 1660
   Ile Glu Tyr Cys Cys Gly Leu Val Thr Leu Pro Asp Asn Pro Phe Ala
   1665 1670 1675 1680
   Ser Cys Val Phe Ala Phe Ile Ala Gly Ile Thr Thr Pro Leu Pro His
   1685 1690 1695
   Lys Ile Lys Met Phe Leu Ser Leu Phe Gly Gly Ala Ile Ala Ser Lys
   1700 1705 1710
   Leu Thr Asp Ala Arg Gly Ala Leu Ala Phe Met Met Ala Gly Ala Ala
   1715 1720 1725
   Gly Thr Ala Leu Gly Thr Trp Thr Ser Val Gly Phe Val Phe Asp Met
   1730 1735 1740
   Leu Gly Gly Tyr Ala Ala Ala Ser Ser Thr Ala Cys Leu Thr Phe Lys
   1745 1750 1755 1760
   Cys Leu Met Gly Glu Trp Pro Thr Met Asp Gln Leu Ala Gly Leu Val
   1765 1770 1775
   Tyr Ser Ala Phe Asn Pro Ala Ala Gly Val Val Gly Val Leu Ser Ala
   1780 1785 1790
   Cys Ala Met Phe Ala Leu Thr Thr Ala Gly Pro Asp His Trp Pro Asn
   1795 1800 1805
   Arg Leu Leu Thr Met Leu Ala Arg Ser Asn Thr Val Cys Asn Glu Tyr
   1810 1815 1820
   Phe Ile Ala Thr Arg Asp Ile Arg Arg Lys Ile Leu Gly Ile Leu Glu
   1825 1830 1835 1840
   Ala Ser Thr Pro Trp Ser Val Ile Ser Ala Cys Ile Arg Trp Leu His
   1845 1850 1855
   Thr Pro Thr Glu Asp Asp Cys Gly Leu Ile Ala Trp Gly Leu Glu Ile
   1860 1865 1870
   Trp Gln Tyr Val Cys Asn Phe Phe Val Ile Cys Phe Asn Val Leu Lys
   1875 1880 1885
   Ala Gly Val Gln Ser Met Val Asn Ile Pro Gly Cys Pro Phe Tyr Ser
   1890 1895 1900
   Cys Gln Lys Gly Tyr Lys Gly Pro Trp Ile Gly Ser Gly Met Leu Gln
   1905 1910 1915 1920
   Ala Arg Cys Pro Cys Gly Ala Glu Leu Ile Phe Ser Val Glu Asn Gly
   1925 1930 1935
   Phe Ala Lys Leu Tyr Lys Gly Pro Arg Thr Cys Ser Asn Tyr Trp Arg
   1940 1945 1950
   Gly Ala Val Pro Val Asn Ala Arg Leu Cys Gly Ser Ala Arg Pro Asp
   1955 1960 1965
   Pro Thr Asp Trp Thr Ser Leu Val Val Asn Tyr Gly Val Arg Asp Tyr
   1970 1975 1980
   Cys Lys Tyr Glu Lys Met Gly Asp His Ile Phe Val Thr Ala Val Ser
   1985 1990 1995 2000
   Ser Pro Asn Val Cys Phe Thr Gln Val Pro Pro Thr Leu Arg Ala Ala
   2005 2010 2015
   Val Ala Val Asp Gly Val Gln Val Gln Cys Tyr Leu Gly Glu Pro Lys
   2020 2025 2030
   Thr Pro Trp Thr Thr Ser Ala Cys Cys Tyr Gly Pro Asp Gly Lys Gly
   2035 2040 2045
   Lys Thr Val Lys Leu Pro Phe Arg Val Asp Gly His Thr Pro Gly Val
   2050 2055 2060
   Arg Met Gln Leu Asn Leu Arg Asp Ala Leu Glu Thr Asn Asp Cys Asn
   2065 2070 2075 2080
   Ser Thr Asn Asn Thr Pro Ser Asp Glu Ala Ala Val Ser Ala Leu Val
   2085 2090 2095
   Phe Lys Gln Glu Leu Arg Arg Thr Asn Gln Leu Leu Glu Ala Ile Ser
   2100 2105 2110
   Ala Gly Val Asp Thr Thr Lys Leu Pro Ala Pro Ser Ile Glu Glu Val
   2115 2120 2125
   Val Val Arg Lys Arg Gln Phe Arg Ala Arg Thr Gly Ser Leu Thr Leu
   2130 2135 2140
   Pro Pro Pro Pro Arg Ser Val Pro Gly Val Ser Cys Pro Glu Ser Leu
   2145 2150 2155 2160
   Gln Arg Ser Asp Pro Leu Glu Gly Pro Ser Asn Leu Pro Pro Ser Pro
   2165 2170 2175
   Pro Val Leu Gln Leu Ala Met Pro Met Pro Leu Leu Gly Ala Gly Glu
   2180 2185 2190
   Cys Asn Pro Phe Thr Ala Ile Gly Cys Ala Met Thr Glu Thr Gly Gly
   2195 2200 2205
   Gly Pro Asp Asp Leu Pro Ser Tyr Pro Pro Lys Lys Glu Val Ser Glu
   2210 2215 2220
   Trp Ser Asp Glu Ser Trp Ser Thr Ala Thr Thr Ala Ser Ser Tyr Val
   2225 2230 2235 2240
   Thr Gly Pro Pro Tyr Pro Lys Ile Arg Gly Lys Asp Ser Thr Gln Ser
   2245 2250 2255
   Ala Pro Ala Lys Arg Pro Thr Lys Lys Lys Leu Gly Lys Ser Glu Phe
   2260 2265 2270
   Ser Cys Ser Met Ser Tyr Thr Trp Thr Asp Val Ile Ser Phe Lys Thr
   2275 2280 2285
   Ala Ser Lys Val Leu Ser Ala Thr Arg Ala Ile Thr Ser Gly Phe Leu
   2290 2295 2300
   Lys Gln Arg Ser Leu Val Tyr Val Thr Glu Pro Arg Asp Ala Glu Leu
   2305 2310 2315 2320
   Arg Lys Gln Lys Val Thr Ile Asn Arg Gln Pro Leu Phe Pro Pro Ser
   2325 2330 2335
   Tyr His Lys Gln Val Arg Leu Ala Lys Glu Lys Ala Ser Lys Val Val
   2340 2345 2350
   Gly Val Met Trp Asp Tyr Asp Glu Val Ala Ala His Thr Pro Ser Lys
   2355 2360 2365
   Ser Ala Lys Ser His Ile Thr Gly Leu Arg Gly Thr Asp Val Arg Ser
   2370 2375 2380
   Gly Ala Ala Arg Lys Ala Val Leu Asp Leu Gln Lys Cys Val Glu Ala
   2385 2390 2395 2400
   Gly Glu Ile Pro Ser His Tyr Arg Gln Thr Val Ile Val Pro Lys Glu
   2405 2410 2415
   Glu Val Phe Val Lys Thr Pro Gln Lys Pro Thr Lys Lys Pro Pro Arg
   2420 2425 2430
   Leu Ile Ser Tyr Pro His Leu Glu Met Arg Cys Val Glu Lys Met Tyr
   2435 2440 2445
   Tyr Gly Gln Val Ala Pro Asp Val Val Lys Ala Val Met Gly Asp Ala
   2450 2455 2460
   Tyr Gly Phe Val Asp Pro Arg Thr Arg Val Lys Arg Leu Leu Ser Met
   2465 2470 2475 2480
   Trp Ser Pro Asp Ala Val Gly Ala Thr Cys Asp Thr Val Cys Phe Asp
   2485 2490 2495
   Ser Thr Ile Thr Pro Glu Asp Ile Met Val Glu Thr Asp Ile Tyr Ser
   2500 2505 2510
   Ala Ala Lys Leu Ser Asp Gln His Arg Ala Gly Ile His Thr Ile Ala
   2515 2520 2525
   Arg Gln Leu Tyr Ala Gly Gly Pro Met Ile Ala Tyr Asp Gly Arg Glu
   2530 2535 2540
   Ile Gly Tyr Arg Arg Cys Arg Ser Ser Gly Val Tyr Thr Thr Ser Ser
   2545 2550 2555 2560
   Ser Asn Ser Leu Thr Cys Trp Leu Lys Val Asn Ala Ala Ala Glu Gln
   2565 2570 2575
   Ala Gly Met Lys Asn Pro Arg Phe Leu Ile Cys Gly Asp Asp Cys Thr
   2580 2585 2590
   Val Ile Trp Lys Ser Ala Gly Ala Asp Ala Asp Lys Gln Ala Met Arg
   2595 2600 2605
   Val Phe Ala Ser Trp Met Lys Val Met Gly Ala Pro Gln Asp Cys Val
   2610 2615 2620
   Pro Gln Pro Lys Tyr Ser Leu Glu Glu Leu Thr Ser Cys Ser Ser Asn
   2625 2630 2635 2640
   Val Thr Ser Gly Ile Thr Lys Ser Gly Lys Pro Tyr Tyr Phe Leu Thr
   2645 2650 2655
   Arg Asp Pro Arg Ile Pro Leu Gly Arg Cys Ser Ala Glu Gly Leu Gly
   2660 2665 2670
   Tyr Asn Pro Ser Ala Ala Trp Ile Gly Tyr Leu Ile His His Tyr Pro
   2675 2680 2685
   Cys Leu Trp Val Ser Arg Val Leu Ala Val His Phe Met Glu Gln Met
   2690 2695 2700
   Leu Phe Glu Asp Lys Leu Pro Glu Thr Val Thr Phe Asp Trp Tyr Gly
   2705 2710 2715 2720
   Lys Asn Tyr Thr Val Pro Val Glu Asp Leu Pro Ser Ile Ile Ala Gly
   2725 2730 2735
   Val His Gly Ile Glu Ala Phe Ser Val Val Arg Tyr Thr Asn Ala Glu
   2740 2745 2750
   Ile Leu Arg Val Ser Gln Ser Leu Thr Asp Met Thr Met Pro Pro Leu
   2755 2760 2765
   Arg Ala Trp Arg Lys Lys Ala Arg Ala Val Leu Ala Ser Ala Lys Arg
   2770 2775 2780
   Arg Gly Gly Ala His Ala Lys Leu Ala Arg Phe Leu Leu Trp His Ala
   2785 2790 2795 2800
   Thr Ser Arg Pro Leu Pro Asp Leu Asp Lys Thr Ser Val Ala Arg Tyr
   2805 2810 2815
   Thr Thr Phe Asn Tyr Cys Asp Val Tyr Ser Pro Glu Gly Asp Val Phe
   2820 2825 2830
   Ile Thr Pro Gln Arg Arg Leu Gln Lys Phe Leu Val Lys Tyr Leu Ala
   2835 2840 2845
   Val Ile Val Phe Ala Leu Gly Leu Ile Ala Val Gly Leu Ala Ile Ser
   2850 2855 2860
<210> 3
   <211> 9139
   <212> DNA
   <213> GBV-B virus
<400> 3
   accacaaaca ctccagtttg ttacactccg ctaggaatgc tcctggagca ccccccctag 60
   cagggcgtgg gggatttccc ctgcccgtct gcagaagggt ggagccaacc accttagtat 120
   gtaggcggcg ggactcatga cgctcgcgtg atgacaagcg ccaagcttga cttggatggc 180
   cctgatgggc gttcatgggt tcggtggtgg tggcgcttta ggcagcctcc acgcccacca 240
   cctcccagat agagcggcgg cactgtaggg aagaccgggg accggtcact accaaggacg 300
   cagacctctt tttgagtatc acgcctccgg aagtagttgg gcaagcccac ctatatgtgt 360
   tgggatggtt ggggttagcc atccataccg tactgcctga tagggtcctt gcgaggggat 420
   ctgggagtct cgtagaccgt agcacatgcc tgttatttct actcaaacaa gtcctgtacc 480
   tgcgcccaga acgcgcaaga acaagcagac gcaggcttca tatcctgtgt ccattaaaac 540
   atctgttgaa aggggacaac gagcaaagcg caaagtccag cgcgatgctc ggcctcgtaa 600
   ttacaaaatt gctggtatcc atgatggctt gcagacattg gctcaggctg ctttgccagc 660
   tcatggttgg ggacgccaag accctcgcca taagtctcgc aatcttggaa tccttctgga 720
   ttaccctttg gggtggattg gtgatgttac aactcacaca cctctagtag gcccgctggt 780
   ggcaggagcg gtcgttcgac cagtctgcca gatagtacgc ttgctggagg atggagtcaa 840
   ctgggctact ggttggttcg gtgtccacct ttttgtggta tgtctgctat ctttggcctg 900
   tccctgtagt ggggcgcggg tcactgaccc agacacaaat accacaatcc tgaccaattg 960
   ctgccagcgt aatcaggtta tctattgttc tccttccact tgcctacacg agcctggttg 1020
   tgtgatctgt gcggacgagt gctgggttcc cgccaatccg tacatctcac acccttccaa 1080
   ttggactggc acggactcct tcttggctga ccacattgat tttgttatgg gcgctcttgt 1140
   gacctgtgac gcccttgaca ttggtgagtt gtgtggtgcg tgtgtattag tcggtgactg 1200
   gcttgtcagg cactggctta ttcacataga cctcaatgaa actggtactt gttacctgga 1260
   agtgcccact ggaatagatc ctgggttcct agggtttatc gggtggatgg ccggcaaggt 1320
   cgaggctgtc atcttcttga ccaaactggc ttcacaagta ccatacgcta ttgcgactat 1380
   gtttagcagt gtacactacc tggcggttgg cgctctgatc tactatgcct ctcggggcaa 1440
   gtggtatcag ttgctcctag cgcttatgct ttacatagaa gcgacctctg gaaaccccat 1500
   cagggtgccc actggatgct caatagctga gttttgctcg cctttgatga taccatgtcc 1560
   ttgccactct tatttgagtg agaatgtgtc agaagtcatt tgttacagtc caaagtggac 1620
   caggcctatc actctagagt ataacaactc catatcttgg tacccctata caatccctgg 1680
   tgcgagggga tgtatggtta aattcaaaaa taacacatgg ggttgctgcc gtattcgcaa 1740
   tgtgccatcg tactgcacta tgggcactga tgcagtgtgg aacgacactc gcaacactta 1800
   cgaagcatgc ggtgtaacac catggctaac aaccgcatgg cacaacggct cagccctgaa 1860
   attggctata ttacaatacc ctgggtctaa agaaatgttt aaacctcata attggatgtc 1920
   aggccatttg tattttgagg gatcagatac ccctatagtt tacttttatg accctgtgaa 1980
   ttccactctc ctaccaccgg agaggtgggc taggttgccc ggtaccccac ctgtggtacg 2040
   tggttcttgg ttacaggttc cgcaagggtt ttacagtgat gtgaaagacc tagccacagg 2100
   attgatcacc aaagacaaag cctggaaaaa ttatcaggtc ttatattccg ccacgggtgc 2160
   tttgtctctt acgggagtta ccaccaaggc cgtggtgcta attctgttgg ggttgtgtgg 2220
   cagcaagtat cttattttag cctacctctg ttacttgtcc ctttgttttg ggcgcgcttc 2280
   tggttaccct ttgcgtcctg tgctcccatc ccagtcgtat ctccaagctg gctgggatgt 2340
   tttgtctaaa gctcaagtag ctccttttgc tttgattttc ttcatctgtt gctatctccg 2400
   ctgcaggcta cgttatgctg cccttttagg gtttgtgccc atggctgcgg gcttgcccct 2460
   aactttcttt gttgcagcag ctgctgccca accagattat gactggtggg tgcgactgct 2520
   agtggcaggg ttagttttgt gggccggccg taaccgtggt caccgcatag ctctgcttgt 2580
   aggtccttgg cctctggtag cgcttttaac cctcttgcat ttggttacgc ctgcttcagc 2640
   ttttgatacc gagataattg gagggctgac aataccacct gtagtagcat tagttgtcat 2700
   gtctcgtttt ggcttctttg ctcacttgtt acctcgctgt gctttagtta actcctatct 2760
   ttggcaacgt tgggagaatt ggttttggaa cgttacacta agaccggaga ggtttttcct 2820
   tgtgctggtt tgtttccccg gtgcgacata tgacgcgctg gtgactttct gtgtgtgtca 2880
   cgtagctctt ctatgtttaa catccagtgc agcatcgttc tttgggactg actctagggt 2940
   tagggcccat agaatgttgg tgcgtctcgg aaagtgtcat gcttggtatt ctcattatgt 3000
   tcttaagttt ttcctcttag tgtttggtga gaatggtgtg tttttctata agcacttgca 3060
   tggtgatgtc ttgcctaatg attttgcctc gaaactacca ttgcaagagc catttttccc 3120
   ttttgaaggc aaggcaaggg tctataggaa tgaaggaaga cgcttggcgt gtggggacac 3180
   ggttgatggt ttgcccgttg ttgcgcgtct cggcgacctt gttttcgcag ggttggctat 3240
   gccgccagat gggtgggcca ttaccgcacc ttttacgctg cagtgtctct ctgaacgtgg 3300
   cacgctgtca gcgatggcag tggtcatgac tggtatagac ccccgaactt ggactggaac 3360
   tatcttcaga ttaggatctc tggccactag ctacatggga tttgtttgtg acaacgtgtt 3420
   gtatactgct caccatggca gcaaggggcg ccggttggct catcccacag gctctataca 3480
   cccaataacc gttgacgcgg ctaatgacca ggacatctat caaccaccat gtggagctgg 3540
   gtcccttact cggtgctctt gcggggagac caaggggtat ctggtaacac gactggggtc 3600
   attggttgag gtcaacaaat ccgatgaccc ttattggtgt gtgtgcgggg cccttcccat 3660
   ggctgttgcc aagggttctt caggtgcccc gattctgtgc tcctccgggc atgttattgg 3720
   gatgttcacc gctgctagaa attctggcgg ttcagtcagt cagattaggg ttaggccgtt 3780
   ggtgtgtgct ggataccatc cccagtacac agcacatgcc actcttgata caaaacctac 3840
   tgtgcctaac gagtattcag tgcaaatttt aattgccccc actggcagcg gcaagtcaac 3900
   caaattacca ctttcttaca tgcaggagaa gtatgaggtc ttggtcctaa atcccagtgt 3960
   ggctacaaca gcatcaatgc caaagtacat gcacgcgacg tacggcgtga atccaaattg 4020
   ctattttaat ggcaaatgta ccaacacagg ggcttcactt acgtacagca catatggcat 4080
   gtacctgacc ggagcatgtt cccggaacta tgatgtaatc atttgtgacg aatgccatgc 4140
   taccgatgca accaccgtgt tgggcattgg aaaggtccta accgaagctc catccaaaaa 4200
   tgttaggcta gtggttcttg ccacggctac cccccctgga gtaatcccta caccacatgc 4260
   caacataact gagattcaat taaccgatga aggcactatc ccctttcatg gaaaaaagat 4320
   taaggaggaa aatctgaaga aagggagaca ccttatcttt gaggctacca aaaaacactg 4380
   tgatgagctt gctaacgagt tagctcgaaa gggaataaca gctgtctctt actatagggg 4440
   atgtgacatc tcaaaaatcc ctgagggcga ctgtgtagta gttgccactg atgccttgtg 4500
   tacagggtac actggtgact ttgattccgt gtatgactgc agcctcatgg tagaaggcac 4560
   atgccatgtt gaccttgacc ctactttcac catgggtgtt cgtgtgtgcg gggtttcagc 4620
   aatagttaaa ggccagcgta ggggccgcac aggccgtggg agagctggca tatactacta 4680
   tgtagacggg agttgtaccc cttcgggtat ggttcctgaa tgcaacattg ttgaagcctt 4740
   cgacgcagcc aaggcatggt atggtttgtc atcaacagaa gctcaaacta ttctggacac 4800
   ctatcgcacc caacctgggt tacctgcgat aggagcaaat ttggacgagt gggctgatct 4860
   cttttctatg gtcaaccccg aaccttcatt tgtcaatact gcaaaaagaa ctgctgacaa 4920
   ttatgttttg ttgactgcag cccaactaca actgtgtcat cagtatggct atgctgctcc 4980
   caatgacgca ccacggtggc agggagcccg gcttgggaaa aaaccttgtg gggttctgtg 5040
   gcgcttggac ggcgctgacg cctgtcctgg cccagagccc agcgaggtga ccagatacca 5100
   aatgtgcttc actgaagtca atacttctgg gacagccgca ctcgctgttg gcgttggagt 5160
   ggctatggct tatctagcca ttgacacttt tggcgccact tgtgtgcggc gttgctggtc 5220
   tattacatca gtccctaccg gtgctactgt cgccccagtg gttgacgaag aagaaatcgt 5280
   ggaggagtgt gcatcattca ttcccttgga ggccatggtt gctgcaattg acaagctgaa 5340
   gagtacaatc accacaacta gtcctttcac attggaaacc gcccttgaaa aacttaacac 5400
   ctttcttggg cctcatgcag ctacaatcct tgctatcata gagtattgct gtggtttagt 5460
   cactttacct gacaatccct ttgcatcatg cgtgtttgct ttcattgcgg gtattactac 5520
   cccactacct cacaagatca aaatgttcct gtcattattt ggaggcgcaa ttgcgtccaa 5580
   gcttacagac gctagaggcg cactggcgtt catgatggcc ggggctgcgg gaacagctct 5640
   tggtacatgg acatcggtgg gttttgtctt tgacatgcta ggcggctatg ctgccgcctc 5700
   atccactgct tgcttgacat ttaaatgctt gatgggtgag tggcccacta tggatcagct 5760
   tgctggttta gtctactccg cgttcaatcc ggccgcagga gttgtgggcg tcttgtcagc 5820
   ttgtgcaatg tttgctttga caacagcagg gccagatcac tggcccaaca gacttcttac 5880
   tatgcttgct aggagcaaca ctgtatgtaa tgagtacttt attgccactc gtgacatccg 5940
   caggaagata ctgggcattc tggaggcatc taccccctgg agtgtcatat cagcttgcat 6000
   ccgttggctc cacaccccga cggaggatga ttgcggcctc attgcttggg gtctagagat 6060
   ttggcagtat gtgtgcaatt tctttgtgat ttgctttaat gtccttaaag ctggagttca 6120
   gagcatggtt aacattcctg gttgtccttt ctacagctgc cagaaggggt acaagggccc 6180
   ctggattgga tcaggtatgc tccaagcacg ctgtccatgc ggtgctgaac tcatcttttc 6240
   tgttgagaat ggttttgcaa aactttacaa aggacccaga acttgttcaa attactggag 6300
   aggggctgtt ccagtcaacg ctaggctgtg tgggtcggct agaccggacc caactgattg 6360
   gactagtctt gtcgtcaatt atggcgttag ggactactgt aaatatgaga aaatgggaga 6420
   tcacattttt gttacagcag tatcctctcc aaatgtctgt ttcacccagg tgcccccaac 6480
   cttgagagct gcagtggccg tggacggcgt acaggttcag tgttatctag gtgagcccaa 6540
   aactccttgg acgacatctg cttgctgtta cggtcctgac ggtaagggta aaactgttaa 6600
   gcttcccttc cgcgttgacg gtcacacacc tggtgtgcgc atgcaactta atttgcgtga 6660
   tgcacttgag acaaatgact gtaattccac aaacaacact cctagtgatg aagccgcagt 6720
   gtccgctctt gttttcaaac aggagttgcg gcgtacaaac caattgcttg aggcaatttc 6780
   agctggcgtt gacaccacca aactgccagc cccctccatc gaagaggtag tggtaagaaa 6840
   gcgccagttc cgggcaagaa ctggttcgct taccttgcct ccccctccga gatccgtccc 6900
   aggagtgtca tgtcctgaaa gcctgcaacg aagtgacccg ttagaaggtc cttcaaacct 6960
   ccctccttca ccacctgttc tacagttggc catgccgatg cccctgttgg gagcgggtga 7020
   gtgtaaccct ttcactgcaa ttggatgtgc aatgaccgaa acaggcggag gccctgatga 7080
   tttacccagt taccctccca aaaaggaggt ctctgaatgg tcagacgaaa gttggtcgac 7140
   ggctacaacc gcttccagct acgttactgg ccccccgtac cctaagatac ggggaaagga 7200
   ttccactcag tcagcccccg ccaaacggcc tacaaaaaag aagttgggaa agagtgagtt 7260
   ttcgtgcagc atgagctaca cctggaccga cgtgattagc ttcaaaactg cttctaaagt 7320
   tctgtctgca actcgggcca tcactagtgg tttcctcaaa caaagatcat tggtgtatgt 7380
   gactgagccg cgggatgcgg agcttagaaa acaaaaagtc actattaata gacaacctct 7440
   gttcccccca tcataccaca agcaagtgag attggctaag gaaaaagctt caaaagttgt 7500
   cggtgtcatg tgggactatg atgaagtagc agctcacacg ccctctaagt ctgctaagtc 7560
   ccacatcact ggccttcggg gcactgatgt tcgttctgga gcagcccgca aggctgttct 7620
   ggacttgcag aagtgtgtcg aggcaggtga gataccgagt cattatcggc aaactgtgat 7680
   agttccaaag gaggaggtct tcgtgaagac cccccagaaa ccaacaaaga aacccccaag 7740
   gcttatctcg tacccccacc ttgaaatgag atgtgttgag aagatgtact acggtcaggt 7800
   tgctcctgac gtagttaaag ctgtcatggg agatgcgtac gggtttgtag atccacgtac 7860
   ccgtgtcaag cgtctgttgt cgatgtggtc acccgatgca gtcggagcca catgcgatac 7920
   agtgtgtttt gacagtacca tcacacccga ggatatcatg gtggagacag acatctactc 7980
   agcagctaaa ctcagtgacc aacaccgagc tggcattcac accattgcga ggcagttata 8040
   cgctggagga ccgatgatcg cttatgatgg ccgagagatc ggatatcgta ggtgtaggtc 8100
   ttccggcgtc tatactacct caagttccaa cagtttgacc tgctggctga aggtaaatgc 8160
   tgcagccgaa caggctggca tgaagaaccc tcgcttcctt atttgcggcg atgattgcac 8220
   cgtaatttgg aagagcgccg gagcagatgc agacaaacaa gcaatgcgtg tctttgctag 8280
   ctggatgaag gtgatgggtg caccacaaga ttgtgtgcct caacccaaat acagtttgga 8340
   agaattaaca tcatgctcat caaatgttac ctctggaatt accaaaagtg gcaagcctta 8400
   ctactttctt acaagagatc ctcgtatccc ccttggcagg tgctctgccg agggtctggg 8460
   atacaacccc agtgctgcgt ggattgggta tctaatacat cactacccat gtttgtgggt 8520
   tagccgtgtg ttggctgtcc atttcatgga gcagatgctc tttgaggaca aacttcccga 8580
   gactgtgacc tttgactggt atgggaaaaa ttatacggtg cctgtagaag atctgcccag 8640
   catcattgct ggtgtgcacg gtattgaggc tttctcggtg gtgcgctaca ccaacgctga 8700
   gatcctcaga gtttcccaat cactaacaga catgaccatg ccccccctgc gagcctggcg 8760
   aaagaaagcc agggcggtcc tcgccagcgc caagaggcgt ggcggagcac acgcaaaatt 8820
   ggctcgcttc cttctctggc atgctacatc tagacctcta ccagatttgg ataagacgag 8880
   cgtggctcgg tacaccactt tcaattattg tgatgtttac tccccggagg gggatgtgtt 8940
   tattacacca cagagaagat tgcagaagtt ccttgtgaag tatttggctg tcattgtttt 9000
   tgccctaggg ctcattgctg ttggattagc catcagctga acccccaaat tcaaaattaa 9060
   ctaacagttt tttttttttt tttttttttt agggcagcgg caacagggga gaccccgggc 9120
   ttaacgaccc cgcgatgtg                                              9139
<210> 4
   <211> 9711
   <212> DNA
   <213> Hepatitis C virus
<400> 4
   acccgcccct aataggggcg acactccgcc atgaatcact cccctgtgag gaactactgt 60
   cttcacgcag aaagcgtcta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gccatttggg cgtgcccccg 240
   caagactgct agccgagtag cgttgggttg cgaaaggcct tgtggtactg cctgataggg 300
   tgcttgcgag tgccccggga ggtctcgtag accgtgcacc atgagcacaa atcctaaacc 360
   tcaaagaaaa accaaaagaa acaccaaccg tcgcccacaa gacgttaagt ttccgggcgg 420
   cggccagatc gttggcggag tatacttgtt gccgcgcagg ggccccaggt tgggtgtgcg 480
   cgcgacaagg aagacttcgg agcggtccca gccacgtgga aggcgccagc ccatccctaa 540
   agatcggcgc tccactggca aatcctgggg aaaaccagga tacccctggc ccctatacgg 600
   gaatgaggga ctcggctggg caggatggct cctgtccccc cgaggttccc gtccctcttg 660
   gggccccaat gacccccggc ataggtcgcg caacgtgggt aaggtcatcg ataccctaac 720
   gtgcggcttt gccgacctca tggggtacat ccctgtcgtg ggcgccccgc tcggcggcgt 780
   cgccagagct ctcgcgcatg gcgtgagagt cctggaggac ggggttaatt ttgcaacagg 840
   gaacttaccc ggttgctcct tttctatctt cttgctggcc ctgctgtcct gcatcaccac 900
   cccggtctcc gctgccgaag tgaagaacat cagtaccggc tacatggtga ctaacgactg 960
   caccaatgac agcattacct ggcagctcca ggctgctgtc ctccacgtcc ccgggtgcgt 1020
   cccgtgcgag aaagtgggga atgcatctca gtgctggata ccggtctcac cgaatgtggc 1080
   cgtgcagcgg cccggcgccc tcacgcaggg cttgcggacg cacatcgaca tggttgtgat 1140
   gtccgccacg ctctgctctg ccctctacgt gggggacctc tgcggtgggg tgatgctcgc 1200
   agcccaaatg ttcattgtct cgccgcagca ccactggttt gtccaagact gcaattgctc 1260
   catctaccct ggtaccatca ctggacaccg catggcatgg gacatgatga tgaactggtc 1320
   gcccacggct accatgatct tggcgtacgc gatgcgtgtc cccgaggtca ttatagacat 1380
   cattagcggg gctcattggg gcgtcatgtt cggcttggcc tacttctcta tgcagggagc 1440
   gtgggcgaaa gtcgttgtca tccttctgtt ggccgccggg gtggacgcgc gcacccatac 1500
   tgttgggggt tctgccgcgc agaccaccgg gcgcctcacc agcttatttg acatgggccc 1560
   caggcagaaa atccagctcg ttaacaccaa tggcagctgg cacatcaacc gcaccgccct 1620
   gaactgcaat gactccttgc acaccggctt tatcgcgtct ctgttctaca cccacagctt 1680
   caactcgtca ggatgtcccg aacgcatgtc cgcctgccgc agtatcgagg ccttccgggt 1740
   gggatggggc gccttgcaat atgaggataa tgtcaccaat ccagaggata tgagacccta 1800
   ttgctggcac tacccaccaa ggcagtgtgg cgtggtctcc gcgaagactg tgtgtggccc 1860
   agtgtactgt ttcaccccca gcccagtggt agtgggcacg accgacaggc ttggagcgcc 1920
   cacttacacg tggggggaga atgagacaga tgtcttccta ttgaacagca ctcgaccacc 1980
   gctggggtca tggttcggct gcacgtggat gaactcttct ggctacacca agacttgcgg 2040
   cgcaccaccc tgccgtacta gagctgactt caacgccagc acggacctgt tgtgccccac 2100
   ggactgtttt aggaagcatc ctgataccac ttacctcaaa tgcggctctg ggccctggct 2160
   cacgccaagg tgcctgatcg actaccccta caggctctgg cattacccct gcacagttaa 2220
   ctataccatc ttcaaaataa ggatgtatgt gggaggggtt gagcacaggc tcacggctgc 2280
   atgcaatttc actcgtgggg atcgttgcaa cttggaggac agagacagaa gtcaactgtc 2340
   tcctttgttg cactccacca cggaatgggc cattttacct tgctcttact cggacctgcc 2400
   cgccttgtcg actggtcttc tccacctcca ccaaaacatc gtggacgtac aattcatgta 2460
   tggcctatca cctgccctca caaaatacat cgtccgatgg gagtgggtaa tactcttatt 2520
   cctgctctta gcggacgcca gggtttgcgc ctgcttatgg atgctcatct tgttgggcca 2580
   ggccgaagca gcactagaga agctggtcat cttgcacgct gcgagcgcag ctagctgcaa 2640
   tggcttccta tattttgtca tctttttcgt ggctgcttgg tacatcaagg gtcgggtagt 2700
   ccccttagct acctattccc tcactggcct gtggtccttt agcctactgc tcctagcatt 2760
   gccccaacag gcttatgctt atgacgcatc tgtgcatggc cagataggag cggctctgct 2820
   ggtaatgatc actctcttta ctctcacccc cgggtataag acccttctca gccggttttt 2880
   gtggtggttg tgctatcttc tgaccctggg ggaagctatg gtccaggagt gggcaccacc 2940
   tatgcaggtg cgcggtggcc gtgatggcat catatgggcc gtcgccatat tctacccagg 3000
   tgtggtgttt gacataacca agtggctctt ggcggtgctt gggcctgctt acctcctaaa 3060
   aggtgctttg acgcgcgtgc cgtacttcgt cagggctcac gctctactga ggatgtgcac 3120
   catggcaagg catctcgcgg ggggcaggta cgtccagatg gcgctactag cccttggcag 3180
   gtggactggc acttacatct atgaccacct cacccctatg tcggattggg ctgctagtgg 3240
   cctgcgggac ctggcggtcg ccgttgagcc tatcatcttc agtccgatgg agaagaaagt 3300
   cattgtctgg ggagcggaga cagctgcttg tggggacatt ttacacggac ttcccgtgtc 3360
   cgcccgactt ggtcgggagg tcctccttgg cccagctgat ggctatacct ccaaggggtg 3420
   gagtcttctc gcccccatca ctgcttacgc ccagcagaca cgtggccttt tgggcaccat 3480
   agtggtgagc atgacggggc gcgacaagac agaacaggct ggggaaattc aggtcctgtc 3540
   cacagtcact cagtccttcc tcggaacatc catctcgggg gttttgtgga ctgtctacca 3600
   tggagctggc aacaagactc tggccggctc acggggtccg gtcacgcaga tgtactccag 3660
   tgctgagggg gacttagtag ggtggcccag cccccctggg actaaatctt tggagccgtg 3720
   cacgtgtgga gcggtcgacc tgtacctggt cacgcggaac gctgatgtca tcccggctcg 3780
   aagacgcggg gacaaacggg gagcgctact ctccccgaga cctctttcca ccttgaaggg 3840
   gtcctcagga ggcccggtgc tatgccccag gggccacgct gtcggagtct tccgggcagc 3900
   tgtgtgctct cggggcgtgg ctaagtccat agatttcatc cccgttgaga cactcgacat 3960
   cgtcacgcgg tcccccacct ttagtgacaa cagcacacca cctgctgtgc cccagaccta 4020
   tcaggtcggg tacttgcatg ccccgactgg cagtggaaag agcaccaaag ttcctgtcgc 4080
   atatgctgct caggggtata aagtgctagt gcttaatccc tcagtggctg ccaccctggg 4140
   gtttggggcg tacttgtcta aggcacatgg catcaatccc aacattagga ctggagtcag 4200
   gactgtgacg accggggcgc ccatcacgta ctccacatat ggcaaattcc tcgccgatgg 4260
   gggctgtgcg ggcggcgcct acgacatcat catatgtgat gaatgccatg ccgtggactc 4320
   taccaccatc cttggcatcg gaacagtcct tgatcaagca gagacagctg gggtcagact 4380
   aactgtgctg gctacagcta cgccccctgg gtcagtgaca accccccacc ccaacataga 4440
   ggaggtggcc cttgggcagg agggcgagat ccccttctat gggagggcga ttcccctgtc 4500
   ttacatcaag ggaggaagac atctgatctt ctgccattca aagaaaaagt gtgacgagct 4560
   cgcggcggcc cttcggggta tgggcttgaa ctcagtggca tactacagag ggttggacgt 4620
   ctccgtaata ccaactcagg gagacgtagt ggtcgtcgcc accgacgccc tcatgacagg 4680
   gtatactggg gactttgact ccgtgatcga ctgcaacgta gcggtcactc aagttgtaga 4740
   cttcagttta gaccccacat tcaccataac cacacagatt gtccctcaag acgctgtctc 4800
   acgtagccag cgccggggtc gcacgggtag gggaagactg ggcatttata ggtatgtttc 4860
   cactggtgag cgagcctcag gaatgtttga cagtgtagtg ctctgtgagt gctacgacgc 4920
   aggggccgca tggtatgagc tcacaccatc ggagaccacc gtcaggctca gggcgtattt 4980
   caacacgccc ggtttgcctg tgtgccaaga ccatcttgag ttttgggagg cagttttcac 5040
   cggcctcaca cacatagatg cccacttcct ttcccaaaca aagcaatcgg gggaaaattt 5100
   cgcatactta acagcctacc aggctacagt gtgcgctagg gccaaagccc cccccccgtc 5160
   ctgggacgtc atgtggaagt gtttgactcg actcaagccc acactcgtgg gccccacacc 5220
   tctcctgtac cgcttgggct ctgttaccaa cgaggtcacc ctcacacatc ccgtgacgaa 5280
   atacatcgcc acctgcatgc aagccgacct tgaggtcatg accagcacat gggtcttggc 5340
   agggggagtc ttggcggccg tcgccgcgta ttgcctggcg accgggtgtg tttgcatcat 5400
   cggccgcttg cacattaacc agcgagccgt cgttgcgccg gacaaggagg tcctctatga 5460
   ggcttttgat gagatggagg aatgtgcctc tagggcggct ctcattgaag aggggcagcg 5520
   gatagccgag atgctgaagt ccaagatcca aggcttattg cagcaagctt ccaaacaagc 5580
   tcaagacata caacccactg tgcaggcttc atggcccaag gtagaacaat tctgggccaa 5640
   acacatgtgg aacttcatta gcggcatcca atacctcgca ggactatcaa cactgccagg 5700
   gaaccctgca gtagcttcca tgatggcgtt cagtgccgcc ctcaccagtc cgctgtcaac 5760
   aagcaccact atccttctca acattttggg gggctggcta gcatcccaaa ttgcaccacc 5820
   cgcgggggcc actggcttcg ttgtcagtgg cctagtggga gctgccgtag gcagtatagg 5880
   cttaggtaag gtgctagtgg acatcctggc agggtatggt gcgggcattt cgggggctct 5940
   cgtcgcattc aagatcatgt ctggcgagaa gccctccatg gaggatgtcg tcaacttgct 6000
   gcctggaatt ctgtctccgg gtgccttggt agtgggagtc atctgcgcgg ccattctgcg 6060
   ccgacacgtg ggaccggggg aaggcgccgt ccaatggatg aatagactca ttgcctttgc 6120
   ttccagagga aatcacgtcg cccccaccca ctacgtgacg gagtcggatg cgtcgcagcg 6180
   tgtgacccaa ctacttggct cccttaccat aaccagcctg ctcagaagac tccacaactg 6240
   gattactgag gactgcccca tcccatgcgg cggctcgtgg ctccgcgatg tgtgggactg 6300
   ggtttgcacc atcctaacag actttaaaaa ttggctgacc tccaaattat tcccaaagat 6360
   gcccggcctc ccctttgtct cctgtcaaaa ggggtacaag ggcgtgtggg ccggcactgg 6420
   catcatgacc acacggtgtc cttgcggcgc caatatctct ggcaatgtcc gcttgggctc 6480
   catgagaatc acggggccta agacctgcat gaatatctgg caggggacct ttcctatcaa 6540
   ttgttacacg gagggccagt gcgtgccgaa acccgcgcca aactttaagg tcgccatctg 6600
   gagggtggcg gcctcagagt acgcggaggt gacgcagcac gggtcatacc actacataac 6660
   aggactcacc actgataact tgaaagtccc ctgccaacta ccctctcccg agttcttttc 6720
   ctgggtggac ggagtgcaga tccataggtt tgcccccaca ccgaagccgt ttttccggga 6780
   tgaggtctcg ttctgcgttg ggcttaattc atttgtcgtc gggtcccagc ttccttgcga 6840
   ccctgaaccc gacacagacg tattgatgtc catgctaaca gatccatctc atatcacggc 6900
   ggagactgca gcgcggcgtt tagcgcgggg gtcaccccca tccgaggcaa gctcctcggc 6960
   gagccagcta tcggcaccat cgctgcgagc cacctgcacc acccacggca aagcctatga 7020
   tgtggacatg gtggatgcta acctgttcat ggggggcgat gtgactcgga tagagtctgg 7080
   gtccaaagtg gtcgttctgg actctctcga cccaatggtc gaagaaagga gcgaccttga 7140
   gccttcgata ccatcagaat acatgctccc caagaagagg ttcccaccag ctttaccggc 7200
   ctgggcacgg cctgattaca acccaccgct tgtggaatcg tggaaaaggc cagattacca 7260
   accggccact gttgcgggct gtgctctccc tcctcctagg aaaaccccga cgcctccccc 7320
   aaggaggcgc cggacagtgg gcctaagtga ggactccata ggagatgccc ttcaacagct 7380
   ggccattaag tcctttggcc agcccccccc aagcggcgat tcaggccttt ccacgggggc 7440
   gggcgctgcc gattccggca gtcagacgcc tcctgatgag ttggcccttt cggagacagg 7500
   ttccatctct tccatgcccc ccctcgaggg ggagcttgga gatccagacc tggagcctga 7560
   gcaggtagag ccccaacccc ccccccaggg gggggtggca gctcccggct cggactcggg 7620
   gtcctggtct acttgctccg aggaggacga ctccgtcgtg tgctgctcca tgtcatactc 7680
   ctggaccggg gctctaataa ctccttgtag tcccgaagag gagaagttac cgattaaccc 7740
   cttgagcaac tccctgttgc gatatcacaa caaggtgtac tgtaccacaa caaagagcgc 7800
   ctcactaagg gctaaaaagg taacttttga taggatgcaa gtgctcgact cctactacga 7860
   ctcagtctta aaggacatta agctagcggc ctccaaggtc accgcaaggc tcctcaccat 7920
   ggaggaggct tgccagttaa ccccacccca ttctgcaaga tctaaatatg ggtttggggc 7980
   taaggaggtc cgcagcttgt ccgggagggc cgttaaccac atcaagtccg tgtggaagga 8040
   cctcctggag gactcagaaa caccaattcc cacaaccatt atggccaaaa atgaggtgtt 8100
   ctgcgtggac cccaccaagg ggggcaagaa agcagctcgc cttatcgttt accctgacct 8160
   cggcgtcagg gtctgcgaga agatggccct ttatgacatt acacaaaaac ttcctcaggc 8220
   ggtgatgggg gcttcttatg gattccagta ttcccccgct cagcgggtag agtttctctt 8280
   gaaagcatgg gcggaaaaga aggaccctat gggtttttcg tatgataccc gatgctttga 8340
   ctcaaccgtc actgagagag acatcaggac tgaggagtcc atatatcggg cctgctcctt 8400
   gcccgaggag gcccacactg ccatacactc gctaactgag agactttacg tgggagggcc 8460
   tatgttcaac agcaagggcc aaacctgcgg gtacaggcgt tgccgcgcca gcggggtgct 8520
   caccactagc atggggaaca ccatcacatg ctacgtgaaa gccttagcgg cttgtaaagc 8580
   tgcagggata atcgcgccca caatgctggt atgcggcgat gacttggttg tcatctcaga 8640
   aagccagggg accgaggagg acgagcggaa cctgagagcc ttcacggagg ctatgaccag 8700
   gtattctgcc cctcctggtg acccccccag accggagtat gatctggagc tgataacatc 8760
   ttgctcctca aatgtgtctg tggcgctggg cccacaaggc cgccgcagat actacctgac 8820
   cagagaccct accactccaa tcgcccgggc tgcctgggaa acagttagac actcccctgt 8880
   caattcatgg ctgggaaaca tcatccagta cgccccgacc atatgggctc gcatggtcct 8940
   gatgacacac ttcttctcca ttctcatggc tcaagacacg ctggaccaga acctcaactt 9000
   tgagatgtac ggagcggtgt actccgtgag tcccttggac ctcccagcta taattgaaag 9060
   gttacatggg cttgacgctt tttctctgca cacatacact ccccacgaac tgacacgggt 9120
   ggcttcagcc ctcagaaaac ttggggcgcc acccctcaga gcgtggaaga gccgggcacg 9180
   tgcagtcagg gcgtccctca tctcccgtgg ggggagagcg gccgtttgcg gtcgatatct 9240
   cttcaattgg gcggtgaaga ccaagctcaa actcactcca ttgccggaag cgcgcctcct 9300
   ggatttatcc agctggttca ccgtcggcgc cggcgggggc gacatttatc acagcgtgtc 9360
   gcgtgcccga ccccgcttat tgctctttgg cctactccta ctttttgtag gggtaggcct 9420
   tttcctactc cccgctcggt agagcggcac acattagcta cactccatag ctaactgtcc 9480
   cttttttttt tttttttttt tttttttttt tttttttttt tttttttttt tttttttttt 9540
   tttttttttt tttttttttt tttttctttt tttctctttt ccttctttct taccttattt 9600
   tactttcttt cctggtggct ccatcttagc cctagtcacg gctagctgtg aaaggtccgt 9660
   gagccgcatg actgcagaga gtgccgtaac tggtctctct gcagatcatg t          9711
<210> 5
   <211> 3033
   <212> PRT
   <213> Hepatitis C virus
<400> 5
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Asp Arg Arg Ser Thr Gly Lys Ser Trp Gly Lys Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Asn Asp Pro
   100 105 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Gly Gly Val Ala Arg Ala Leu Ala His Gly Val Arg Val Leu Glu Asp
   145 150 155 160
   Gly Val Asn Phe Ala Thr Gly Asn Leu Pro Gly Cys Ser Phe Ser Ile
   165 170 175
   Phe Leu Leu Ala Leu Leu Ser Cys Ile Thr Thr Pro Val Ser Ala Ala
   180 185 190
   Glu Val Lys Asn Ile Ser Thr Gly Tyr Met Val Thr Asn Asp Cys Thr
   195 200 205
   Asn Asp Ser Ile Thr Trp Gln Leu Gln Ala Ala Val Leu His Val Pro
   210 215 220
   Gly Cys Val Pro Cys Glu Lys Val Gly Asn Ala Ser Gln Cys Trp Ile
   225 230 235 240
   Pro Val Ser Pro Asn Val Ala Val Gln Arg Pro Gly Ala Leu Thr Gln
   245 250 255
   Gly Leu Arg Thr His Ile Asp Met Val Val Met Ser Ala Thr Leu Cys
   260 265 270
   Ser Ala Leu Tyr Val Gly Asp Leu Cys Gly Gly Val Met Leu Ala Ala
   275 280 285
   Gln Met Phe Ile Val Ser Pro Gln His His Trp Phe Val Gln Asp Cys
   290 295 300
   Asn Cys Ser Ile Tyr Pro Gly Thr Ile Thr Gly His Arg Met Ala Trp
   305 310 315 320
   Asp Met Met Met Asn Trp Ser Pro Thr Ala Thr Met Ile Leu Ala Tyr
   325 330 335
   Ala Met Arg Val Pro Glu Val Ile Ile Asp Ile Ile Ser Gly Ala His
   340 345 350
   Trp Gly Val Met Phe Gly Leu Ala Tyr Phe Ser Met Gln Gly Ala Trp
   355 360 365
   Ala Lys Val Val Val Ile Leu Leu Leu Ala Ala Gly Val Asp Ala Arg
   370 375 380
   Thr His Thr Val Gly Gly Ser Ala Ala Gln Thr Thr Gly Arg Leu Thr
   385 390 395 400
   Ser Leu Phe Asp Met Gly Pro Arg Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu His Thr Gly Phe Ile Ala Ser Leu Phe Tyr Thr His Ser Phe Asn
   435 440 445
   Ser Ser Gly Cys Pro Glu Arg Met Ser Ala Cys Arg Ser Ile Glu Ala
   450 455 460
   Phe Arg Val Gly Trp Gly Ala Leu Gln Tyr Glu Asp Asn Val Thr Asn
   465 470 475 480
   Pro Glu Asp Met Arg Pro Tyr Cys Trp His Tyr Pro Pro Arg Gln Cys
   485 490 495
   Gly Val Val Ser Ala Lys Thr Val Cys Gly Pro Val Tyr Cys Phe Thr
   500 505 510
   Pro Ser Pro Val Val Val Gly Thr Thr Asp Arg Leu Gly Ala Pro Thr
   515 520 525
   Tyr Thr Trp Gly Glu Asn Glu Thr Asp Val Phe Leu Leu Asn Ser Thr
   530 535 540
   Arg Pro Pro Leu Gly Ser Trp Phe Gly Cys Thr Trp Met Asn Ser Ser
   545 550 555 560
   Gly Tyr Thr Lys Thr Cys Gly Ala Pro Pro Cys Arg Thr Arg Ala Asp
   565 570 575
   Phe Asn Ala Ser Thr Asp Leu Leu Cys Pro Thr Asp Cys Phe Arg Lys
   580 585 590
   His Pro Asp Thr Thr Tyr Leu Lys Cys Gly Ser Gly Pro Trp Leu Thr
   595 600 605
   Pro Arg Cys Leu Ile Asp Tyr Pro Tyr Arg Leu Trp His Tyr Pro Cys
   610 615 620
   Thr Val Asn Tyr Thr Ile Phe Lys Ile Arg Met Tyr Val Gly Gly Val
   625 630 635 640
   Glu His Arg Leu Thr Ala Ala Cys Asn Phe Thr Arg Gly Asp Arg Cys
   645 650 655
   Asn Leu Glu Asp Arg Asp Arg Ser Gln Leu Ser Pro Leu Leu His Ser
   660 665 670
   Thr Thr Glu Trp Ala Ile Leu Pro Cys Ser Tyr Ser Asp Leu Pro Ala
   675 680 685
   Leu Ser Thr Gly Leu Leu His Leu His Gln Asn Ile Val Asp Val Gln
   690 695 700
   Phe Met Tyr Gly Leu Ser Pro Ala Leu Thr Lys Tyr Ile Val Arg Trp
   705 710 715 720
   Glu Trp Val Ile Leu Leu Phe Leu Leu Leu Ala Asp Ala Arg Val Cys
   725 730 735
   Ala Cys Leu Trp Met Leu Ile Leu Leu Gly Gln Ala Glu Ala Ala Leu
   740 745 750
   Glu Lys Leu Val Ile Leu His Ala Ala Ser Ala Ala Ser Cys Asn Gly
   755 760 765
   Phe Leu Tyr Phe Val Ile Phe Phe Val Ala Ala Trp Tyr Ile Lys Gly
   770 775 780
   Arg Val Val Pro Leu Ala Thr Tyr Ser Leu Thr Gly Leu Trp Ser Phe
   785 790 795 800
   Ser Leu Leu Leu Leu Ala Leu Pro Gln Gln Ala Tyr Ala Tyr Asp Ala
   805 810 815
   Ser Val His Gly Gln Ile Gly Ala Ala Leu Leu Val Met Ile Thr Leu
   820 825 830
   Phe Thr Leu Thr Pro Gly Tyr Lys Thr Leu Leu Ser Arg Phe Leu Trp
   835 840 845
   Trp Leu Cys Tyr Leu Leu Thr Leu Gly Glu Ala Met Val Gln Glu Trp
   850 855 860
   Ala Pro Pro Met Gln Val Arg Gly Gly Arg Asp Gly Ile Ile Trp Ala
   865 870 875 880
   Val Ala Ile Phe Tyr Pro Gly Val Val Phe Asp Ile Thr Lys Trp Leu
   885 890 895
   Leu Ala Val Leu Gly Pro Ala Tyr Leu Leu Lys Gly Ala Leu Thr Arg
   900 905 910
   Val Pro Tyr Phe Val Arg Ala His Ala Leu Leu Arg Met Cys Thr Met
   915 920 925
   Ala Arg His Leu Ala Gly Gly Arg Tyr Val Gln Met Ala Leu Leu Ala
   930 935 940
   Leu Gly Arg Trp Thr Gly Thr Tyr Ile Tyr Asp His Leu Thr Pro Met
   945 950 955 960
   Ser Asp Trp Ala Ala Ser Gly Leu Arg Asp Leu Ala Val Ala Val Glu
   965 970 975
   Pro Ile Ile Phe Ser Pro Met Glu Lys Lys Val Ile Val Trp Gly Ala
   980 985 990
   Glu Thr Ala Ala Cys Gly Asp Ile Leu His Gly Leu Pro Val Ser Ala
   995 1000 1005
   Arg Leu Gly Arg Glu Val Leu Leu Gly Pro Ala Asp Gly Tyr Thr Ser
   1010 1015 1020
   Lys Gly Trp Ser Leu Leu Ala Pro Ile Thr Ala Tyr Ala Gln Gln Thr
   1025 1030 1035 1040
   Arg Gly Leu Leu Gly Thr Ile Val Val Ser Met Thr Gly Arg Asp Lys
   1045 1050 1055
   Thr Glu Gln Ala Gly Glu Ile Gln Val Leu Ser Thr Val Thr Gln Ser
   1060 1065 1070
   Phe Leu Gly Thr Ser Ile Ser Gly Val Leu Trp Thr Val Tyr His Gly
   1075 1080 1085
   Ala Gly Asn Lys Thr Leu Ala Gly Ser Arg Gly Pro Val Thr Gln Met
   1090 1095 1100
   Tyr Ser Ser Ala Glu Gly Asp Leu Val Gly Trp Pro Ser Pro Pro Gly
   1105 1110 1115 1120
   Thr Lys Ser Leu Glu Pro Cys Thr Cys Gly Ala Val Asp Leu Tyr Leu
   1125 1130 1135
   Val Thr Arg Asn Ala Asp Val Ile Pro Ala Arg Arg Arg Gly Asp Lys
   1140 1145 1150
   Arg Gly Ala Leu Leu Ser Pro Arg Pro Leu Ser Thr Leu Lys Gly Ser
   1155 1160 1165
   Ser Gly Gly Pro Val Leu Cys Pro Arg Gly His Ala Val Gly Val Phe
   1170 1175 1180
   Arg Ala Ala Val Cys Ser Arg Gly Val Ala Lys Ser Ile Asp Phe Ile
   1185 1190 1195 1200
   Pro Val Glu Thr Leu Asp Ile Val Thr Arg Ser Pro Thr Phe Ser Asp
   1205 1210 1215
   Asn Ser Thr Pro Pro Ala Val Pro Gln Thr Tyr Gln Val Gly Tyr Leu
   1220 1225 1230
   His Ala Pro Thr Gly Ser Gly Lys Ser Thr Lys Val Pro Val Ala Tyr
   1235 1240 1245
   Ala Ala Gln Gly Tyr Lys Val Leu Val Leu Asn Pro Ser Val Ala Ala
   1250 1255 1260
   Thr Leu Gly Phe Gly Ala Tyr Leu Ser Lys Ala His Gly Ile Asn Pro
   1265 1270 1275 1280
   Asn Ile Arg Thr Gly Val Arg Thr Val Thr Thr Gly Ala Pro Ile Thr
   1285 1290 1295
   Tyr Ser Thr Tyr Gly Lys Phe Leu Ala Asp Gly Gly Cys Ala Gly Gly
   1300 1305 1310
   Ala Tyr Asp Ile Ile Ile Cys Asp Glu Cys His Ala Val Asp Ser Thr
   1315 1320 1325
   Thr Ile Leu Gly Ile Gly Thr Val Leu Asp Gln Ala Glu Thr Ala Gly
   1330 1335 1340
   Val Arg Leu Thr Val Leu Ala Thr Ala Thr Pro Pro Gly Ser Val Thr
   1345 1350 1355 1360
   Thr Pro His Pro Asn Ile Glu Glu Val Ala Leu Gly Gln Glu Gly Glu
   1365 1370 1375
   Ile Pro Phe Tyr Gly Arg Ala Ile Pro Leu Ser Tyr Ile Lys Gly Gly
   1380 1385 1390
   Arg His Leu Ile Phe Cys His Ser Lys Lys Lys Cys Asp Glu Leu Ala
   1395 1400 1405
   Ala Ala Leu Arg Gly Met Gly Leu Asn Ser Val Ala Tyr Tyr Arg Gly
   1410 1415 1420
   Leu Asp Val Ser Val Ile Pro Thr Gln Gly Asp Val Val Val Val Ala
   1425 1430 1435 1440
   Thr Asp Ala Leu Met Thr Gly Tyr Thr Gly Asp Phe Asp Ser Val Ile
   1445 1450 1455
   Asp Cys Asn Val Ala Val Thr Gln Val Val Asp Phe Ser Leu Asp Pro
   1460 1465 1470
   Thr Phe Thr Ile Thr Thr Gln Ile Val Pro Gln Asp Ala Val Ser Arg
   1475 1480 1485
   Ser Gln Arg Arg Gly Arg Thr Gly Arg Gly Arg Leu Gly Ile Tyr Arg
   1490 1495 1500
   Tyr Val Ser Thr Gly Glu Arg Ala Ser Gly Met Phe Asp Ser Val Val
   1505 1510 1515 1520
   Leu Cys Glu Cys Tyr Asp Ala Gly Ala Ala Trp Tyr Glu Leu Thr Pro
   1525 1530 1535
   Ser Glu Thr Thr Val Arg Leu Arg Ala Tyr Phe Asn Thr Pro Gly Leu
   1540 1545 1550
   Pro Val Cys Gln Asp His Leu Glu Phe Trp Glu Ala Val Phe Thr Gly
   1555 1560 1565
   Leu Thr His Ile Asp Ala His Phe Leu Ser Gln Thr Lys Gln Ser Gly
   1570 1575 1580
   Glu Asn Phe Ala Tyr Leu Thr Ala Tyr Gln Ala Thr Val Cys Ala Arg
   1585 1590 1595 1600
   Ala Lys Ala Pro Pro Pro Ser Trp Asp Val Met Trp Lys Cys Leu Thr
   1605 1610 1615
   Arg Leu Lys Pro Thr Leu Val Gly Pro Thr Pro Leu Leu Tyr Arg Leu
   1620 1625 1630
   Gly Ser Val Thr Asn Glu Val Thr Leu Thr His Pro Val Thr Lys Tyr
   1635 1640 1645
   Ile Ala Thr Cys Met Gln Ala Asp Leu Glu Val Met Thr Ser Thr Trp
   1650 1655 1660
   Val Leu Ala Gly Gly Val Leu Ala Ala Val Ala Ala Tyr Cys Leu Ala
   1665 1670 1675 1680
   Thr Gly Cys Val Cys Ile Ile Gly Arg Leu His Ile Asn Gln Arg Ala
   1685 1690 1695
   Val Val Ala Pro Asp Lys Glu Val Leu Tyr Glu Ala Phe Asp Glu Met
   1700 1705 1710
   Glu Glu Cys Ala Ser Arg Ala Ala Leu Ile Glu Glu Gly Gln Arg Ile
   1715 1720 1725
   Ala Glu Met Leu Lys Ser Lys Ile Gln Gly Leu Leu Gln Gln Ala Ser
   1730 1735 1740
   Lys Gln Ala Gln Asp Ile Gln Pro Thr Val Gln Ala Ser Trp Pro Lys
   1745 1750 1755 1760
   Val Glu Gln Phe Trp Ala Lys His Met Trp Asn Phe Ile Ser Gly Ile
   1765 1770 1775
   Gln Tyr Leu Ala Gly Leu Ser Thr Leu Pro Gly Asn Pro Ala Val Ala
   1780 1785 1790
   Ser Met Met Ala Phe Ser Ala Ala Leu Thr Ser Pro Leu Ser Thr Ser
   1795 1800 1805
   Thr Thr Ile Leu Leu Asn Ile Leu Gly Gly Trp Leu Ala Ser Gln Ile
   1810 1815 1820
   Ala Pro Pro Ala Gly Ala Thr Gly Phe Val Val Ser Gly Leu Val Gly
   1825 1830 1835 1840
   Ala Ala Val Gly Ser Ile Gly Leu Gly Lys Val Leu Val Asp Ile Leu
   1845 1850 1855
   Ala Gly Tyr Gly Ala Gly Ile Ser Gly Ala Leu Val Ala Phe Lys Ile
   1860 1865 1870
   Met Ser Gly Glu Lys Pro Ser Met Glu Asp Val Val Asn Leu Leu Pro
   1875 1880 1885
   Gly Ile Leu Ser Pro Gly Ala Leu Val Val Gly Val Ile Cys Ala Ala
   1890 1895 1900
   Ile Leu Arg Arg His Val Gly Pro Gly Glu Gly Ala Val Gln Trp Met
   1905 1910 1915 1920
   Asn Arg Leu Ile Ala Phe Ala Ser Arg Gly Asn His Val Ala Pro Thr
   1925 1930 1935
   His Tyr Val Thr Glu Ser Asp Ala Ser Gln Arg Val Thr Gln Leu Leu
   1940 1945 1950
   Gly Ser Leu Thr Ile Thr Ser Leu Leu Arg Arg Leu His Asn Trp Ile
   1955 1960 1965
   Thr Glu Asp Cys Pro Ile Pro Cys Gly Gly Ser Trp Leu Arg Asp Val
   1970 1975 1980
   Trp Asp Trp Val Cys Thr Ile Leu Thr Asp Phe Lys Asn Trp Leu Thr
   1985 1990 1995 2000
   Ser Lys Leu Phe Pro Lys Met Pro Gly Leu Pro Phe Val Ser Cys Gln
   2005 2010 2015
   Lys Gly Tyr Lys Gly Val Trp Ala Gly Thr Gly Ile Met Thr Thr Arg
   2020 2025 2030
   Cys Pro Cys Gly Ala Asn Ile Ser Gly Asn Val Arg Leu Gly Ser Met
   2035 2040 2045
   Arg Ile Thr Gly Pro Lys Thr Cys Met Asn Ile Trp Gln Gly Thr Phe
   2050 2055 2060
   Pro Ile Asn Cys Tyr Thr Glu Gly Gln Cys Val Pro Lys Pro Ala Pro
   2065 2070 2075 2080
   Asn Phe Lys Val Ala Ile Trp Arg Val Ala Ala Ser Glu Tyr Ala Glu
   2085 2090 2095
   Val Thr Gln His Gly Ser Tyr His Tyr Ile Thr Gly Leu Thr Thr Asp
   2100 2105 2110
   Asn Leu Lys Val Pro Cys Gln Leu Pro Ser Pro Glu Phe Phe Ser Trp
   2115 2120 2125
   Val Asp Gly Val Gln Ile His Arg Phe Ala Pro Thr Pro Lys Pro Phe
   2130 2135 2140
   Phe Arg Asp Glu Val Ser Phe Cys Val Gly Leu Asn Ser Phe Val Val
   2145 2150 2155 2160
   Gly Ser Gln Leu Pro Cys Asp Pro Glu Pro Asp Thr Asp Val Leu Met
   2165 2170 2175
   Ser Met Leu Thr Asp Pro Ser His Ile Thr Ala Glu Thr Ala Ala Arg
   2180 2185 2190
   Arg Leu Ala Arg Gly Ser Pro Pro Ser Glu Ala Ser Ser Ser Ala Ser
   2195 2200 2205
   Gln Leu Ser Ala Pro Ser Leu Arg Ala Thr Cys Thr Thr His Gly Lys
   2210 2215 2220
   Ala Tyr Asp Val Asp Met Val Asp Ala Asn Leu Phe Met Gly Gly Asp
   2225 2230 2235 2240
   Val Thr Arg Ile Glu Ser Gly Ser Lys Val Val Val Leu Asp Ser Leu
   2245 2250 2255
   Asp Pro Met Val Glu Glu Arg Ser Asp Leu Glu Pro Ser Ile Pro Ser
   2260 2265 2270
   Glu Tyr Met Leu Pro Lys Lys Arg Phe Pro Pro Ala Leu Pro Ala Trp
   2275 2280 2285
   Ala Arg Pro Asp Tyr Asn Pro Pro Leu Val Glu Ser Trp Lys Arg Pro
   2290 2295 2300
   Asp Tyr Gln Pro Ala Thr Val Ala Gly Cys Ala Leu Pro Pro Pro Arg
   2305 2310 2315 2320
   Lys Thr Pro Thr Pro Pro Pro Arg Arg Arg Arg Thr Val Gly Leu Ser
   2325 2330 2335
   Glu Asp Ser Ile Gly Asp Ala Leu Gln Gln Leu Ala Ile Lys Ser Phe
   2340 2345 2350
   Gly Gln Pro Pro Pro Ser Gly Asp Ser Gly Leu Ser Thr Gly Ala Gly
   2355 2360 2365
   Ala Ala Asp Ser Gly Ser Gln Thr Pro Pro Asp Glu Leu Ala Leu Ser
   2370 2375 2380
   Glu Thr Gly Ser Ile Ser Ser Met Pro Pro Leu Glu Gly Glu Leu Gly
   2385 2390 2395 2400
   Asp Pro Asp Leu Glu Pro Glu Gln Val Glu Pro Gln Pro Pro Pro Gln
   2405 2410 2415
   Gly Gly Val Ala Ala Pro Gly Ser Asp Ser Gly Ser Trp Ser Thr Cys
   2420 2425 2430
   Ser Glu Glu Asp Asp Ser Val Val Cys Cys Ser Met Ser Tyr Ser Trp
   2435 2440 2445
   Thr Gly Ala Leu Ile Thr Pro Cys Ser Pro Glu Glu Glu Lys Leu Pro
   2450 2455 2460
   Ile Asn Pro Leu Ser Asn Ser Leu Leu Arg Tyr His Asn Lys Val Tyr
   2465 2470 2475 2480
   Cys Thr Thr Thr Lys Ser Ala Ser Leu Arg Ala Lys Lys Val Thr Phe
   2485 2490 2495
   Asp Arg Met Gln Val Leu Asp Ser Tyr Tyr Asp Ser Val Leu Lys Asp
   2500 2505 2510
   Ile Lys Leu Ala Ala Ser Lys Val Thr Ala Arg Leu Leu Thr Met Glu
   2515 2520 2525
   Glu Ala Cys Gln Leu Thr Pro Pro His Ser Ala Arg Ser Lys Tyr Gly
   2530 2535 2540
   Phe Gly Ala Lys Glu Val Arg Ser Leu Ser Gly Arg Ala Val Asn His
   2545 2550 2555 2560
   Ile Lys Ser Val Trp Lys Asp Leu Leu Glu Asp Ser Glu Thr Pro Ile
   2565 2570 2575
   Pro Thr Thr Ile Met Ala Lys Asn Glu Val Phe Cys Val Asp Pro Thr
   2580 2585 2590
   Lys Gly Gly Lys Lys Ala Ala Arg Leu Ile Val Tyr Pro Asp Leu Gly
   2595 2600 2605
   Val Arg Val Cys Glu Lys Met Ala Leu Tyr Asp Ile Thr Gln Lys Leu
   2610 2615 2620
   Pro Gln Ala Val Met Gly Ala Ser Tyr Gly Phe Gln Tyr Ser Pro Ala
   2625 2630 2635 2640
   Gln Arg Val Glu Phe Leu Leu Lys Ala Trp Ala Glu Lys Lys Asp Pro
   2645 2650 2655
   Met Gly Phe Ser Tyr Asp Thr Arg Cys Phe Asp Ser Thr Val Thr Glu
   2660 2665 2670
   Arg Asp Ile Arg Thr Glu Glu Ser Ile Tyr Arg Ala Cys Ser Leu Pro
   2675 2680 2685
   Glu Glu Ala His Thr Ala Ile His Ser Leu Thr Glu Arg Leu Tyr Val
   2690 2695 2700
   Gly Gly Pro Met Phe Asn Ser Lys Gly Gln Thr Cys Gly Tyr Arg Arg
   2705 2710 2715 2720
   Cys Arg Ala Ser Gly Val Leu Thr Thr Ser Met Gly Asn Thr Ile Thr
   2725 2730 2735
   Cys Tyr Val Lys Ala Leu Ala Ala Cys Lys Ala Ala Gly Ile Ile Ala
   2740 2745 2750
   Pro Thr Met Leu Val Cys Gly Asp Asp Leu Val Val Ile Ser Glu Ser
   2755 2760 2765
   Gln Gly Thr Glu Glu Asp Glu Arg Asn Leu Arg Ala Phe Thr Glu Ala
   2770 2775 2780
   Met Thr Arg Tyr Ser Ala Pro Pro Gly Asp Pro Pro Arg Pro Glu Tyr
   2785 2790 2795 2800
   Asp Leu Glu Leu Ile Thr Ser Cys Ser Ser Asn Val Ser Val Ala Leu
   2805 2810 2815
   Gly Pro Gln Gly Arg Arg Arg Tyr Tyr Leu Thr Arg Asp Pro Thr Thr
   2820 2825 2830
   Pro Ile Ala Arg Ala Ala Trp Glu Thr Val Arg His Ser Pro Val Asn
   2835 2840 2845
   Ser Trp Leu Gly Asn Ile Ile Gln Tyr Ala Pro Thr Ile Trp Ala Arg
   2850 2855 2860
   Met Val Leu Met Thr His Phe Phe Ser Ile Leu Met Ala Gln Asp Thr
   2865 2870 2875 2880
   Leu Asp Gln Asn Leu Asn Phe Glu Met Tyr Gly Ala Val Tyr Ser Val
   2885 2890 2895
   Ser Pro Leu Asp Leu Pro Ala Ile Ile Glu Arg Leu His Gly Leu Asp
   2900 2905 2910
   Ala Phe Ser Leu His Thr Tyr Thr Pro His Glu Leu Thr Arg Val Ala
   2915 2920 2925
   Ser Ala Leu Arg Lys Leu Gly Ala Pro Pro Leu Arg Ala Trp Lys Ser
   2930 2935 2940
   Arg Ala Arg Ala Val Arg Ala Ser Leu Ile Ser Arg Gly Gly Arg Ala
   2945 2950 2955 2960
   Ala Val Cys Gly Arg Tyr Leu Phe Asn Trp Ala Val Lys Thr Lys Leu
   2965 2970 2975
   Lys Leu Thr Pro Leu Pro Glu Ala Arg Leu Leu Asp Leu Ser Ser Trp
   2980 2985 2990
   Phe Thr Val Gly Ala Gly Gly Gly Asp Ile Tyr His Ser Val Ser Arg
   2995 3000 3005
   Ala Arg Pro Arg Leu Leu Leu Phe Gly Leu Leu Leu Leu Phe Val Gly
   3010 3015 3020
   Val Gly Leu Phe Leu Leu Pro Ala Arg
   3025 3030